(19) Europäisches Patentamt
European Patent Office
Office européen des brevets

(11) EP 0 890 564 B1

(12) **FASCICULE DE BREVET EUROPEEN**

(45) Date de publication et mention de la délivrance du brevet:
**24.11.2004 Bulletin 2004/48**

(51) Int Cl.[7]: **C07C 45/51**, C07C 49/683, C07C 49/747, C07C 49/753, C07C 37/60, C07C 39/08

(21) Numéro de dépôt: **98110051.4**

(22) Date de dépôt: **05.01.1994**

(54) **Procédé de préparation d'un composé aromatique p-dihydroxyle**

Verfahren zur Herstellung einer p-dihydroxyaromatischen Verbindung

Process for the preparation of a p-dihydroxyl aromatic compound

(84) Etats contractants désignés:
**DE FR GB IT NL SE**

(30) Priorité: **08.01.1993 FR 9300119**
**08.01.1993 FR 9300120**
**08.01.1993 FR 9300121**
**22.09.1993 FR 9311262**

(43) Date de publication de la demande:
**13.01.1999 Bulletin 1999/02**

(62) Numéro(s) de document de la (des) demande(s) initiale(s) en application de l'article 76 CBE:
**94400020.7 / 0 606 183**

(73) Titulaire: **RHODIA CHIMIE**
**92408 Courbevoie Cédex (FR)**

(72) Inventeurs:
- **Constantini, Michel M.**
  **69003 Lyon (FR)**
- **Manaut, Daniel M.**
  **69330 Meyzieu (FR)**
- **Michelet, Daniel M.**
  **78860 Saint-Nom-La-Breteche (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
**FR-A- 2 071 464** **FR-A- 2 336 364**
**US-A- 3 649 653** **US-A- 4 078 006**

- **M. PISOVA, ET AL.: "Quinone methides and fuchsones. XXVII. Oxidative rearrangement of o-fuchsone to 2,2-diphenyl-1,3-benzodioxole" COLLECTION OF CZECHOSLOVAK CHEMICAL COMMUNICATIONS., vol. 47, no. 12, décembre 1982 (1982-12), pages 3318-3327, XP002193487 Academic Press, London, GB ISSN: 0010-0765**
- **CHEMICAL ABSTRACTS, vol. 108, no. 17, 25 avril 1988 (1988-04-25) Columbus, Ohio, US; abstract no. 150241s, YA. B. KOZLIKOVSKII, ET AL.: "Reaction of phenol with benzophenone in the presence of aluminium phenolate." page 723; colonne 1; XP002193489 & IZV. VYSSH. UCHEBN. ZAVED., KHIM. KHIM. TEKHNOL., vol. 30, no. 7, 1987, pages 31-34,**
- **P. GRÜNANGER: "Chinonmethide" METHODEN DER ORGANISCHEN CHEMIE (HOUBEN - WEYL), vol. VII, no. 3B, 1979, pages 458-462, XP002193488 STUTTGART**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 890 564 B1

Printed by Jouve, 75001 PARIS (FR)

## Description

La présente invention concerne la préparation d'un composé aromatique para-dihydroxylé.

Un mode de réalisation de l'invention consiste à préparer le composé aromatique p-dihydroxylé, à partir d'une p-fuchsone.

Une autre variante de l'invention consiste à oxyder un composé phénolique en présence d'une p-fuchsone.

De nombreux procédés d'hydroxylation des phénols sont décrits dans l'état de la technique.

Citons, entre autres, le brevet FR-A 2 336 364 qui concerne un procédé d'hydroxylation de phénols et d'éthers de phénols qui consiste à réaliser l'hydroxylation par l'eau oxygénée, en présence d'un acide fort, d'un agent complexant et d'un aldéhyde, en particulier le benzaldéhyde.

La présente invention fournit un procédé totalement différent au départ d'une fuchsone.

Il existe dans la littérature, peu de références sur la synthèse des fuchsones.

Généralement, leur préparation est basée sur la déhydratation d'un carbinol.

Ainsi, I. S. IOFFE et al [J. Gen. Chem. USSR, 19, p.917-28 (1949)], ont décrit la préparation d'hydroxy-4 triphénylcarbinol, par réaction de $Ph_2CCl_2$ avec le phénol fondu.

H. BURTON et al [J. Chem. Soc., 3089-3090 (1955)] préconisent la synthèse de l'hydroxy-4 triphényl carbinol par addition de $Ph_2CCl_2$ dans une suspension de chlorure d'aluminium dans du sulfure de carbone et ensuite, introduction dans ce milieu réactionnel de phénol en solution dans le sulfure de carbone.

L'hydroxy-4 triphényl carbinol ainsi obtenu est ensuite déshydraté. Une technique de déshydratation décrite par I. S. IOFFE et al (loc. cit.) est de le soumettre, après addition d'acide acétique, à un traitement thermique, conduisant ainsi à la fuchsone suivante :

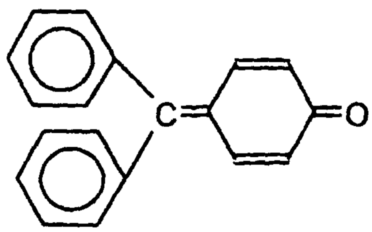

L'inconvénient dont souffrent les procédés décrits dans l'état de la technique est qu'il est quasiment impossible de mettre en oeuvre de tels procédés à l'échelle industrielle, en raison d'une matière première difficilement accessible ($Ph_2CCl_2$) et de la difficulté de manipulation de réactifs tels que le chlorure d'aluminium et le sulfure de carbone.

Par ailleurs, Kozlikovski et al [(Chemical Abstracts 108, 150241s (1988)] ont décrit la condensation du phénol, de la benzophénone et du phénolate d'aluminium à 190°C. De nombreux produits sont obtenus : p-fuchsone (21,3 %), 2,4'-(diphénylméthylène)diphénol (11,1 %) et 4,4'-(diphénylméthylène)diphénol (12,1 %).

Selon US-A-3 649 653, on a obtenu un triphénylcarbinol, par irradiation d'une solution de 2,6-dialkylphénol et d'une benzophénone : le carbinol étant transformé en fuchsone par acidification.

L'objectif de la présente invention est de fournir un procédé industriel de fabrication de fuchsones et ceci avec de bons rendements réactionnels.

Plus précisément, l'invention vise un procédé de préparation d'un composé aromatique p-dihydroxylé caractérisé par le fait qu'il consiste à faire réagir un agent oxydant avec une p-fuchsone.

Dans l'exposé qui suit de la présente invention, on entend par "p-fuchsone", tout composé chimique comportant le motif méthylène-1,4 quinone suivant :

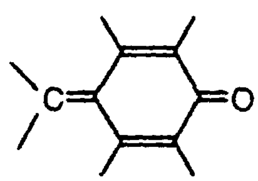

Une variante préférée du procédé de l'invention consiste à mettre en oeuvre la p-fuchsone obtenue selon un procédé qui consiste à faire réagir un composé phénolique présentant un atome d'hydrogène en position para du groupe hydroxyle et un composé cétonique non énolisable, en présence d'une quantité efficace d'un acide et éventuellement en présence d'une quantité efficace d'un composé soufré ionisable.

Une autre variante d'exécution du procédé de l'invention consiste à préparer le composé aromatique p-dihydroxylé, selon un procédé enchaînant la synthèse de la p-fuchsone par réaction d'un composé phénolique présentant un atome d'hydrogène en position para du groupe hydroxyle et un composé cétonique non énolisable, en présence d'une quantité efficace d'un acide et éventuellement en présence d'une quantité efficace d'un composé soufré ionisable, puis à effectuer l'oxydation de la p-fuchsone ainsi obtenue, sans séparation de cette dernière.

Enfin, un autre objet de la présente invention est un procédé de préparation d'un composé aromatique p-hydroxylé par oxydation du composé phénolique de départ, en présence d'une quantité efficace d'une p-fuchsone.

Conformément à une variante préférée du procédé de l'invention, on prépare une p-fuchsone en faisant réagir un composé phénolique présentant un atome d'hydrogène, en position para du groupe hydroxyle et un composé cétonique non énolisable, en présence d'un acide et éventuellement d'un composé soufré ionisable.

Le substrat de départ est un composé aromatique porteur d'au moins un groupe hydroxyle et présentant un atome d'hydrogène en position para du groupe hydroxyle. Par "composé aromatique", on entend la notion classique d'aromaticité telle que définie dans la littérature, notamment par Jerry MARCH, Advanced Organic Chemistry, 3ème édition, John Wiley and Sons, 1985, p.37 et suivantes.

Dans le présent texte, on désigne par "composé cétonique non énolisable", une cétone qui possède deux atomes de carbone tertiaire en position □ du groupe carbonyle,

On appelle "composé soufré ionisable", tout composé soufré qui s'ionise en présence d'eau pour donner un ion présentant une valence libre sur l'atome de soufre.

On fait appel tout particulièrement aux composés phénoliques de formule générale (I) :

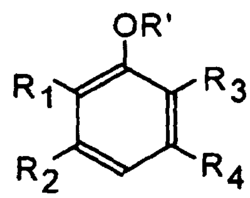

(I)

dans ladite formule (I) :

- la position en para est libre,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle,
- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

Par substituant cyclique, on entend un carbocycle saturé, insaturé ou aromatique ayant, généralement, de 4 à 7 atomes de carbone, et de préférence 6 atomes de carbone.

On choisit de préférence tout composé phénolique répondant à la formule générale (I) et, plus particulièrement, aux composés phénoliques de formule (I) dans laquelle R' représente :

. un atome d'hydrogène
. un radical alkyle linéaire ou ramifié ayant de 1 à 6 atomes de carbone, de préférence de 1 à 4 atomes de carbone, et plus particulièrement un radical méthyle ou éthyle,
. un radical cyclohexyle,
. un radical benzyle.

Le composé phénolique de formule (I) peut être porteur d'un ou plusieurs substituants $R_1$, $R_2$, $R_3$ et $R_4$. Des exemples de substituants sont donnés ci-après mais cette liste ne présente aucun caractère limitatif. N'importe quel substituant peut être présent sur le cycle dans la mesure où il n'interfère pas au niveau du produit désiré.

Encore plus préférentiellement, on fait appel aux composés phénoliques de formule (I) dans lesquels :

- $R_1$, $R_2$, $R_3$ et $R_4$, identiques ou différents représentent $R_0$, l'un des groupes suivants :

- un atome d'hydrogène,
- un radical alkyle, linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
- un radical alcényle linéaire ou ramifié ayant de 2 à 6 atomes de carbone, de préférence, de 2 à 4 atomes de carbone, tel que vinyle, allyle,
- un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
- un groupe acyle ayant de 2 à 6 atomes de carbone,
- un radical de formule :

$$-R_5-OH$$

$$-R_5-COOR_6$$

$$-R_5-X$$

$$-R_5-CF_3$$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

  - un radical carbocyclique saturé ou insaturé ayant de 4 à 7 atomes de carbone, de préférence un radical cyclohexyle,
  - un radical de formule

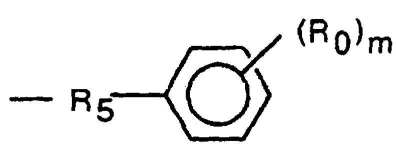

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

  - un radical - $R_5$ - A - $R_8$ dans lequel $R_5$ a la signification donnée précédemment, $R_8$ représente un radical alkyle linéaire ou ramifié ayant de 1 à 6 atomes de carbone ou un radical de formule

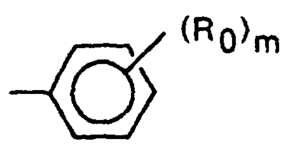

et A symbolise l'un des groupes suivants :
-O-, -COO-, -OCOO-, $-SO_2-$,

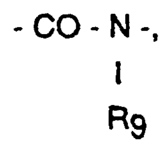

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone,

Parmi les composés de formule (I), on met en oeuvre plus particulièrement ceux répondant à la formule (I) dans laquelle :

- R' représente un atome d'hydrogène
- $R_1$, $R_2$, $R_3$ et $R_4$, identiques ou différents représentent l'un des groupes suivants :
  . un atome d'hydrogène,
  . un radical alkyle, linéaire ou ramifié ayant de 1 à 4 atomes de carbone,
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,
- deux groupes $R_1$ et $R_2$ et/ou $R_3$ et $R_4$ placés sur deux atomes de carbone vicinaux peuvent former ensemble et avec les atomes de carbone qui les portent un cycle benzénique.

Encore plus préférentiellement, on choisit les composés de formule (I) dans laquelle R' représente un atome d'hydrogène et l'un des radicaux $R_1$, $R_2$, $R_3$ et $R_4$ représente un groupe hydroxyle, un radical méthyle ou un radical méthoxy et les 3 autres représentent un atome d'hydrogène.

A titre illustratif de composés phénoliques de formule (I) susceptibles d'être mis en oeuvre dans la variante préféré du procédé de l'invention, on peut mentionner plus particulièrement :

- ceux répondant à la formule (I) dans laquelle $R_1$, $R_2$, $R_3$ et $R_4$ représentent un atome d'hydrogène, tels que le phénol ou l'anisole,
- ceux répondant à la formule (I) avec un substituant sur le cycle benzénique, tels que l'orthocrésol, le métacrésol, le 2-méthoxyphénol, le 2-éthylphénol, le 3-éthylphénol, 2-propylphénol, le 2-sec-butylphénol, le 2-tert-butylphénol, le 3-tert-butylphénol, le 2-méthoxyphénol, le 3-méthoxyphénol, le salicylate de méthyle, le 2-chlorophénol, le 3-chlorophénol,
- ceux répondant à la formule (I) avec deux substituants sur le cycle benzénique, tels que le 2,3-diméthylphénol, le 2,5-diméthylphénol, le 2,6-diméthylphénol, le 3,5-diméthyphénol, le 2,3-dichlorophénol, le 2,5-dichlorophénol, le 2,6-dichlorophénol, le 3,5-dichlorophénol, le 2,6-ditert-butylphénol, le 3,5-ditert-butylphénol,
- ceux répondant à la formule (I) avec trois substituants sur le cycle benzénique, tels que le 2,3,5-triméthylphénol, le 2,3,6-triméthylphénol, le 2,3,5-trichlorophénol, le 2,3,6-trichlorophénol,
- ceux répondant à la formule (I) dans laquelle $R_1$ et $R_2$ forment un cycle benzénique, tels que le 1-hydroxynaphtalène,
- ceux répondant à la formule (I) dans laquelle $R_1$ représente un radical de type $R_7$, tels que le 2-phénoxyphénol, le 3-phénoxyphénol.

Parmi les composés phénoliques de formule (I) précités, on choisit de préférence, le phénol, l'orthocrésol, le métacrésol.

Comme mentionné précédemment, la caractéristique du composé cétonique intervenant dans la variante du procédé de l'invention est qu'il s'agit d'une cétone non énolisable.

Plus spécifiquement, il répond à la formule (II) suivante :

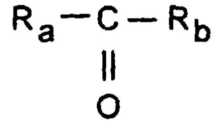

(II)

dans ladite formule (II) :

- $R_a$ et $R_b$, identiques ou différents sont des radicaux hydrocarbonés ayant chacun de 3 à 30 atomes de carbone; les atomes de carbone de chaque radical $R_a$ et $R_b$ en position $\alpha$ par rapport au groupe carbonyle étant des carbones tertiaires.

A titre d'exemples de radicaux $R_a$ et $R_b$, on peut citer entre autres, les radicaux alkyle ramifiés ayant au moins 3 atomes de carbone et les radicaux aryle ayant au moins 6 atomes de carbone et plus particulièrement les radicaux tert-butyle, tert-pentyle, tert-hexyle ou phényle éventuellement substitué.

Les composés cétoniques convenant à la variante préférée du procédé de l'invention peuvent être représenter plus précisément par la formule générale (IIa) suivante :

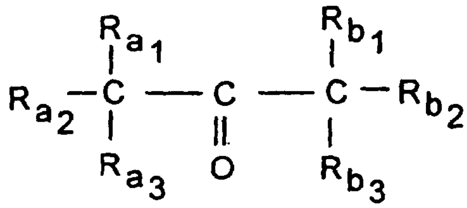

(IIa)

dans ladite formule (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ et/ou $R_{b1}$, $R_{b2}$, $R_{b3}$ peuvent former ensemble et avec l'atome de carbone qui les porte un cycle benzénique ou naphtalénique éventuellement substitué.

Parmi tous les composés cétoniques répondant à la formule (IIa), on fait appel tout particulièrement aux composés cétoniques répondant à la formule (IIb) suivante :

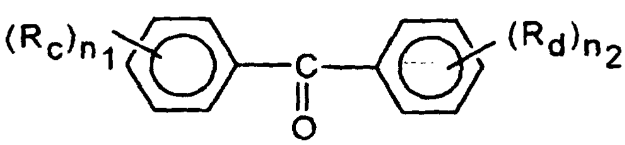

(IIb)

dans ladite formule (IIb) :

- $R_c$ et $R_d$ identiques ou différents représentent un atome d'hydrogène ou un substituant, de préférence un groupe électro-donneur,
- $n_1$, $n_2$ identiques ou différents est un nombre égal à 0, 1, 2 ou 3,

Le substituant est choisi de telle sorte qu'il ne réagisse pas dans les conditions d'acidité de l'invention. Il s'agit préférentiellement d'un groupe électro-donneur.

On entend par "groupe électro-donneur", un groupe tel que défini par H.C. BROWN dans l'ouvrage de Jerry MARCH - Advanced Organic Chemistry, chapitre 9, pages 243 et 244 (1985).

Des exemples de substituants sont les suivants :

- les radicaux alkyle linéaires ou ramifiés ayant de 1 à 4 atomes de carbone,

- le radical phényle,
- les radicaux alkoxy $R_{10}$ - O dans lesquels $R_{10}$ représente un radical alkyle linéaire ou ramifié ayant de 1 à 4 atomes de carbone ou le radical phényle,
- le groupe hydroxyle,
- l'atome de fluor.

Comme exemples de composés cétoniques choisis tout particulièrement, on peut citer les composés cétoniques répondant à la formule générale (IIb) dans laquelle $R_c$ et $R_d$, identiques ou différents représentent un atome d'hydrogène ou un substituant tel que précité, de préférence en position 4,4' et $n_1$, $n_2$ identiques ou différents sont égaux à 0 ou 1.

On fait appel préférentiellement aux composés cétoniques répondant à la formule (IIb) dans laquelle $R_c$ et $R_d$ identiques ou différents représentent un atome d'hydrogène ; un radical méthyle, éthyle, tert-butyle, phényle un radical méthoxy ou éthoxy; un groupe hydroxyle, de préférence en position 3,3' ou 4,4'.

Comme exemples spécifiques de cétones qui peuvent être utilisées préférentiellement dans la variante préférée du procédé de l'invention, on peut citer plus particulièrement :

- la benzophénone
- la méthyl-2 benzophénone
- la diméthyl-2,4 benzophénone
- la diméthyl-4,4' benzophénone
- la diméthyl-2,2' benzophénone
- la diméthoxy-4,4' benzophénone
- l'hydroxy-4 benzophénone
- la dihydroxy-4-4' benzophénone
- le benzoyl-4 biphényle

Selon la variante préférée du procédé de l'invention, on fait réagir, le composé phénolique de formule (I) avec le composé cétonique de formule générale (II), en présence d'un catalyseur acide.

Le catalyseur convenant tout à fait bien est choisi de telle sorte qu'il présente une fonction d'acidité - Ho comprise entre 5 et 20, de préférence entre 10 et 15.

Le catalyseur intervenant dans la variante préférée du procédé de l'invention est un catalyseur acide. Il peut s'agir d'une catalyse homogène ou hétérogène.

On peut faire appel à un acide protonique fort.

Comme exemples non limitatifs de tels acides forts, on peut citer les acides halogénés tels que l'acide chlorhydrique, l'acide bromhydrique, l'acide fluorhydrique, les oxyacides halogénés ou non tels que l'acide sulfurique, l'acide pyrosulfurique, l'acide perchlorique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide fluorhydrique, l'acide perchlorique, l'acide trifluorométhanesulfonique, l'acide paratoluènesulfonique l'acide chlorosulfonique, l'acide fluorosulfonique, l'acide méthanesulfonique, l'acide benzène sulfonique.

On choisit, tout particulièrement, l'acide fluorhydrique, l'acide perchlorique, l'acide trifluorométhanesulfonique ou l'acide méthanesulfonique.

Comme autres exemples de catalyseurs acides protoniques, on peut citer les résines sulfoniques.

Ainsi, on peut mettre en oeuvre dans la variante préférée du procédé de l'invention, les résines sulfoniques existant sur le marché, résines commercialisées sous différentes dénominations commerciales. On peut citer, entre autres, les résines suivantes : TEMEX 50, AMBERLYST 15, AMBERLYST 35, AMBERLYST 36, DOWEX 50W.

Les résines précitées qui conviennent tout à fait bien sont constituées d'un squelette polystyrénique qui porte des groupes fonctionnels qui sont des groupes sulfoniques.

Le squelette polystyrénique est obtenu par polymérisation du styrène et du divinylbenzène, sous l'influence d'un catalyseur d'activation, le plus souvent un peroxyde organique, ce qui conduit à un polystyrène réticulé. La polymérisation se fait le plus souvent en suspension et l'on obtient des billes ou des granules de polymère. Ceux-ci sont traités par de l'acide sulfurique ou sulfochlorique concentré. On obtient un copolymère styrène-divinylbenzène sulfoné.

Il est également possible de faire appel à des résines sulfoniques qui sont des copolymères phénol-formol et qui portent sur le noyau aromatique un groupe méthylène sulfonique. Comme exemples desdites résines, on peut mentionner entre autres, la résine commercialisée sous la dénomination DUOLITE ARC 9359.

D'autres résines sont également disponibles sur le marché et l'on peut citer les résines perfluorées porteuses

de groupes sulfoniques et, plus particulièrement le NAFION qui présente la structure générale donnée ci-après :

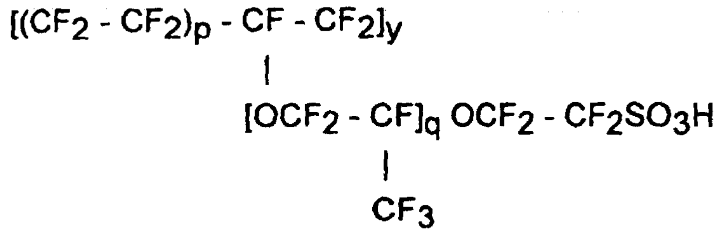

dans ladite formule, q est un nombre entier égal à 1,2,3,---, p est compris entre 5 et 13,5 et y est égal à environ 1000. Le NAFION est préparé à partir d'un copolymère de tétrafluoroéthylène et de perfluoro[2-(fluorosulfonyléthoxy)-propyl] vinyl éther.

Les résines en cause peuvent être du type gel ou du type macroréticulé. Elles sont mises en oeuvre sous la forme acide.

De nombreuses résines sont des produits disponibles sur le marché sous forme sèche ou humide. L'une ou l'autre des formes peuvent être utilisées dans la variante préférée du procédé de l'invention.

Elles se présentent habituellement sous forme de particules sensiblement sphériques ayant un diamètre variant de 0,3 à 1,5 mm, de préférence entre 0,5 et 1,2 mm.

Les résines précitées sont mises en oeuvre de préférence dans la variante préférée du procédé de l'invention et plus préférentiellement les résines ayant un squelette polystyrénique. Toutefois, l'invention n'exclut pas la mise en oeuvre de résines ayant un squelette d'une autre nature dans la mesure où elle porte les groupes sulfoniques adéquates.

La proportion de groupes sulfoniques par rapport à la masse polymérique peut être variable et l'on en tiendra compte lors de la détermination de la quantité de polymère à mettre en oeuvre.

La concentration en sites acides du polymère sulfonique varie avantageusement entre 1 et 10 milliéquivalents d'ions $H^+$ par gramme de polymère sec, et, de préférence, entre 2 et 7 milliéquivalents d'ions $H^+$ par gramme de polymère sec.

On assimilera également dans ce qui suit aux acides protoniques forts, les formes acides des oxydes mixtes, des argiles et des zéolithes.

Comme exemples de catalyseurs à propriétés acides, on peut citer, tout particulièrement les combinaisons d'oxydes métalliques et métalloïdiques telles que : silice-alumine, silice-$Ga_2O_3$,silice-$B_2O_3$.

Un autre type de catalyseurs minéraux acides convenant tout à fait bien à la mise en oeuvre de la variante préférée du procédé de l'invention sont les argiles acides.

Pour préparer les argiles acides, on part préférentiellement des argiles naturelles présentant une structure dite "TOT" ou tétraèdre-octaèdre-tétraèdre.

Les argiles TOT se présentent sous forme de feuillets élémentaires comprenant deux couches de tétraèdres d'atomes d'oxygène dans lesquels sont inclus les atomes de silicium séparées par une couche d'octaèdres d'atomes d'oxygène dans lesquels est inclus le métal M de type $MO_4(OH)_2$ ou M est un cation divalent ou trivalent.

Lorsque tous les tétraèdres sont occupés par des ions $Si^{4+}$, la neutralité électrique du feuillet peut être mesurée de deux façons, selon la charge du cation octaédrique :

- s'il est divalent ($Mg^{2+}$, $Fe^{2+}$,...), toutes les cavités octaédriques sont occupées; le feuillet est alors dit trioctaédrique,
- s'il est trivalent ($Al^{3+}$, $Fe^{3+}$,...), deux cavités octaédriques sur trois sont occupées et le feuillet est dioctaédrique.

Mais de nombreuses substitutions sont possibles, aussi bien dans la couche tétraédrique que dans la couche octaédrique. Celles-ci peuvent entraîner un déficit de charge du feuillet et la neutralité du cristal est alors réalisée par insertion de cations compensateurs entre les feuillets.

On trouve parmi les argiles TOT, les trois classes suivantes : les smectites, les vermiculites et les micas.

Parmi les argiles, la classe utilisée préférentiellement est celle des smectites.

Les smectites sont classifiées selon la nature du métal M (aluminium, magnésium, fer, lithium), la nature du cation compensateur (sodium, potassium, calcium) et la nature de la substitution tétraédrique ou octaédrique.

On peut ainsi citer parmi la classe des smectites :

- les montmorillonites de formule $Si_4(Al_{2-y}\,Mg_y)O_{10}(OH)_2,M^+{}_y$
- les beidellites de formule $(Si_{4-x}Al_x)Al_2O_{10}(OH)_2,M^+{}_x$
- les nontronites de formule $(Si_{4-x}Al_x)Fe_2O_{10}(OH)_2,M^+{}_x$

- les hectorites de formule $Si_4(Mg_{3-y} Li_y)O_{10}(OH)_2,M^+_y$
- les stévensites de formule $Si_4(Mg_{3-y})O_{10}(OH_2),M^+_{2y}$
- les saponites de formule $(Si_{4-x}Al_x)Mg_3O_{10}(OH_2),M^+_x$

On préfère encore tout particulièrement les montmorillonites.

On peut utiliser les argiles commerciales déjà acides telles que notamment : les argiles suivantes : KSF, TONSIL OPTIMUM FF et K 10 vendues par Süd Chemie.

On peut également traiter les argiles commerciales, déjà acides ou non par une solution aqueuse d'un acide. La concentration de la solution aqueuse en acide est variable, cependant elle devra avoir un pH supérieur ou égal à 2 de façon à ne pas détruire l'argile et elle devra contenir une quantité d'ions $H^+$ exprimé en milliéquivalents correspondant à au moins la capacité d'échange de l'argile. On définit la capacité d'échange d'une argile comme le nombre de cations monovalents, exprimé en milliéquivalents que peuvent échanger 1 g d'échantillon.

Cette capacité d'échange (ou charge par demi-maille) varie pour les smectites entre 0,2 et 0,6 et pour les vermiculites entre 0,6 et 0,9. Il est préférable donc dans le cadre de la présente invention d'utiliser une solution acide contenant autant d'équivalents $H^+$ qu'il y aura de cations à échanger dans l'argile, donc contenant au moins 0,6 et de préférence au moins 1 milliéquivalent acide pour 1 g d'argile.

On peut éventuellement, pour améliorer la surface d'échange de l'argile, la traiter ensuite avec un alcool tel que le méthanol, puis la sécher.

Conviennent également à la mise en oeuvre de la variante préférée du procédé de l'invention, les argiles de type pontée.

On entend par argile pontée, des argiles entre les feuillets desquelles ont été introduits des ponts ou piliers qui maintiennent un espacement basal. L'espacement basal (ou distance interfoliaire) est la somme de l'épaisseur d'un feuillet de l'argile et de l'espacement interfoliaire.

Comme exemples d'espèces minérales créant les piliers ou ponts, on peut citer celles dérivées des éléments suivants : aluminium, nickel, cobalt, vanadium, molybdène, rhénium, fer, cuivre, ruthénium, chrome, lanthane, cérium, titane, bore, gallium, zirconium, niobium, tantale, silicium.

Les argiles pontées mises en oeuvre préférentiellement dans la variante préférée du procédé de l'invention sont les argiles pontées par des piliers d'oxyde d'aluminium, de zirconium, de chrome, de silicium et de cérium.

Les dites argiles sont préparées selon les procédés décrits dans la littérature.

On peut se référer, entre autres, pour la préparation d'argiles pontées à l'aluminium à FR-A 2 563 446 et à FR-A 2 656 298 ; d'argiles pontées au titane à FR-A 2 669 016 et d'argiles pontées au cérium à la demande de brevet française n°91/04409.

Parmi les argiles pontées, on fait appel tout particulièrement aux argiles de type smectite. Parmi ces smectites, on peut citer à titre d'exemples non limitatifs, les beidellites, en particulier les beidellites synthétiques et les montmorillonites.

Les argiles pontées peuvent être obtenues par un procédé comportant les étapes suivantes :

a) traitement d'une argile naturelle ou synthétique, sous forme d'une suspension aqueuse, par une solution aqueuse contenant au moins un hydroxyde d'au moins un métal,
b) l'élimination de l'excès d'hydroxyde de métal n'ayant pas réagi sur l'argile ; ladite élimination et séchage de l'argile traitée,
c) le traitement thermique de l'argile traitée ; ledit traitement conduisant à l'obtention de l'argile pontée.

Pour la préparation des argiles pontées, on peut également mettre en oeuvre que les étapes a) et b).

Le pontage des argiles peut être effectué à l'aide des hydroxydes des métaux précités.

De préférence, on utilise comme catalyseur dans la variante préférée du procédé de l'invention, une beidellite ou une montmorillonite pontée à l'aide d'hydroxyde d'aluminium, par exemple selon le procédé décrit dans FR-A 2 563 446.

Comme exemples de catalyseurs convenant également à la variante préférée de l'invention, on peut citer les zéolithes naturelles telles que par exemple : la chabazite, la clinoptilolite, l'érionite, la mordénite, la phillipsite, l'offrétite.

Conviennent tout à fait bien à la mise en oeuvre de ladite variante, les zéolithes synthétiques telles que la zéolithe ZSM-5, la zéolithe Y, la ferrierite; la zéolithe X de type faujasite, la zéolithe de type L, la mordénite, la zéolithe ZSM-11 ; la mazzite, l'offrétite. Quelle que soit la zéolithe, on fait un traitement qui la rend acide.

On fait appel préférentiellement aux zéolithes synthétiques et plus particulièrement aux zéolithes commerciales qui sont sous les formes suivantes :

- des zéolithes sous forme acide telles que les zéolithes ZSM-5 ou silicalite d'aluminium de rapport molaire Si/Al de 10 à 500 ; les zéolithes Y en particulier les zéolithes obtenues après traitement de désalumination (par exemple

hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60; les zéolithes telles que la ferrierite de rapport molaire Si/Al de 5 à 10,

- des zéolithes sous forme sodique ou potassique échangeable telles que la zéolithe X de type faujasite de rapport molaire Si/Al de 1 à 1,5, la zéolithe de type L de rapport molaire Si/Al de 3 à 3,5, la mordénite de rapport molaire Si/Al de 5 à 6, la zéolithe ZSM-11 de rapport molaire Si/Al de 5 à 30,
- des zéolithes à la fois sous forme acide ou sous forme sodique ou potassique échangeable telles que la mazzite de rapport molaire Si/Al de 3,4, l'offrétite de rapport molaire Si/Al de 4.

la zéolithe mise en oeuvre dans la variante préférée du procédé de l'invention est une zéolithe acidifiée.

Pour acidifier la zéolithe si nécessaire, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $5.10^{-3}$ à $10^{-5}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

Les zéolithes peuvent être également acidifiées en soumettant celles-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100ml/g. Ce passage peut être réalisé en une seule étape ou de préférence en plusieurs étapes successives.

On peut également faire appel à une zéolithe acide modifiée par exemple en faisant appel à des zéolithes de type ZSM-5 et ZSM-11 dans lesquelles une partie de l'aluminium est remplacé par un élément tel que fer, gallium, bore, indium, chrome, scandium, cobalt, nickel, beryllium, zinc, cuivre, antimoine, arsenic, vanadium ou leur mélange.

Comme exemples d'autres catalyseurs solides à propriétés acides convenant tout à fait bien, on peut citer les phosphates tels que notamment les phosphates de bore, d'aluminium ou de gallium.

On peut en particulier faire appel aux phosphates lamellaires de métaux tétravalents répondant à la formule $\alpha$- $M(HPO_4)_2$, $pH_2O$ dans laquelle M représente un métal tétravalent choisi parmi le titane, le germanium, le zirconium ou l'étain et p est un nombre inférieur à 2. Lesdits composés sont décrits dans la littérature et l'on peut se référer entre autres aux travaux de ALBERTI et al - J. of Inorg. Nucl. Chem. 40, p. 1113 (1978).

On peut également mettre en oeuvre. des phosphonates lamellaires de métaux tétravalents et tout particulièrement les phosphonates lamellaires de zirconium (US-A 4 436 899).

Conviennent également bien, les oxydes rendus acides par un traitement. On peut citer les oxydes sulfatés, chlorés ou fluorés. Ceux qui sont mis en oeuvre préférentiellement sont les oxydes sulfatés de titane, de zirconium ou de fer. Ces oxydes sont décrits dans la littérature et l'on peut se référer entre autres à EP-A 0 397 553.

Les modes préférés de réalisation de l'invention consistent à faire appel aux catalyseurs acides suivants : l'acide fluorhydrique, l'acide perchlorique, l'acide trifluorométhanesulfonique, l'acide méthanesulfonique et les résines sulfoniques de type NAFION.

Une autre variante de l'invention, consiste à préparer la p-fuchsone, en présence d'un additif, à savoir un composé soufré ionisable. En effet, il a été trouvé que la mise en oeuvre d'un tel composé permettait d'accroître la cinétique réactionnelle. Ainsi, on a constaté que la vitesse de formation de la fuchsone était considérablement augmentée et qu'elle pouvait être multipliée par dix ou voire même plus.

Pour ce qui est du composé soufré intervenant éventuellement comme additif, on peut faire appel plus particulièrement aux composés suivants :

. composés minéraux soufrés :

  - les halogénures de soufre, de préférence le monochlorure de soufre ou le bichlorure de soufre,
  - les thiosulfates d'ammonium, de métal alcalin ou alcalino-terreux, de préférence le thiosulfate d'ammonium, de sodium ou de potassium,
  - l'hydrogène sulfuré ou ses précurseurs tels que, par exemple, le sulfure de calcium ou de potassium qui, ajoutés au milieu réactionnel acide, conduisent à la formation d'hydrogène sulfuré,

. composés organiques soufrés :

- les mercaptans notamment les alkylmercarptans, phénylmercaptans et phénylalkylmercaptans tels que par exemple, l'éthylmercaptan, le butylmercaptan, le 1-nonylmercaptan, le benzylmercaptan,
- les thioalcools comme par exemple, le 2-mercaptoéthanol, le 3-mercapto-2-butanol,
- les thiophénols tels que notamment, le thiophénol, l'o-thiocrésol, le m-thiocrésol, le p-thiocrésol, le thioxylénol, le p-méthylthiophénol, l'o-éthylthiophénol, le thiohydroquinone, le thionaphtol,
- les acides thioorganiques, leurs sels ou leurs esters tels que l'acide thioacétique, l'acide thiopropionique, l'acide thiolactique, l'acide thiosalicylique, l'acide 2-mercaptoéthanesulfonique, l'acide 3-mercaptopropanesulfonique, l'acide mercaptosuccinique, l'acide thioglycolique, le thioglycolate de méthyle, d'éthyle,
- les résines échangeuses de cations modifiées par réaction avec une alkyl mercaptoamine, de préférence les résines à squelette polystyrénique porteuses de groupes sulfoniques telles que précédemment définies, ayant réagi avec une alkyl mercaptoamine, de préférence celle avec un groupe amine primaire et encore plus préférentiellement celle ayant de 1 à 4 atomes de carbone, et notamment, la 2-mercaptoéthylamine, la 2-mercaptoisopropylamine, la 3-mercaptobutylamine.

Les composé soufrés choisis préférentiellement sont les alkylmercaptans et le benzylmercaptan.

Conformément à une variante du procédé de l'invention, on effectue la préparation de la p-fuchsone par réaction du composé phénolique de formule (I), et du composé cétonique de formule (II), en présence du catalyseur acide et éventuellement d'un composé soufré ionisable.

La quantité de composé phénolique de formule (I) mis en oeuvre et exprimée par rapport au composé cétonique de formule (II) est généralement au moins égale à la quantité stoechiométrique. On choisit avantageusement un rapport molaire entre le composé phénolique de formule (I) et le composé cétonique de formule (II) compris entre 1 et 20, de préférence entre 1 et 10.

Pour ce qui est du catalyseur acide, la quantité d'acide exprimée par le rapport d'équivalents de protons au nombre de moles de composé cétonique de formule (II) peut varier entre $1.10^{-3}$ et 1,0, de préférence entre $5.10^{-3}$ et 0,5.

Il est également possible d'utiliser l'acide en tant que milieu réactionnel. On peut citer notamment le cas de l'acide fluorhydrique et de l'acide trifluorométhanesulfonique. Ainsi, le rapport précité peut être supérieur à 1,0 et être très élevé et dépassé 20. D'une manière préférée, il est compris entre 5,0 et 10.

S'agissant d'un catalyseur hétérogène solide, la quantité à mettre en oeuvre est déterminée de telle sorte que le catalyseur représente en poids par rapport au composé phénolique de formule (I) engagé, de 0,1 à 20 %, de préférence de 0,5 à 10 %. Cependant si l'on réalise le procédé en continu, par exemple en faisant réagir un mélange de composé phénolique de formule (I) et de composé cétonique de formule (II), sur un lit fixe de catalyseur, ces rapports catalyseur /composé cétonique de formule (II) n'ont pas de sens et à un instant donné, on pourra avoir un excès pondéral de catalyseur par rapport au composé cétonique de formule (II).

La quantité de composé soufré mis en oeuvre exprimée par rapport au composé cétonique de formule (II) est choisie avantageusement de telle sorte que le rapport molaire entre le composé soufré et le composé cétonique de formule (II) soit compris entre 0,001 et 1,0, de préférence entre 0,005 et 0,20.

La préparation de la fuchsone préparée selon la variante préférée du procédé de l'invention, est réalisée à une température qui peut être comprise entre 45°C et 200°C.

On choisit de préférence la température entre 60°C et 150°C.

La réaction est conduite avantageusement sous pression atmosphérique.

La préparation de la fuchsone conformément à la variante préférée du procédé de l'invention peut être également conduite, en présence d'un solvant organique.

L'un des intérêts de la mise en oeuvre d'un solvant organique est la possibilité d'éliminer l'eau du milieu réactionnel, par distillation azéotropique.

On peut faire appel à tout solvant organique inerte dans les conditions réactionnelles. Comme exemples de solvants organiques convenant tout à fait bien, on peut mentionner entre autres :

- les hydrocarbures aliphatiques et/ou aromatiques et plus particulièrement les paraffines tels que notamment, l'hexane, l'heptane, l'octane, le nonane, le décane, le undécane, le dodécane ou le tétradécane, le cyclohexane, le méthylcyclohexane ; les hydrocarbures aromatiques comme notamment les xylènes, le cumène, les coupes pétrolières constituées de mélange d'alkylbenzènes notamment les coupes de type Solvesso ®.
- les hydrocarbures halogénés aliphatiques ou aromatiques, et l'on peut mentionner : les hydrocarbures perchlorés tels que notamment le tétrachlorure de carbone, le tétrachloroéthylène, l'hexachloroéthane, l'hexachloropropène, les hydrocarbures partiellement chlorés tels que le chlorure de méthylène, le dichloroéthane, le tétrachloroéthane, le trichloroéthylène, le 1-chlorobutane, le 1,2-dichlorobutane ; le mono-chlorobenzène, le 1,2-dichlorobenzène, le

1,3-dichlorobenzène, le 1,4-dichlorobenzène ou des mélanges de différents chlorobenzènes ; le monobromobenzène ou des mélanges de monobromobenzène avec un ou plusieurs dibromobenzènes.

On peut également mettre en oeuvre un mélange de solvants organiques.

La concentration des réactifs dans le solvant organique peut varier largement. La concentration de la cétone non énolisable dans le solvant organique peut varier, par exemple, entre 0,1 et 2 mol/litre, de préférence, entre 0,2 et 1,0 mol/litre.

D'un point de vue pratique, le procédé de préparation de la p-fuchsone effectuée selon le mode préféré est simple à mettre en oeuvre de façon continue ou discontinue.

Les différents réactifs peuvent être introduits dans n'importe quel ordre. D'une manière préférée, on choisit l'ordre des réactifs suivants : on introduit le composé phénolique de formule (I), le composé cétonique de formule (II) et le catalyseur acide et éventuellement un composé soufré ionisable. On peut également additionner un solvant organique.

On porte le milieu réactionnel à la température désirée, tout en maintenant le milieu réactionnel sous agitation.

Au cours de la réaction, il y a formation d'eau dans le milieu réactionnel. Une variante préférée de l'invention consiste à limiter la concentration de l'eau dans le milieu réactionnel, en éliminant celle-ci au fur et à mesure de sa formation, par tout moyen connu, notamment par distillation azéotropique.

En fin de réaction, on récupère la fuchsone formée à partir du milieu réactionnel.

On commence par séparer le catalyseur. S'il s'agit d'une catalyse homogène, on lave la masse réactionnelle à l'eau et l'on additionne un solvant organique convenant pour extraire les réactifs n'ayant pas réagi et la fuchsone formée. Comme exemples de solvants organiques, on peut citer notamment l'acétate d'éthyle, le dichlorométhane. Le catalyseur acide passe en phase aqueuse et l'on récupère en phase organique, la fuchsone formée et éventuellement les réactifs qui n'ont pas été transformés.

Dans le cas d'un catalyseur acide hétérogène, on lave le catalyseur acide solide à l'eau en présence d'un solvant organique qui extrait les réactifs n'ayant pas réagi et la fuchsone formée, on sépare le catalyseur selon les techniques classiques de séparation solide-liquide, de préférence par filtration puis on sépare la phase organique.

On peut récupérer la fuchsone à partir de la phase organique, par chromatographie liquide préparative sur colonne de silice.

Il est également possible de séparer la fuchsone, du composé phénolique non transformé et du composé cétonique de formule (II), par les moyens usuels, notamment, par cristallisation.

En ce qui concerne le composé soufré, celui-ci peut selon sa nature, se retrouver, soit dans la phase aqueuse, soit dans la phase organique. Il peut être récupéré selon les procédés habituels de séparation, notamment par distillation.

La préparation de la p-fuchsone selon ce mode préféré, présente de nombreux avantages. Il fait appel à des matières premières aisément disponibles et permet d'obtenir la fuchsone avec une très bonne sélectivité.

Le perfectionnement qui consiste à ajouter un composé soufré ionisable permet d'augmenter la vitesse réactionnelle de formation de la fuchsone, d'une manière appréciable.

La fuchsone obtenue selon le mode préféré, peut être symbolisée par la formule générale suivante (III) :

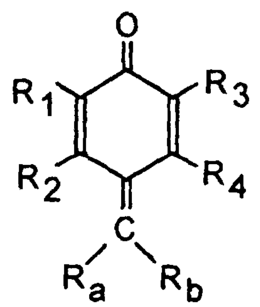

(III)

- dans ladite formule (III) les différents symboles $R_1$, $R_2$, $R_3$, $R_4$, $R_a$ et $R_b$ ont la ont la signification donnée précédemment, à savoir :

  - $R_a$ et $R_b$, identiques ou différents sont des radicaux hydrocarbonés ayant chacun de 3 à 30 atomes de carbone ; les atomes de carbone de chaque radical $R_a$ et $R_b$ en position □ par rapport à l'atome de carbone qui les porte, étant des carbones tertiaires,
  - $R_1$, $R_2$, $R_3$ et $R_4$, identiques ou différents représentent un atome d'hydrogène ou un substituant quelconque,

- deux groupes $R_1$ et $R_2$ et/ou $R_3$ et $R_4$ placés sur deux atomes de carbone vicinaux peuvent former ensemble et avec les atomes de carbone qui les portent un cycle, de préférence un carbocycle insaturé ou aromatique ayant de 4 à 7 atomes de carbone.

Il convient tout particulièrement bien à la préparation de diphénylfuchsone à partir de phénol et de benzophénone.

Ledit procédé permet d'accéder à une fuchsone de formule (III) mais il est à noter qu'une partie de la fuchsone formée peut se trouver sous une forme hydratée que l'on nomme carbinol et qui peut être représentée par la formule suivante (IV) :

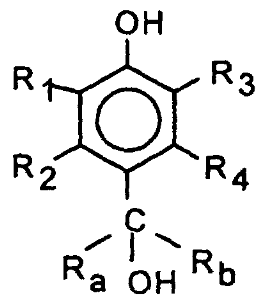

(IV)

dans ladite formule (IV), les différents symboles $R_1$, $R_2$, $R_3$, $R_4$, $R_a$ et $R_b$ ont les significations données précédemment.

Le rapport molaire fuchsone/carbinol varie avec la quantité d'eau mise en oeuvre lors du traitement de la masse réactionnelle et avec le pH du milieu. A titre d'exemples, on précise que ce rapport peut aller de 1 à 2.

L'objet de la présente invention est un procédé de préparation d'un composé aromatique para-dihydroxylé caractérisé par le fait qu'il consiste à faire réagir un agent oxydant avec une p-fuchsone.

On part d'une p-fuchsone sous forme anhydre ou hydratée ou d'un mélange des deux formes. On met en oeuvre préférentiellement une p-fuchsone répondant à la formule générale (III) et/ou (IV).

Parmi les composés de formule (III) et/ou (IV), on met en oeuvre plus particulièrement ceux répondant à la formule (III) et/ou (IV) dans laquelle :

- $R_1$, $R_2$, $R_3$ et $R_4$, identiques ou différents représentent $R_0$, l'un des groupes définis pour la formule (I),
- $R_1$, $R_2$, $R_3$ et $R_4$, identiques ou différents représentent $R_7$, l'un des radicaux plus complexes suivants définis pour la formule (I),

A titre d'exemples de radicaux $R_a$ et $R_b$ convenant bien à la présente invention, on peut citer entre autres, les radicaux alkyle ramifiés ayant au moins 3 atomes de carbone et les radicaux aryle ayant au moins 6 atomes de carbone et plus particulièrement les radicaux tert-butyle, tert-pentyle, tert-hexyle ou phényle éventuellement substitué.

Les fuchsones de formule (III) et (IV) peuvent être porteuses d'un ou plusieurs substituants $R_1$, $R_2$, $R_3$ ou $R_4$. Des exemples de substituants sont donnés ci-dessus mais cette liste ne présente aucun caractère limitatif. N'importe quel substituant peut être présent sur le cycle dans la mesure où il n'interfère pas au niveau du produit désiré.

Les fuchsones convenant à la présente invention peuvent être représentées plus précisément par les formules générales (IIIa) et/ou (IVa) suivantes :

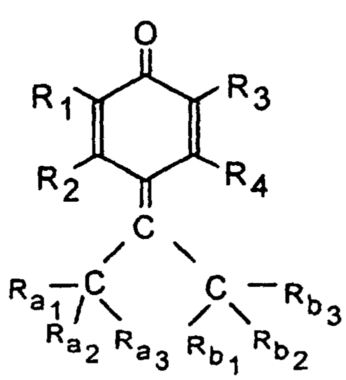

(IIIa)

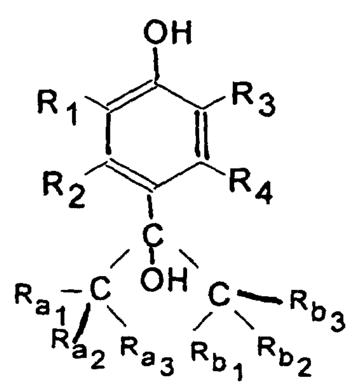

(IVa)

dans lesdites formules (IIIa) et (IVa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ d'une part et $R_{b1}$, $R_{b2}$, $R_{b3}$, d'autre part peuvent former ensemble et avec l'atome de carbone qui les porte, un cycle benzénique ou naphtalénique éventuellement substitué,
- $R_1$, $R_2$, $R_3$ et $R_4$, identiques ou différents représentent l'un des groupes suivants :

  . un atome d'hydrogène,
  . un radical alkyle linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- les deux groupes $R_1$ et $R_2$ et/ou $R_3$ et $R_4$ placés sur deux atomes de carbone vicinaux peuvent former ensemble et avec les atomes de carbone qui les portent un cycle benzénique.

Par "éventuellement substitué", on entend qu'il y a présence de un ou plusieurs substituants dans les radicaux cycliques. Lesdits substituants symbolisés par Y, sont précisés ultérieurement.
Encore plus préférentiellement, on choisit les composés de formule (IIIa) et/ou (IVa) dans laquelle l'un des radicaux $R_1$ $R_2$, $R_3$ et $R_4$ représente un groupe hydroxyle, un radical méthyle ou un radical méthoxy et les 3 autres représentent un atome d'hydrogène.
Parmi toutes les fuchsones répondant aux formules (IIIa) et/ou (IVa), on fait appel tout particulièrement aux fuchsones répondant aux formules (IIIb) et/ou (IVb) suivantes :

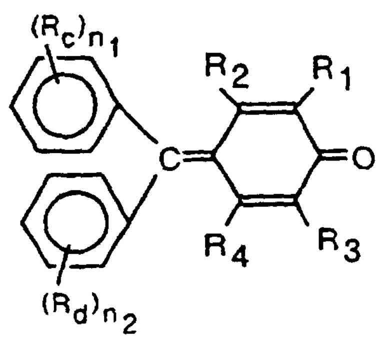

(IIIb)

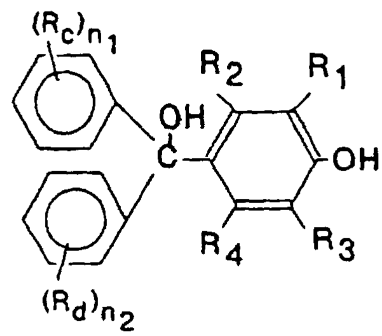

(IVb)

dans lesdites formules (IIIb) et (IVb) :

- $R_1$, $R_2$, $R_3$ et $R_4$, identiques ou différents représentent l'un des groupes suivants :

  . un atome d'hydrogène,
  . un radical alkyle linéaire ou ramifié, ayant de 1 à 4 atomes de carbone,
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- deux groupes $R_1$ et $R_2$ et/ou $R_3$ et $R_4$ placés sur deux atomes de carbone vicinaux peuvent former ensemble et avec les atomes de carbone qui les portent un cycle benzénique.
- $R_c$ et $R_d$ identiques ou différents représentent un atome d'hydrogène ou un substituant,
- $n_1$, $n_2$ identiques ou différents est un nombre égal à 0, 1, 2 ou 3.

Le substituant désigné Y est choisi de telle sorte qu'il ne réagisse pas dans les conditions d'acidité de l'invention.

Des exemples de substituants convenant bien à l'invention sont les suivants :

- les radicaux alkyle linéaires ou ramifiés ayant de 1 à 4 atomes de carbone,
- le radical phényle,
- les radicaux alkoxy $R_{10}$ - O dans lesquels $R_{10}$ représente un radical alkyle linéaire ou ramifié ayant de 1 à 4 atomes de carbone ou le radical phényle,
- le groupe hydroxyle,
- les atomes d'halogène, de préférence de chlore, de brome ou de fluor.

Comme exemples de fuchsones particulièrement adaptées à l'invention, on peut citer tout particulièrement les fuchsones répondant aux formules générales (IIIb) et/ou (IVb) dans laquelle $R_c$ et $R_d$, identiques ou différents représentent un atome d'hydrogène ou un substituant tel que précité ; $R_c$ et $R_d$ étant de préférence en position 4,4' et $n_1$, $n_2$ identiques ou différents sont égaux à 0 ou 1.

On fait appel préférentiellement aux fuchsones répondant aux formules (IIIb) et/ou (IVb) dans laquelle $R_c$ et $R_d$ identiques ou différents représentent un atome d'hydrogène ; un radical méthyle, éthyle, tert-butyle, phényle ; un radical méthoxy ou éthoxy ; un groupe hydroxyle : $R_c$ et $R_d$ étant de préférence en position 3,3' ou 4,4'.

Comme exemples spécifiques de fuchsones qui peuvent être utilisées dans le procédé de l'invention, on peut citer plus particulièrement les fuchsones suivantes et/ou leurs carbinols :

- la diphénylfuchsone et/ou son carbinol :

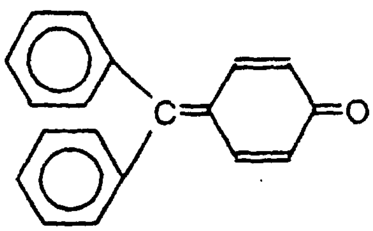

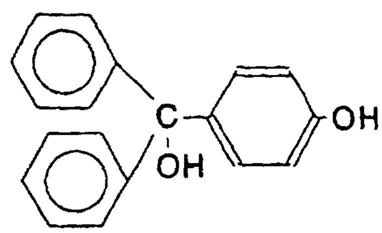

- l'acide rosolique et/ou son carbinol :

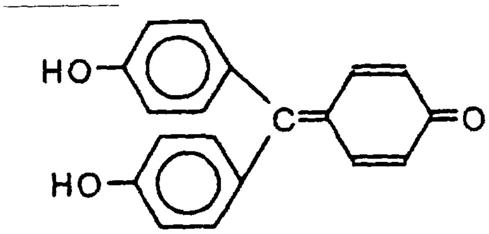

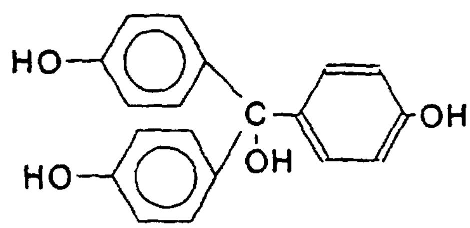

- la diphénylméthylfuchsone et/ou son carbinol :

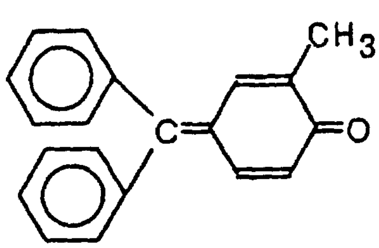

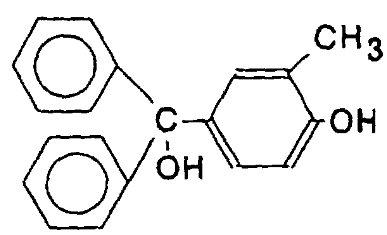

On peut mettre en oeuvre dans le procédé de l'invention, toute fuchsone, quelque soit son mode d'obtention. Ainsi, on peut partir de fuchsones obtenues selon les procédés décrits dans la littérature notamment par I. S. IOFFE et al [J. Gen. Chem. USSR, 19, p.917-28 (1949)] et H. BURTON et al [J. Chem. Soc., 3089-3090 (1955)].

Comme agents oxydants susceptibles d'être mis en oeuvre dans le procédé de l'invention, on peut citer notamment, le peroxyde d'hydrogène, les peracides tels que l'acide peracétique, les hydroperoxydes tels que l'hydroperoxyde de tertiobutyle, l'hydroperoxyde de cyclohexyle, l'hydroperoxyde de cumyle.

Parmi les agents oxydants précités, on fait appel préférentiellement au peroxyde d'hydrogène.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de $H_2O_2$ et, de préférence, aux environs de 70 %.

La quantité de peroxyde d'hydrogène est de préférence égale à la quantité stoechiométrique soit 1 mole de $H_2O_2$ pour 1 mole de fuchsone de formule (III) et/ou (IV). On peut envisager de mettre en oeuvre un léger excès pouvant atteindre 20 % de la quantité stoechiométrique.

Une variante préférée du procédé de l'invention consiste à faire réagir la fuchsone et l'agent oxydant, en présence d'un catalyseur acide.

Intervient éventuellement dans le procédé de l'invention, un catalyseur acide qui est un acide fort. Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation par le peroxyde d'hydrogène.

On peut citer plus particulièrement les oxyacides halogénés ou non tels que l'acide sulfurique, l'acide pyrosulfurique, l'acide perchlorique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesuifoniques et les acides naphtalènedisulfoniques.

Parmi ces acides, on utilisera de préférence l'acide perchlorique, l'acide trifluorométhanesulfonique, l'acide paratoluènesulfonique, l'acide chlorosulfonique, l'acide fluorosulfonique, l'acide méthanesulfonique.

On choisit, tout particulièrement, l'acide perchlorique ou l'acide trifluorométhanesulfonique.

La quantité d'acide exprimée par le rapport du nombre d'équivalents de protons au nombre de moles de peroxyde d'hydrogène peut varier entre environ $1.10^{-4}$ et environ 1,0.

Une variante préférée du procédé de l'invention consiste à choisir un rapport $H^+/H_2O_2$ compris entre $1.10^{-3}$ et 0,1.

La réaction est avantageusement conduite en phase liquide. Ainsi, on commence par solubiliser la fuchsone dans un solvant organique qui peut être le composé phénolique de formule (I) tel que défini ci-après ou tout solvant organique protique ou aprotique, polaire ou apolaire dans la mesure où il convient à la présente invention.

Plusieurs impératifs président au choix du solvant.

Le solvant organique doit être stable dans les conditions de l'invention. Sont exclus de la présente invention, les solvants qui ne sont pas stables dans le milieu réactionnel et qui se dégradent par oxydation.

Le solvant organique doit, de préférence, solubiliser à la fois l'agent oxydant et la fuchsone présente.

De plus, il est souhaitable que le solvant organique ne soit pas trop basique c'est-à-dire que sa basicité soit telle qu'il possède un "nombre donneur" inférieur à 25. Pour apprécier la basicité d'un solvant, on se réfère au "nombre donneur". On rappelera que le "nombre donneur" ou "donor number" désigné de manière abrégée DN, donne une indication sur le caractère nucléophile du solvant et révèle son aptitude à donner son doublet.

Dans l'ouvrage de Christian REINHARDT, [Solvents and Solvent Effects in Organic Chemistry - VCH p.19 (1988)], on trouve la définition du "donor number" qui est défini comme le négatif ($-\Delta H$) de l'enthalpie (Kcal/mol) de l'interaction entre le solvant et le pentachlorure d'antimoine, dans une solution diluée de dichloroéthane.

Comme exemples de solvants organiques susceptibles d'être mis en oeuvre dans le procédé de l'invention, on peut citer plus particulièrement :

- les nitriles alphatiques ou aromatiques comme l'acétonitrile, le propionitrile, le butanenitrile, l'isobutanenitrile, le benzonitrile, le cyanure de benzyle,
- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde

de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,

- les hydrocarbures aliphatiques chlorés, par exemple, le dichlorométhane, le tétrachlorométhane, le dichloroéthane,
- les alcools tels que le méthanol, l'éthanol, l'isopropanol, le tertiobutanol.

On peut également utiliser un mélange de solvants et, en particulier un mélange de composé phénolique de formule (I) et d'un solvant organique.

La quantité de solvant organique mise en oeuvre est fonction de la solubilité de la fuchsone dans ledit solvant organique.

La concentration de la fuchsone dans le solvant organique peut varier largement. Elle est avantageusement comprise entre 0,1 et 2 moles/litre.

Conformément au procédé de l'invention, on fait réagir la fuchsone et l'agent oxydant, éventuellement en présence d'un catalyseur acide à une température qui peut être comprise entre 20°C et 150°C.

Une variante préférée du procédé de l'invention consiste à choisir la température entre 30°C et 80°C.

La réaction est conduite avantageusement sous pression atmosphérique.

D'un point de vue pratique, le procédé selon l'invention est simple à mettre en oeuvre de façon continue ou discontinue.

Les différents réactifs peuvent être introduits dans n'importe quel ordre dans la mesure où l'agent oxydant est introduit en dernier. D'une manière préférée, on choisit l'ordre des réactifs suivants : on introduit la fuchsone de formule (III) et/ou (IV), le solvant réactionnel et le catalyseur acide.

On porte le milieu réactionnel à la température désirée puis, l'on ajoute l'agent oxydant de préférence une solution de peroxyde d'hydrogène, de manière progressive.

En fin de réaction, les produits d'hydroxylation sont séparés selon les moyens usuels, notamment, par distillation.

Une variante préférée du procédé de l'invention consiste à partir d'une fuchsone de formule (III) et/ou (IV) préparée par le procédé défini ci-dessus qui consiste à faire réagir un composé phénolique présentant un atome d'hydrogène en position para du groupe hydroxyle et un composé cétonique non énolisable, en présence d'une quantité efficace d'un acide.

Conformément au procédé de l'invention, on réalise la préparation d'un composé aromatique p-dihydroxylé, en faisant réagir la fuchsone isolée avec l'agent oxydant ; la fuchsone étant isolée selon n'importe quel mode de séparation dont un exemple est donné ci-dessus à titre illustratif.

Selon une autre variante d'exécution de l'invention, il est possible en fin de synthèse de la fuchsone, d'enchaîner la réaction de celle-ci avec l'agent oxydant.

A cet effet, on introduit directement l'agent oxydant dans le milieu réactionnel comprenant la fuchsone.

L'agent oxydant est de même nature telle que décrite précédemment.

Les conditions données précédemment (température, proportions) restent valables.

Il peut être inutile d'ajouter à nouveau un catalyseur acide à l'étape de réaction de la fuchsone avec l'agent oxydant dans la mesure où la quantité d'acide mise en oeuvre lors de la préparation de la fuchsone est suffisante.

En fin de réaction, les produits d'hydroxylation sont séparés par les moyens usuels, notamment, par distillation.

Le procédé de l'invention permet d'obtenir sélectivement des composés aromatiques para-dihydroxylés.

Un autre intérêt du procédé de l'invention est que la mise en oeuvre de la fuchsone de formule (III) et/ou (IV) conduit après réaction avec l'agent oxydant, à un composé aromatique para-dihydroxylé et à une cétone de formule (II); ladite cétone pouvant être recyclée lors de la synthèse de la fuchsone de formule (III) et/ou (IV).

Poursuivant ses recherches, la demanderesse a trouvé qu'il était également possible d'obtenir un composé aromatique p-dihydroxylé par oxydation d'un composé phénolique, la p-fuchsone servant alors de catalyseur de réaction.

Plus précisément, la présente invention a pour objet un procédé de préparation d'un composé aromatique p-dihydroxylé, par réaction d'un composé phénolique présentant un atome d'hydrogène en position para avec le peroxyde d'hydrogène, en présence d'une quantité efficace d'un acide fort, ledit procédé étant caractérisé par le fait que la réaction est conduite en présence d'une quantité efficace d'une p-fuchsone.

La présente invention s'applique tout particulièrement aux composés phénoliques de formule générale (I) tels que précédemment définis.

Parmi les composés phénoliques de formule (I) qui pourront être mis en oeuvre dans le procédé de l'invention, on peut citer à titre non limitatif, le phénol, l'anisole, l'orthocrésol, le métacrésol.

Comme mentionné précédemment, la caractéristique du procédé de l'invention consiste à mettre en oeuvre une p-fuchsone, à titre de catalyseur.

Les fuchsones mises en oeuvre préférentiellement répondent aux formules (III) et/ou (IV) telles que précédemment définies et, plus particulièrement aux formules (IIIa) et/ou (IVa) et, encore plus préférentiellement aux formules (IIIb) et/ou (IVb).

Les fuchsones choisies préférentiellement sont la diphénylfuchsone, l'acide rosolique, la diphénylméthylfuchsone.

On peut faire appel dans le procédé de l'invention à toute fuchsone et notamment celles qui sont décrites dans la littérature par I. S. IOFFE et al [J. Gen. Chem. USSR, 19, p.917-28 (1949)] et H. BURTON et al [J. Chem. Soc., 3089-3090 (1955)] mais l'on préfère mettre en oeuvre celle préparée selon le procédé de l'invention, par réaction d'un composé phénolique et d'une cétone non énolisable.

En ce qui concerne le choix du catalyseur, il y a lieu de choisir la nature de la p-fuchsone de telle sorte qu'elle libère un composé aromatique p-dihydroxylé identique à celui que l'on souhaite préparer par oxydation ultérieure du composé phénolique de départ.

Conformément au procédé de l'invention, la présence de p-fuchsone au cours du procédé d'oxydation du composé phénolique de formule (I), joue sur la régiosélectivité de la réaction.

Ledit composé est mis en oeuvre en quantité catalytique.

Généralement, la quantité de fuchsone de formule (III) et/ou (IV) est inférieure à 1,0 mole par mole de peroxyde d'hydrogène. Elle est comprise généralement entre $1.10^{-3}$ mole et 1,0, de préférence entre 0,05 et 0,5.

On fait appel aux solutions de peroxyde d'hydrogène précédemment décrites.

La quantité de peroxyde d'hydrogène peut aller jusqu'à 1 mole de $H_2O_2$ pour 1 mole de composé phénolique de formule (I).

Il est cependant préférable pour obtenir un rendement industriellement acceptable d'utiliser un rapport molaire peroxyde d'hydrogène/ composé phénolique de formule (I) de 0,01 à 0,3 et, de préférence, de 0,05 à 0,10.

Afin d'avoir une vitesse de réaction suffisante, on limite la teneur initiale du milieu en eau à 20 % en poids et, de préférence, à 10 % en poids.

Les teneurs pondérales indiquées sont exprimées par rapport au mélange composé phénolique de formule (I)/peroxyde d'hydrogène/eau.

Cette eau initiale correspond à l'eau introduite avec les réactifs et notamment avec le peroxyde d'hydrogène.

Intervient dans le procédé de l'invention, un acide fort. On peut se référer aux acides forts mis en oeuvre à l'étape d'oxydation de la p-fuchsone.

On choisit, tout particulièrement, l'acide perchlorique, l'acide trifluorométhanesulfonique ou l'acide méthanesulfonique.

La quantité d'acide exprimée par le rapport du nombre d'équivalents de protons au nombre de moles de peroxyde d'hydrogène peut varier entre environ $1.10^{-4}$ et environ 1,0.

Une variante préférée du procédé de l'invention consiste à choisir un rapport $H^+/H_2O_2$ compris entre $1.10^{-3}$ et 0,1.

Conformément au procédé de l'invention, il est possible selon une variante de l'invention de mettre en oeuvre un solvant organique aprotique polaire présentant certaines caractéristiques de polarité et de basicité ; la présence dudit solvant pouvant améliorer la régio-sélectivité de la réaction.

Une première variante du procédé de l'invention consiste à faire appel à un solvant organique polaire et peu basique c'est-à-dire présentant une polarité telle que sa constante diélectrique est supérieure ou égale à 20 et une basicité telle qu'il possède un "nombre donneur" inférieur à 25.

Une deuxième variante du procédé de l'invention consiste à faire appel à un solvant organique peu polaire mais basique c'est-à-dire présentant une polarité telle que sa constante diélectrique est inférieure à environ 20 et une basicité telle qu'il possède un "nombre donneur" supérieur ou égal à 15 et inférieur à 25.

Plusieurs impératifs président au choix du solvant organique.

Une première caractéristique du solvant organique est qu'il soit aprotique et stable dans le milieu réactionnel.

On entend par solvant aprotique, un solvant qui, dans la théorie de Lewis n'a pas de protons à libérer.

Sont exclus de la présente invention, les solvants qui ne sont pas stables dans le milieu réactionnel et qui se dégradent soit par oxydation, soit par hydrolyse. A titre d'exemples de solvants réactionnels ne convenant pas à l'invention, on peut citer les solvants de type ester dérivés des acides carboxyliques tels que notamment l'acétate de méthyle ou d'éthyle, le phtalate de méthyle ou d'éthyle, le benzoate de méthyle etc...

Les solvants organiques convenant pour la mise en oeuvre du procédé de l'invention, doivent répondre à certaines exigences au niveau de leur polarité et de leur basicité qui est caractérisée par le nombre donneur.

Une première classe de solvants organiques convenant tout à fait bien à la mise en oeuvre du procédé de l'invention, sont les solvants organiques polaires et peu basiques.

On choisit, conformément à l'invention, un solvant organique qui présente une constante diélectrique supérieure ou égale à 20. La borne supérieure ne présente aucun caractère critique. On préfère faire appel à un solvant organique ayant une constante diélectrique élevée, de préférence comprise entre 25 et 75.

Le solvant organique présentant les caractéristiques de polarité précitées doit également satisfaire à certaines conditions de basicité. En effet, ledit solvant ne doit pas être trop basique. Pour déterminer si un solvant satisfait à cette exigence, on apprécie sa basicité en se référant au "nombre donneur". On choisit un solvant organique polaire présentant un nombre donneur inférieur à 25, de préférence inférieur ou égal à 20. La borne inférieure ne présente aucun caractère critique. On choisit, de préférence, un solvant organique ayant un nombre donneur compris entre 2 et 17.

En ce qui concerne l'autre classe de solvants revendiqués, les caractéristiques desdits solvants sont définis ci-après.

Les solvants appartenant à cette catégorie sont des solvants organiques peu polaires mais basiques.

On choisit, conformément à l'invention, un solvant organique qui présente une constante diélectrique inférieure à environ 20. La borne inférieure ne présente aucun caractère critique. On préfère faire appel à un solvant organique ayant une constante diélectrique faible, de préférence comprise entre 2 et 15.

En ce qui concerne sa basicité, elle doit être telle qu'il possède un "nombre donneur" supérieur ou égal à 15 et inférieur à 25. On choisit, de préférence, un solvant organique ayant un nombre donneur compris entre 15 et 25.

Afin de déterminer si le solvant organique répond aux conditions de constante diélectrique énoncées ci-dessus, on peut se reporter, entre autres, aux tables de l'ouvrage : Techniques of Chemistry, II - Organic solvents - p. 536 et suivantes, 3ème édition (1970).

Pour ce qui des exigences concernant la basicité du solvant organique à mettre en oeuvre, on se réfère à la définition du "nombre donneur" donnée précédemment.

Comme exemples de solvants organiques aprotiques, polaires répondant aux caractéristiques de basicité précitées, susceptibles d'être mis en oeuvre dans le procédé de l'invention, on peut citer plus particulièrement :

- les composés nitrés tels que le nitrométhane, le nitroéthane, le nitro-1 propane, le nitro-2 propane ou leurs mélanges, le nitrobenzène,
- les nitriles alphatiques ou aromatiques comme l'acétonitrile, le propionitrile, le butanenitrile, l'isobutanenitrile, le benzonitrile, le cyanure de benzyle,
- la tétraméthylène sulfone (sulfolane),
- le carbonate de propylène.

On peut également utiliser un mélange de solvants.

Parmi les solvants précités, on met en oeuvre, préférentiellement, l'acétonitrile.

En ce qui concerne l'autre classe de solvants revendiqués, on donne, ci-après, des exemples de solvants aprotiques peu polaires et basiques susceptibles d'être mis en oeuvre dans le procédé de l'invention :

- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les esters neutres phosphoriques tels que, notamment, le phosphate de triméthyle, le phosphate de triéthyle, le phosphate de butyle, le phosphate de triisobutyle, le phosphate de tripentyle,
- le carbonate d'éthylène.

On peut également utiliser un mélange de solvants.

La quantité de solvant organique à mettre en oeuvre est déterminée en fonction de la nature du solvant organique choisi.

Ainsi, lors de la mise en oeuvre d'un solvant organique polaire mais peu basique, elle est déterminée de telle sorte que le rapport molaire entre le nombre de moles de solvant organique et le nombre de moles de composé phénolique de formule (I) varie entre 0,1 et 2,0, de préférence, entre 0,25 et 1,0.

Si l'on fait appel à un solvant organique peu polaire et basique, la quantité mise en oeuvre est déterminée de telle sorte que le rapport molaire entre le nombre de moles de solvant organique et le nombre de moles de composé phénolique de formule (I) varie entre 0,01 et 0,25, de préférence, entre 0,025 et 0,15.

En ce qui concerne les conditions de mise en oeuvre du peroxyde d'hydrogène et de la fuchsone répondant à la formule (III) et/ou (IV), celles-ci correspondent à ce qui est décrit précédemment.

Conformément au procédé de l'invention, on réalise l'hydroxylation du composé phénolique de formule (I) à une température qui peut être comprise entre 45°C et 150°C.

Une variante préférée du procédé de l'invention consiste à choisir la température entre 60°C et 110°C.

La réaction est conduite avantageusement sous pression atmosphérique.

D'un point de vue pratique, le procédé selon l'invention est simple à mettre en oeuvre de façon continue ou

discontinue.

Les différents réactifs peuvent être introduits dans n'importe quel ordre. D'une manière préférée, on choisit l'ordre des réactifs suivants : on introduit le composé phénolique de formule (I), la fuchsone de formule (III) et/ou (IV) et le catalyseur acide.

On porte le milieu réactionnel à la température désirée puis l'on ajoute la solution de peroxyde d'hydrogène, de manière progressive.

En fin de réaction, le composé phénolique non transformé, et la cétone formée sont séparés des produits d'hydroxylation par les moyens usuels, notamment, par distillation.

Le procédé de l'invention permet ainsi d'obtenir un mélange de composés aromatiques dihydroxylés, à savoir l'isomère ortho et para avec une très bonne sélectivité en isomère para puisque le rapport para/ortho est nettement supérieur à 1, et de préférence compris entre 1,5 et 10,0.

On donne ci-après des exemples de réalisation de l'invention.

Les exemples 1 à 83 qui suivent, illustrent l'invention, sans toutefois la limiter.

## EXEMPLES 1 à 51

Les exemples 1 à 24 concernent la préparation de p-fuchsone.

Les exemples 25 à 51 illustrent la mise en oeuvre d'un composé soufré ionisable.

Dans les exemples, les abréviations suivantes signifient :

$$TT_{CETONE} = \frac{\text{nombre de moles de cétone transformées}}{\text{nombre de moles de cétone introduites}} \%$$

$$RR_{FUCHSONE} = \frac{\text{nombre de moles de fuchsone formées}}{\text{nombre de moles de cétone introduites}} \%$$

$$RT_{FUCHSONE} = \frac{\text{nombre de moles de fuchsone formées}}{\text{nombre de moles de cétone transformées}} \%$$

En ce qui concerne l'expression des résultats, les RR et RT sont donnés sans discerner la fuchsone de sa forme hydratée, le carbinol. On appelle donc fuchsone, le mélange de fuchsone et de son carbinol. Comme mentionné précédemment, la quantité de fuchsone hydratée dépend du traitement réactionnel : ce rapport est généralement voisin de 1,5.

### Exemple 1

Dans un ballon de verre de 100 ml muni d'une agitation centrale, d'un réfrigérant, d'une ampoule de coulée et d'un thermomètre, on charge :

- 19,8 g (0,2 mol) de phénol
- 18,2 g (0,1 mol) de benzophénone
- 7,5 g (0,05 mol) d'acide trifluorométhanesulfonique $CF_3SO_3H$

On porte le mélange réactionnel à la température réactionnelle choisie 70°C, tout en le maintenant sous agitation de 1200 tours/mn.

Au bout de 19 heures, on refroidit alors le mélange réactionnel et l'on effectue le dosage des produits de la réaction : la diphénylfuchsone obtenue et la benzophénone résiduelle sont dosées par chromatographie en phase gazeuse.

Les résultats obtenus sont les suivants :

- $RR_{FUCHSONE}$ = 26,2 %
- $TT_{BENZOPHENONE}$ = 28,6 %
- $RT_{FUCHSONE}$ = 91,5 %

### Exemple 2

Dans cet exemple, on reproduit l'exemple 1 sauf que la température réactionnelle est de 80°C au lieu de 70°C.

Au bout de 4 heures de réaction, les résultats obtenus sont les suivants :

- RR $_{FUCHSONE}$ = 20,8 %
- TT $_{BENZOPHENONE}$ = 20,8 %
- RT $_{FUCHSONE}$ = 100 %

Au bout de 5 heures de réaction, les résultats obtenus sont les suivants :

- RR $_{FUCHSONE}$ = 22,6 %
- TT $_{BENZOPHENONE}$ = 22,6 %
- RT $_{FUCHSONE}$ = 100 %

Exemple 3

Dans un appareillage tel que décrit dans l'exemple 1, on charge :

- 19,8 g (0,20 mol) de phénol
- 18,2 g (0,10 mol) de benzophénone
- 0,75 g (0,005 mol) d'acide trifluorométhanesulfonique $CF_3SO_3H$

On porte le mélange réactionnel à la température de 80°C, tout en le maintenant sous agitation de 1200 tours/mn.
Dans cet exemple, on reproduit l'exemple 1 sauf que la température réactionnelle est de 150°C au lieu de 70°C.
Au bout d'1 heure de réaction, les résultats obtenus sont les suivants :

- RR $_{FUCHSONE}$ = 3,8 %
- TT $_{BENZOPHENONE}$ = 4,6 %
- RT $_{FUCHSONE}$ = 83 %

Au bout de 6 heures de réaction, les résultats obtenus sont les suivants :

- RR $_{FUCHSONE}$ = 3,8 %
- TT $_{BENZOPHENONE}$ = 6,5 %
- RT $_{FUCHSONE}$ = 58,5 %

Exemple 4

Dans un appareillage tel que décrit dans l'exemple 1, on charge :

- 9,4 g (0,10 mol) de phénol
- 18,2 g (0,10 mol) de benzophénone
- 7,5 g (0,05 mol) d'acide trifluorométhanesulfonique $CF_3SO_3H$

On porte le mélange réactionnel à la température de 80°C, tout en le maintenant sous agitation de 1200 tours/mn.
Au bout de 4 heures de réaction, les résultats obtenus sont les suivants :

- RR $_{FUCHSONE}$ = 17 %
- TT $_{BENZOPHENONE}$ = 17 %
- RT $_{FUCHSONE}$ = 100 %

On ajoute alors 0,05 mol d'acide trifluorométhanesulfonique $CF_3SO_3H$.
Au bout de 6 heures 45, les résultats obtenus sont les suivants :

- RR $_{FUCHSONE}$ = 29 %
- TT $_{BENZOPHENONE}$ = 29 %
- RT $_{FUCHSONE}$ = 100 %

Exemple 5

On reproduit l'exemple 1 à la seule différence que l'on met en oeuvre de l'acide perchlorique à 70% dans une même quantité.
La température réactionnelle est aussi de 70°C.
Au bout de 28 heures de réaction, les résultats obtenus sont les suivants :

- $RR_{FUCHSONE}$ = 5,9 %
- $TT_{BENZOPHENONE}$ = 6,0 %
- $RT_{FUCHSONE}$ = 98 %

Exemple 6

Dans un appareillage tel que décrit dans l'exemple 1, on charge :

- 19,8 g (0,2 mol) de phénol
- 18,2 g (0,1 mol) de benzophénone
- 4,8 g (0,05 mol) d'acide méthanesulfonique

On porte le mélange réactionnel à la température réactionnelle choisie de 150°C, tout en le maintenant sous agitation de 1200 tours/mn.
Au bout de 5 heures de réaction, les résultats obtenus sont les suivants :

- $RR_{FUCHSONE}$ = 8 %
- $TT_{BENZOPHENONE}$ = 15 %
- $RT_{FUCHSONE}$ = 53 %

Exemple 7

On reproduit l'exemple précédent mais en remplacant l'acide méthanesulfonique par l'acide benzènesulfonique.
La température réactionnelle choisie est de 120°C.
Au bout de 4 heures 30 minutes de réaction, les résultats obtenus sont les suivants :

- $RR_{FUCHSONE}$ = 1,5 %
- $TT_{BENZOPHENONE}$ = 2,4 %
- $RT_{FUCHSONE}$ =63 %

Exemple 8

On reproduit l'exemple 6 à la seule différence que l'on remplace l'acide méthanesulfonique par l'acide pyrophosphorique.
Au bout de 3 heures de réaction, les résultats obtenus sont les suivants :

- $RR_{FUCHSONE}$ = 1 %
- $TT_{BENZOPHENONE}$ = 1 %
- $RT_{FUCHSONE}$ = 100 %

Exemple 9

Dans un appareillage tel que décrit dans l'exemple 1, on charge :

- 19,8 g (0,2 mol) de phénol
- 9,2 g (0,1 mol) de benzophénone
- 1,1 g (0,014 mol) d'acide bromhydrique

L'acide bromhydrique est introduit sous forme gazeuse par bullage de 1,1 g dans le mélange réactionnel, pendant 20 minutes.

La température réactionnelle choisie est de 75 °C.
Au bout de 6 heures de réaction, les résultats obtenus sont les suivants :

- RR $_{FUCHSONE}$ = 2,9 %
- TT $_{BENZOPHENONE}$ = 3,2 %
- RT $_{FUCHSONE}$ = 90,6 %

Exemple 10

On reproduit l'exemple 9 mais en substituant l'acide bromhydrique par l'acide chlorhydrique gazeux, par bullage pendant toute la durée de l'essai. On opère en présence d'1 ml d'eau.
Au bout de 16 heures de réaction, les résultats obtenus sont les suivants :

- RR $_{FUCHSONE}$ = 1 %
- TT $_{BENZOPHENONE}$ = 1 %
- RT $_{FUCHSONE}$ = 100 %

Exemple 11

Dans un réacteur métallique de 100 ml, on charge :

- 4,7 g (0,050 mol) de phénol
- 9,1 g (0,05 mol) de benzophénone
- 20 g (1,00 mol) d'acide fluorhydrique anhydre

On ferme le réacteur et l'on porte le mélange réactionnel à la température choisie de 80°C, tout en le maintenant sous agitation.
Au bout de 4 heures, le mélange réactionnel est envoyé dans 100 ml d'eau ; on extrait par le dichlorométhane. La phase organique obtenue est lavée à l'eau jusqu'à pH = 5. On sépare les phases aqueuse et organique.
On effectue ensuite les dosages de la benzophénone et de la fuchsone.
Les résultats obtenus sont les suivants :

- RR $_{FUCHSONE}$ = 31 %
- TT $_{BENZOPHENONE}$ = 36 %
- RT $_{FUCHSONE}$ = 86 %

Exemple 12

Dans cet exemple, on reproduit l'exemple 11 sauf que la température réactionnelle est de 120°C au lieu de 80°C.
Au bout de 4 heures de réaction, les résultats obtenus sont les suivants :

- RR $_{FUCHSONE}$ = 10 %
- TT $_{BENZOPHENONE}$ = 18 %
- RT $_{FUCHSONE}$ = 55,5 %

Exemple 13

Dans un appareillage tel que décrit dans l'exemple 1, on charge :

- 18,8 g (0,2 mol) de phénol
- 18,2 g (0,1 mol) de benzophénone
- 6,65 g (0,5 mol) de chlorure d'aluminium $AlCl_3$

On porte le mélange réactionnel à 80°C tout en le maintenant sous agitation.
Au bout de 4 heures de réaction, on effectue le dosage des produits formés après hydrolyse acide à l'aide de 100 ml d'une solution aqueuse chlorhydrique 1,2 N et extraction des produits organiques par l'acétate d'éthyle.
Les résultats obtenus sont les suivants :

- RR $_{FUCHSONE}$ = 9 %
- TT $_{BENZOPHENONE}$ = 16 %
- RT $_{FUCHSONE}$ = 56 %

Exemple 14

On reproduit l'exemple 13 à la seule différence que l'on remplace le chlorure d'aluminium par le chlorure de titane $TiCl_4$.

Au bout de 3 heures de réaction, les résultats obtenus sont les suivants :

- RR $_{FUCHSONE}$ = 1,0 %
- TT $_{BENZOPHENONE}$ = 6,5 %
- RT $_{FUCHSONE}$ = 15,5 %

Exemple 15

Dans un appareillage tel que décrit dans l'exemple 1, on charge :

- 9,4 g (0,20 mol) de phénol
- 9,1 g (0,10 mol) de benzophénone
- 2,0 g de zéolithe US-Y

La zéolithe US-Y mise en oeuvre est une zéolithe commercialisée par la société TOSOH ayant un rapport molaire Si/Al égal à 6,0.

La température réactionnelle choisie est de 150°C.

Au bout de 5 heures de réaction, les résultats obtenus sont les suivants :

- RR $_{FUCHSONE}$ = 2 %
- TT $_{BENZOPHENONE}$ = 2 %
- RT $_{FUCHSONE}$ = 100 %

Exemple 16

On reproduit l'exemple 15 à la différence près que l'on remplace la zéolithe US-Y par une argile KO de type VOLCLAY.

Au bout de 5 heures 30 de réaction, les résultats obtenus sont les suivants :

- RR $_{FUCHSONE}$ = 2 %
- TT $_{BENZOPHENONE}$ = 2 %
- RT $_{FUCHSONE}$ = 100 %

Exemple 17

Dans un appareillage tel que décrit dans l'exemple 1, on charge :

- 0,20 mol de phénol
- 0,10 mol de benzophénone
- 0,05 mol équivalent $H^+$ d'une résine sulfonique.

La résine sulfonique mise en oeuvre est une résine perfluorée préparée à partir d'un copolymère de tétrafluoroéthylène et de perfluoro [2-(fluorosulfonyléthoxy)-propyl] vinyl éther vendue sous la dénomination commerciale de NAFION®.

La température de la réaction est de 145°C.

Au bout de 3 heures de réaction, on obtient les résultats suivants :

- RR $_{FUCHSONE}$ = 14 %
- TT $_{BENZOPHENONE}$ = 16,8 %
- RT $_{FUCHSONE}$ = 83,5 %

Exemple 18

Dans un appareillage tel que décrit dans l'exemple 1, on charge :

- 9,4 g (0,1 mol) de phénol
- 2,14 g (0,01 mol) de 4,4'-dihydroxybenzophénone
- 3,1 g (0,02 mol) d'acide trifluorométhanesulfonique $CF_3SO_3H$

La température de la réaction est de 150°C.
Au bout de 4 heures de réaction, on obtient les résultats suivants :

- RR $_{\text{ACIDE ROSOLIQUE}}$ = 5 %
- TT $_{\text{CETONE}}$ = 15 %
- RT $_{\text{ACIDE ROSOLIQUE}}$ = 33,5 %

Exemple 19

Dans un appareillage tel que décrit dans l'exemple 1, on charge :

- 21,6 g (0,2 mol) d'ortho-crésol
- 18,2 g (0,1 mol) de benzophénone
- 7,5 g (0,05 mol) d'acide trifluorométhanesulfonique $CF_3SO_3H$

La température de la réaction est de 80°C.
Au bout de 3 heures de réaction, on obtient les résultats suivants :

- RR $_{\text{METHYLFUCHSONE}}$ = 25%
- TT $_{\text{BENZOPHENONE}}$ = 26,3%
- RT $_{\text{METHYLFUCHSONE}}$ = 95 %

Exemple 20

Dans un appareillage tel que décrit dans l'exemple 1, on charge :

- 0,22 mol de phénol
- 0,02 mol de benzophénone
- 0,20 mol d'acide trifluorométhanesulfonique hydraté $CF_3SO_3H$, $H_2O$

La température de la réaction est de 120°C.
Au bout de 4 heures de réaction, on obtient les résultats suivants :

- RR $_{\text{FUCHSONE}}$ = 66 %
- TT $_{\text{BENZOPHENONE}}$ = 84,5 %
- RT $_{\text{FUCHSONE}}$ = 78 %

Exemple 21

Dans un appareillage tel que décrit dans l'exemple 1, on charge :

- 23,5 g (0,25 mol) de phénol
- 4,55 g (0,025 mol) de benzophénone
- 7,5 g (0,05 mol) d'acide trifluorométhanesulfonique
- du p-xylène en quantité telle que le volume total atteigne 50 ml.

On porte le mélange réactionnel à 110°C, tout en maintenant sous agitation de 1200 tours/mn.
Après 4 heures, on refroidit alors le mélange réactionnel et l'on effectue le dosage des produits de réaction.
Les résultats obtenus sont les suivants :

- RR $_{FUCHSONE}$ = 68 %
- TT $_{BENZOPHENONE}$ = 84,5 %
- RT $_{FUCHSONE}$ = 80,5 %

Exemple 22

Dans un appareillage tel que décrit dans l'exemple 1, on charge :

- 4,7 g (0,05 mol) de phénol
- 0,91 g (0,005 mol) de benzophénone
- 4,8 g (0,05 mol) d'acide méthanesulfonique
- 30 ml de méthycyclohexane

On porte le mélange réactionnel à 80°C, tout en maintenant sous agitation de 1200 tours/mn. Le milieu est biphasique.

Après 5 heures, on refroidit alors le mélange réactionnel et, dans chaque phase, l'on effectue le dosage des produits de réaction.

Les résultats obtenus sont les suivants :

- RR $_{FUCHSONE}$ = 15 %
- TT $_{BENZOPHENONE}$ = 20 %
- RT $_{FUCHSONE}$ = 75 %

Exemple 23

On opère comme dans l'exemple 22 sauf que l'on remplace le méthylcyclohexane par le même volume de cyclohexane et que l'on élimine l'eau par distillation azéotropique.

Les résultats obtenus sont les suivants :

- RR $_{FUCHSONE}$ = 24 %
- TT $_{BENZOPHENONE}$ = 28 %
- RT $_{FUCHSONE}$ = 85,5 %

Exemple 24

Dans un appareillage tel que décrit dans l'exemple 1, on charge :

- 56,4 g (0,6 mol) de phénol
- 10,92 g (0,06 mol) de benzophénone
- 46,06 g (0,48 mol) d'acide méthanesulfonique

On porte le mélange réactionnel à 100°C pendant 7 heures, tout en maintenant sous agitation de 1200 tours/mn.

On refroidit alors le mélange réactionnel puis on ajoute 50 ml d'eau et 100 ml d'éther isopropylique. On décante la phase éthérée qui est lavée 3 fois par 50 ml d'eau.

Dans la phase organique éthérée, on dose :

- FUCHSONE = 0,0347 mol (RR = 58,3 %)
- BENZOPHENONE = 0,0240 mol (TT = 59,8 %).

EXEMPLES 25 à 52

Exemple 25

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone

- 7,9 g (0,05 mol) d'acide benzènesulfonique
- 0,621 g (0,005 mol) de benzylmercaptan

On porte le mélange réactionnel à la température de réaction choisie, 110°C, tout en maintenant l'agitation à 1200 tours/mn.
Au bout de 4 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivants:

- $RR_{FUCHSONE}$ = 57,6 %
- $TT_{BENZOPHENONE}$ = 62 %
- $RT_{FUCHSONE}$ = 92,9 %

Le même essai effectué sans benzylmercaptan, conauit après 4 heures de réaction à 110°C, aux résultats suivants:

- $RR_{FUCHSONE}$ = 14 %
- $TT_{BENZOPHENONE}$ = 15,2 %
- $RT_{FUCHSONE}$ = 92,1 %

La figure 1 représente un graphe sur lequel sont portées deux courbes de variation du rendement (RR exprimé en %) en fonction de la durée de la réaction (exprimée en heures), l'une en présence de benzylmercaptan (B) et l'autre en l'absence de thiol (A).
On note que la présence d'un thiol permet d'accroître considérablement la vitesse de formation de la fuchsone.

Exemple 26

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone
- 7,5 g (0,05 mol) d'acide trifluoromethanesulfonique
- 0,621 g (0,005 mol) de benzylmercaptan

On porte le mélange réactionnel à la température de réaction choisie, 80°C tout en maintenant l'agitation à 1200 tours/mn.
Au bout de 4 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivants:

- $RR_{FUCHSONE}$ = 94,8 %
- $TT_{BENZOPHENONE}$ = 99,2 %
- $RT_{FUCHSONE}$ = 95,6 %

Le même essai effectué sans benzylmercaptan, conduit après 4 heures de réaction à 80°C, aux résultats suivants:

- $RR_{FUCHSONE}$ = 63,2 %
- $TT_{BENZOPHENONE}$ = 64,8%
- $RT_{FUCHSONE}$ = 97,5 %

Exemple 27

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone
- 7,9 g (0,05 mol) d'acide benzènesulfonique

- 0,801 g (0,005 mol) de 1-nonylmercaptan

On porte le mélange réactionnel à la température de réaction choisie, 110°C tout en maintenant l'agitation à 1200 tours/mn.
Au bout de 4 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivants:

- $RR_{FUCHSONE}$ = 62 %
- $TT_{BENZOPHENONE}$ =67,6 %
- $RT_{FUCHSONE}$ = 91,7 %

Le même essai effectué sans 1-nonylmercaptan, conduit après 4 heures de réaction à 110°C, aux résultats suivants:

- $RR_{FUCHSONE}$ = 14 %
- $TT_{BENZOPHENONE}$ = 15,2 %
- $RT_{FUCHSONE}$ = 92,1 %

Exemple 28

Dans un ballon de verre de 250 ml, muni d'une agitation centrale, d'un réfrigérant, d'un thermomètre, on charge:

- 94 g (1 mol) de phénol
- 18,2 g (0,1 mol) de benzophénone
- 76,8 g (0,8 mol) d'acide méthanesulfonique
- 1,8 g (0,02 mol) de 1-butanethiol

On porte le mélange réactionnel à la température de réaction choisie, 80°C tout en maintenant l'agitation à 1200 tours/mn.
Au bout de 7 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivants:

- $RR_{FUCHSONE}$ = 96,5 %
- $TT_{BENZOPHENONE}$ = 97,4 %
- $RT_{FUCHSONE}$ = 99,1 %

Exemple 29

Dans un ballon de verre de 100 ml, muni d'une agitation centrale, d'un réfrigérant, d'un thermomètre, on charge:

- 28,2 g (0,3 mol) de phénol
- 5,46 g (0,03 mol) de benzophénone
- 23,04 g (0,24 mol) d'acide méthanesulfonique
- 0,984 g (0,006 mol) de 2-mercaptoéthanesulfonate de sodium

On porte le mélange réactionnel à la température de réaction choisie, 110°C tout en maintenant l'agitation à 1200 tours/mn.
Au bout de 4 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivant :

- $RR_{FUCHSONE}$ = 83,7 %
- $TT_{BENZOPHENONE}$ = 99 %
- $RT_{FUCHSONE}$ = 84,5 %

Le même essai est effectué sans thiol. Les résultats obtenus sont les suivants :

- RR $_{FUCHSONE}$ = 46,3 %
- TT $_{BENZOPHENONE}$ = 47 %
- RT $_{FUCHSONE}$ = 98,5 %

Exemple 30

On reproduit l'exemple 29 sauf que l'on met en oeuvre 0,246 g (0,0015 mol) de 2-mercaptoéthanesulfonate de sodium au lieu de 0,984 g (0,006 mol). Au bout de 4 heures à 110°C, les résultats obtenus sont les suivants :

- RR $_{FUCHSONE}$ = 86,3 %
- TT $_{BENZOPHENONE}$ = 91 %
- RT $_{FUCHSONE}$ = 94,8 %

Exemple 31

On reproduit l'exemple 29 sauf que l'on met en oeuvre 0,025 g (0,00015 mol) de 2-mercaptoéthanesulfonate de sodium au lieu de 0,984 g (0,006 mol).
Au bout de 4 heures à 110°C, les résultats obtenus sont les suivants :

- RR $_{FUCHSONE}$ = 65 %
- TT $_{BENZOPHENONE}$ = 65,7 %
- RT $_{FUCHSONE}$ = 98,9 %

Exemple 32

Dans un réacteur muni d'une agitation centrale, d'un réfrigérant et d'un thermomètre, on charge:

- 8,04g (0,085 mol) de phénol
- 1,55g (0,0085 mol) de benzophénone
- 1,67g (0,0085 équivalent H+) de résine BAYER K2431
- 0,212 g (0,0017 mol) de benzylmercaptan

La résine BAYER K2431 est une résine macroporeuse à squelette polystyrénique portant des groupes sulfonique, avec une concentration de sites acides de 5,1 milliéquivalents d'$H^+$ par gramme de polymère sec.
On porte le mélange réactionnel à la température choisie, 145°C, tout en maintenant l'agitation à 1200 tours/mn.
Après 4 heures de réaction, la résine est séparée du mélange réactionnel par filtration sur verre fritté, lavée avec 20ml de phénol à 80°C. On réunit le filtrat et le lavage.
La résine est agitée 1 heure à température ambiante avec un mélange d'eau et d'acétate d'éthyle 50/50, puis filtrée. La phase organique est séparée par décantation.
La diphénylfuchsone et la benzophénone résiduelle contenues dans la phase phénolique et dans la phase acétate d'éthyle, sont dosées par chromatographie en phase en phase gazeuse.
Les résultats obtenus sont les suivants:

- RR $_{FUCHSONE}$ = 11,8 %
- TT $_{BENZOPHENONE}$ = 14 %
- RT $_{FUCHSONE}$ = 84,3 %

Le même essai réalisé sans benzylmercaptan, après 4 heures de réaction à 145°C, conduit aux résultats suivants:

- RR $_{FUCHSONE}$ = 6,2 %
- TT $_{BENZOPHENONE}$ = 6,2 %
- RT $_{FUCHSONE}$ = 100 %

Exemple 33

Dans un réacteur muni d'une agitation centrale, d'un réfrigérant et d'un thermomètre, on charge:

- 8,04g (0,085 mol) de phénol
- 1,55g (0,0085 mol) de benzophénone
- 1,67g (0,0085 équivalent H+) de résine BAYER K2431
- 0,274g (0,0017 mol) de 1-nonylmercaptan

On porte le mélange réactionnel à la température choisie 145°C tout en maintenant l'agitation à 1200 tours/mn.
Après 4 heures de réaction, la résine est séparée du mélange réactionnel par filtration sur verre fritté, lavée avec 20ml de phénol à 80°C. On réunit le filtrat et le lavage.
La résine est agitée 1 heure à température ambiante avec un mélange d'eau et d'acétate d'éthyle 50/50, puis filtrée. La phase organique est séparée par décantation.
La diphénylfuchsone et la benzophénone résiduelle contenues dans la phase phénolique et dans la phase acétate d'éthyle, sont dosées par chromatographie en phase en phase gazeuse.
Les résultats obtenus sont les suivants:

- $RR_{FUCHSONE}$ = 11,6 %
- $TT_{BENZOPHENONE}$ = 17,8 %
- $RT_{FUCHSONE}$= 65,17 %

Le même essai réalisé sans 1-nonylmercaptan, après 4 heures de réaction à 145°C conduit aux résultats suivants:

- $RR_{FUCHSONE}$ = 6,2 %
- $TT_{BENZOPHENONE}$ = 6,2 %
- $RT_{FUCHSONE}$ = 100 %

Exemple 34

Dans un réacteur de 50 ml muni d'une agitation centrale, d'un thermomètre, et d'un réfrigérant, on charge:

- 0,47 g (0,005 mol) de phénol
- 0,91g (0,005 mol) de benzophénone
- 8,9 g (0,05 mol) d'acide orthophosphorique
- 0,164 g (0,001 mol) de 2-mercaptoéthanesufonate de sodium

On porte le milieu réactionnel à la température choisie, 80°C en maintenant l'agitation à 1200 tours/mn.
Au bout de 5 heures de réaction, le mélange réactionnel est refroidi et l'on effectue le dosage des produits de la réaction. La diphénylfuchsone et la benzophénone résiduelle sont dosées par chromatographie liquide haute performance.
Les résultats obtenus sont les suivants:

- $RR_{FUCHSONE}$ = 29,6 %
- $TT_{BENZOPHENONE}$ = 35,6 %
- $RT_{FUCHSONE}$ = 83,1 %

Le même essai réalisé sans 2-mercaptoéthanethiol, conduit après 5 heures de réaction à 80°C aux résultats suivants:

- $RR_{FUCHSONE}$ = 4 %
- $TT_{BENZOPHENONE}$ = 4 %
- $RT_{FUCHSONE}$ = 100 %

Exemple 35

Dans un réacteur de 50 ml muni d'une agitation centrale, d'un thermomètre, et d'un réfrigérant, on charge:

- 0,47 g (0,005 mol) de phénol
- 0,91g (0,005 mol) de benzophénone
- 8,9 g (0,05 mol) d'acide orthophosphorique
- 0,164 g (0,001 mol) de 2-mercaptoéthanesufonate de sodium

On porte le milieu réactionnel à la température choisie, 110°C en maintenant l'agitation à 1200 tours/mn.
Au bout de 5 heures de réaction le mélange réactionnel est refroidi et l'on effectue le dosage des produits de la réaction. La diphénylfuchsone et la benzophénone résiduelle sont dosées par chromatographie liquide haute performance.
Les résultats obtenus sont les suivants:

- $RR_{FUCHSONE}$ = 30,4 %
- $TT_{BENZOPHENONE}$ = 49,6 %
- $RT_{FUCHSONE}$ = 61,3 %

Le même essai réalisé sans 2-mercaptoéthanethiol, conduit après 5 heures de réaction à 110°C aux résultats suivants:

- $RR_{FUCHSONE}$ = 3 %
- $TT_{BENZOPHENONE}$ = 10 %
- $RT_{FUCHSONE}$ = 30 %

<u>Exemples 36 à 49</u>

Dans un ballon de verre de 100 ml, muni d'une agitation centrale, d'un réfrigérant, d'un thermomètre, on charge :

- 28,2 g (0,3 mol) de phénol
- 23,04 g (0,24 mol) d'acide méthanesulfonique.

On ajoute de la benzophénone et du 2-mercaptoéthanesulfonate de sodium dans les rapports molaires indiqués dans le tableau (I).
On porte les mélanges réactionnels aux températures choisies, tout en maintenant l'agitation à 1200 tours/mn.
En fin de réaction, on refroidit les mélanges réactionnels et l'on effectue les dosages des produits de la réaction.
Les résultats et les conditions opératoires de chaque essai sont donnés dans le tableau (I).

Tableau (I)

| Réf. | Réactifs (nombre de moles) | | | | T°C de réaction | Rendements | | | | Rendements | | | |
|---|---|---|---|---|---|---|---|---|---|---|---|---|---|
| | Phénol | Ph2CO | MeSO3H | Thiol | (°C) | durée heures | TT Ph2CO | RR fuchsone | RT fuchsone | durée heures | TT Ph2CO | RR fuchsone | RT fuchsone |
| 36 | 10 | 1 | 8 | 0,2 | 80 | 4 | 96,4 | 75,8 | 78,6 | 5 | 97,6 | 79,6 | 81,6 |
| 37 | 10 | 1 | 8 | 0,05 | 80 | 4 | 80 | 75 | 93,8 | 7 | 88,7 | 86 | 97 |
| 38 | 10 | 1 | 8 | 0,05 | 140 | 4 | 95 | 88 | 92,6 | - | - | - | - |
| 39 | 10 | 1,5 | 8 | 0,05 | 110 | 5 | 76,9 | 76 | 98,8 | 7 | 80,7 | 78,4 | 97,2 |
| 40 | 10 | 1 | 8 | 0,2 | 110 | 4 | 99 | 83,7 | 84,5 | - | - | - | - |
| 41 | 10 | 0,5 | 8 | 0 | 110 | 4 | 56,6 | 55,3 | 97,7 | 7 | 74 | 70,7 | 95,5 |
| 42 | 10 | 1 | 8 | 0 | 140 | 5 | 84,8 | 73 | 86,1 | 7 | 89,7 | 77,8 | 86,7 |
| 43 | 10 | 1 | 8 | 0 | 110 | 4 | 45,2 | 43,7 | 96,6 | 7 | 60,5 | 57,8 | 95,5 |
| 44 | 10 | 1 | 8 | 0 | 110 | 4 | 43,6 | 41,3 | 94,8 | 7 | 59 | 58,7 | 99,4 |
| 45 | 10 | 2 | 8 | 0 | 110 | 4 | 34 | 33,5 | 98,5 | 7 | 44 | 43,5 | 98,9 |
| 46 | 10 | 2 | 8 | 0,005 | 110 | 4 | 41 | 40,5 | 98,8 | 7 | 50 | 50 | 100 |
| 47 | 10 | 2 | 8 | 0,05 | 110 | 4 | 59,5 | 58 | 97,5 | 7 | 66,5 | 65 | 97,7 |
| 48 | 10 | 2 | 8 | 0,1 | 110 | 4 | 67 | 64,5 | 96,3 | 7 | 74,5 | 67,5 | 90,6 |
| 49 | 10 | 3 | 8 | 0 | 110 | 4 | 22,3 | 22,3 | 100 | 7 | 30 | 30 | 100 |

Exemple 50

Dans un appareillage tel que précédemment décrit, on charge :

- 4,7 g (0,05 mol) de phénol
- 0,91 g (0,005 mol) de benzophénone
- 4,8 g (0,05 mol) d'acide méthanesulfonique
- 0,164 g (0,001 mol) de 2-mercaptoéthanesulfonate de sodium
- 30 ml de cyclohexane

On chauffe au reflux pendant 5 heures en éliminant azéotropiquement l'eau formée.
On refroidit alors la solution, on ajoute 10 ml d'eau et l'on extrait par de l'acétate d'éthyle.
Les résultats obtenus sont les suivants :

- $TT_{BENZOPHENONE}$ = 100 %
- $RR_{FUCHSONE}$ = 96 %

Exemple 51

Dans un appareillage tel que précédemment décrit, on charge :

- 4,7 g (0,05 mol) de phénol
- 9,1 g (0,05 mol) de benzophénone
- 89 g (0,5 mol) d'acide pyrophosphorique
- 1,64 g (0,01 mol) de 2-mercaptoéthanesulfonate de sodium

On porte ce mélange à 110°C pendant 5 heures, tout en agitant à 1200 tours/mn.
On refroidit alors la masse réactionnelle et l'on dose la benzophénone résiduelle et la diphénylfuchsone formée.
Les résultats obtenus sont les suivants :

- $TT_{BENZOPHENONE}$ = 50 %
- $RR_{FUCHSONE}$ = 31 %

EXEMPLES 52 A 66

Les exemples 52 à 66 illustrent la préparation d'un composé para-hydroxylé par oxydation d'une p-fuchsone.
Dans les exemples, les abréviations suivantes signifient :

$$TT_{H2O2} = \frac{\text{nombre de moles de peroxyde d'hydrogène transformées}}{\text{nombre de moles de peroxyde d'hydrogène introduites}} \%$$

$$TT_{FUCHSONE} = \frac{\text{nombre de moles de fuchsone transformées}}{\text{nombre de moles de fuchsone introduites}} \%$$

$$RR_{HQ/FUCHSONE} = \frac{\text{nombre de moles d'hydroquinone formées}}{\text{nombre de moles de fuchsone introduites}} \%$$

$$RT_{HQ/FUCHSONE} = \frac{\text{nombre de moles d'hydroquinone formées}}{\text{nombre de moles de fuchsone transformées}} \%$$

$$RT_{PC/FUCHSONE} = \frac{\text{nombre de moles de pyrocatéchine formées}}{\text{nombre de moles de fuchsone transformées}} \%$$

$$RT_{HQ/H2O2} = \frac{\text{nombre de moles d'hydroquinone formées}}{\text{nombre de moles de } H_2O_2 \text{ transformées}} \%$$

$$RT_{PC/H2O2} = \frac{\text{nombre de moles dr pyrocatéchine formées}}{\text{nombre de moles de } H_2O_2 \text{ transformées}} \%$$

Exemple 52

Dans un ballon en verre de 25 ml muni d'une agitation centrale, d'un réfrigérant, d'une ampoule de coulée et d'un thermomètre, on charge :

- 0,774 g (0,003 mol) de diphénylfuchsone
- 7,7 ml d'acétonitrile
- 18 mg (0,12 mmol) d'acide perchlorique à 70 %.

On porte le mélange réactionnel à la température réactionnelle choisie 30°C, tout en le maintenant sous agitation.
On introduit par l'intermédiaire de l'ampoule de coulée, en 2 minutes, 0,146 g (0,003 mol) de peroxyde d'hydrogène sous la forme d'une solution aqueuse à 70,5 %. La température s'élève d'elle-même à 60°C.
On refroidit alors le mélange réactionnel et l'on effectue le dosage des produits de réaction : le peroxyde d'hydrogène résiduel est dosé par iodométrie et les diphénols formés sont dosés par chromatographie liquide, haute performance.
Au bout de 1 heure de réaction, les résultats obtenus sont les suivants :

- $TT_{FUCHSONE}$ = 100 %
- $RR_{HYDROQUINONE/FUCHSONE}$ = 96,5 %
- $RT_{HYDROQUINONE/FUCHSONE}$ = 96,5 %

On récupère la benzophénone avec un taux de récupération de 96 %.

Exemple 53

Dans cet exemple, on réalise la préparation d'hydroquinone par réaction de la diphénylfuchsone obtenue à partir de phénol et de benzophénone, avec le peroxyde d'hydrogène, en présence d'acide perchlorique
Dans un réacteur à agitation contrôlée et thermorégulé, on charge :

- 19,8 g (0,2 mol) de phénol
- 18,2 g (0,1 mol) de benzophénone
- 7,5 g (0,05 mol) d'acide trifluorométhanesulfonique $CF_3SO_3H$ anhydre

On porte le mélange réactionnel à la température réactionnelle choisie 70°C, tout en le maintenant sous agitation.
Au bout de 19 heures, on refroidit alors le mélange réactionnel.
A la masse réactionnelle, on ajoute 100 ml d'acétate d'éthyle. On lave cette phase organique par 3 x 20 ml d'eau puis par 1 x 30 ml d'une solution aqueuse saturée de $NaHCO_3$. On lave ensuite par 2 x 20 ml d'eau.
On sépare les phases aqueuse et organique.
La phase organique est séchée sur $MgSO_4$ pendant 1 heure. On filtre et on lave $MgSO_4$ par 20 ml d'acétate d'éthyle que l'on joint à la phase organique.
On évapore l'acétate d'éthyle sous pression réduite de 25 mm de mercure.
Dans cette masse, on dose :

- benzophénone = 13 g (71,4 mmol) [$TT_{BENZOPHENONE}$ = 28,6 %]
- fuchsone = 6,75 g ( 26,2 mmol) [$RR_{FUCHSONE}$ = 26,2 %]

On place cette masse réactionnelle dans un réacteur.
On y introduit une quantité de peroxyde d'hydrogène correspondant à la quantité stoechiométrique sous la forme d'une solution aqueuse de peroxyde d'hydrogène à 71 % (soit 26,2 mmol $H_2O_2$ pure).
Après 5 heures à 25°C, le rendement $RR_{HYDROQUINONE/FUCHSONE}$ est de 33%.
On ajoute 0,2 mmol d'acide perchlorique à 70% poids.
Après 15 minutes, on dose par chromatographie en phase gazeuse, l'hydroquinone formée :

- $TT_{FUCHSONE}$ = 100 %
- $RR_{HYDROQUINONE/FUCHSONE}$ = 97,8 %

Le taux de récupération de la benzophénone est de 97,7 %.

Exemple 54

Dans cet exemple, on effectue la préparation de la méthylhydroquinone.
Dans le réacteur à agitation contrôlée et thermorégulé on charge :

- 21,6 g (0,200 mol) d'o-crésol
- 18,2 g (0,100 mol) de benzophénone
- 7,8 g (0,052 mol) d'acide trifluorométhanesulfonique $CF_3SO_3H$ anhydre

On chauffe à 80°C pendant 3 heures.
A la masse réactionnelle on ajoute 140 ml d'acétate d'éthyle. On lave cette phase organique par 2 x 30 ml d'$H_2O$ puis par 3 x 30 ml d'une solution aqueuse saturée de $NaHCO_3$. On lave ensuite par 2 x 30 ml de $H_2O$.
On sépare les phases aqueuse et organique.
La phase organique est séchée sur $MgSO_4$ pendant 1 heure. On filtre et on lave $MgSO_4$ par 20 ml d'acétate d'éthyle que l'on joint à la phase organique.
On évapore l'acétate d'éthyle sous pression réduite de 25 mm de mercure (poids obtenu = 38,6 g).
Dans cette masse, on dose :

- benzophénone = 13,41 g (73,7 mmol) [$TT_{BENZOPHENONE}$ = 26,3 %]
- diphénylméthylfuchsone = 6,8 g (25 mmol) [ $RR_{FUCHSONE}$ = 25 %]
- o-crésol = 20,1 g

On place cette masse réactionnelle dans un réacteur et l'on ajoute 37 mg (0,26 mmol) d'acide perchlorique à 70% poids. On porte le mélange réactionnel à 30-35°C et on y introduit 1,225 g d'une solution aqueuse de peroxyde d'hydrogène à 71 % (soit 25,5 mmol $H_2O_2$ pure).
Après 1 heure 30 minutes, la conversion de $H_2O_2$ est de 90%.
On dose par chromatographie en phase gazeuse, la méthylhydroquinone formée soit 2,85 g (23 mmol) ce qui correspond à un rendement $RR_{METHYLHYDROQUINONE/FUCHSONE}$ de 92,5 %.
Le taux de récupération de la benzophénone est de 99,1 %.

Exemples 55 et 56

Dans ces exemples, on met en oeuvre le phénol, la benzophénone, la diphénylfuchsone et l'acide perchlorique dans les rapports molaires suivants : 4/4/1/0,015.
On porte le milieu réactionnel à une température de 75°C et l'on coule une solution aqueuse de peroxyde d'hydrogène, tout en maintenant la température à
75°C. Au bout d'une heure, les résultats obtenus sont les suivants :

Tableau (II)

| Ex. | Rapport molaire $H_2O_2$/ fuchsone | $TT_{FUCHSONE}$ | $TT_{H2O2}$ | % de benzophénone récupérée | $RT_{HQ}$ | $RT_{PC}$ |
|---|---|---|---|---|---|---|
| 55 | 1,04 | 92,5 | 100 | 91 | 90 | 1 |
| 56 | 1,32 | 98,0 | 100 | 95 | 86 | 5 |

Exemple 57

Dans un ballon de verre de 100 ml muni d'une agitation centrale, d'un réfrigérant, d'une ampoule de coulée et d'un thermomètre, on charge :

- 19,8 g (0,2 mol) de phénol
- 18,2 g (0,1 mol) de benzophénone

- 7,5 g (0,05 mol) d'acide trifluorométhanesulfonique $CF_3SO_3H$

On porte le mélange réactionnel à la température réactionnelle choisie 80°C, tout en le maintenant sous agitation.
Au bout de 22 heures, on refroidit alors le mélange réactionnel et l'on effectue le dosage des produits de la réaction : la diphénylfuchsone obtenue est dosée par chromatographie en phase gazeuse.
Les résultats obtenus sont les suivants :

- benzophénone = 12,55 g (69 mmol) [$TT_{BENZOPHENONE}$ = 31 %]
- fuchsone = 7,87 g (30,5 mmol) [$RR_{FUCHSONE}$ = 30,5 %, $RT_{FUCHSONE}$ = 98,4 %]

On y introduit en 15 minutes, 0,96 g d'une solution aqueuse de peroxyde d'hydrogène à 71 % (soit 20,0 mmol $H_2O_2$ pure).
Après 30 minutes, les résultats obtenus sont les suivants :

- $RT_{HYDROQUINONE/\ H2O2}$ = 60,0 %
- $RT_{PYROCATECHINE/\ H2O2}$ =14,0 %
- Rapport $_{HQ/PC}$= 4,3

Exemple 58

On reproduit l'exemple 57 à la seule différence que l'on met en oeuvre de l'acide perchlorique à 70 % dans une même quantité.
La température réactionnelle est aussi de 70°C.
Au bout de 28 heures de réaction, les résultats obtenus sont les suivants :

- benzophénone = 17,1 g (94 mmol) [TT $_{BENZOPHENONE}$ = 6,0 %]
- fuchsone = 1,52 g (5,9 mmol) [$RR_{FUCHSONE}$ = 5,9 % $RT_{FUCHSONE}$ = 98 %]

Après refroidissement, on y introduit 0,31 g d'une solution aqueuse de peroxyde d'hydrogène à 71 % (soit 6,5 mmol $H_2O_2$ pure).
Après 15 minutes, les résultats obtenus sont les suivants :

- $RT_{HYDROQUINONE/\ H2O2}$ = 53,8 %
- $RT_{PYROCATECHINE/\ H2O2}$ = 24,6 %
- Rapport $_{HQ/PC}$= 2,18

Exemple 59

Dans un réacteur métallique de 100 ml, on charge :

- 4,7 g (0,050 mol) de phénol
- 9,1 g (0,05 mol) de benzophénone
- 20 g (1,00 mol) d'acide fluorhydrique anhydre

On ferme le réacteur et l'on porte le mélange réactionnel à la température choisie de 80°C, tout en le maintenant sous agitation.
Au bout de 4 heures, le mélange réactionnel est envoyé dans 100 ml d'eau ; on extrait par le dichlorométhane. La phase organique obtenue est lavée à l'eau jusqu'à pH = 5. On sépare les phases aqueuse et organique.
On évapore le dichlorométhane sous pression réduite.
Dans cette masse (11,97 g) on dose :

- benzophénone = 5,82 g (32 mmol) [TT $_{BENZOPHENONE}$ = 36 %]
- diphénylifuchsone = 3,97 g (15,4 mmol) [RR $_{FUCHSONE}$ = 30,8 %]

On place cette masse réactionnelle dans un réacteur et l'on ajoute 25 mg d'acide perchlorique à 70% en poids. On porte le mélange réactionnel à 30-35°C et on y introduit 0,802 g d'une solution aqueuse de peroxyde d'hydrogène à 71 % (soit 16,7 mmol $H_2O_2$ pure).

La température réactionnelle s'élève à 65°C.
Après 15 minutes, les résultats obtenus sont les suivants :

- $RR_{HYDROQUINONE/FUCHSONE}$ = 77,5 %
- Rapport $_{HQ/PC}$= 27

Le taux de récupération de la benzophénone est de 98,5 %.

Exemple 60

On reproduit l'exemple 59 à la différence près que la masse réactionnelle est diluée 5 fois dans l'acétonitrile (rapport pondéral acétonitrile/masse réactionnelle = 5).
Après 15 minutes, les résultats obtenus sont les suivants :

- $RR_{HYDROQUINONE/FUCHSONE}$ = 82 %
- Rapport $_{HQ/PC}$= 63

Le taux de récupération de la benzophénone est de 92 %.

Exemple 61

Dans un ballon en verre de 100 ml muni d'une agitation centrale, d'un réfrigérant, d'une ampoule de coulée et d'un thermomètre, on charge :

- 24 g (0,255 mol) de phénol
- 3,6 g (0,0124 mol) d'acide rosolique
- 25 mg d'acide perchlorique à 70 % en poids.

On porte le mélange réactionnel à la température réactionnelle choisie 75°C, tout en le maintenant sous agitation à 1200 tours/mn.
On introduit par l'intermédiaire de l'ampoule de coulée, en 2 minutes, 0,0126 mol de peroxyde d'hydrogène sous la forme d'une solution aqueuse à 70,5 %.
On refroidit alors le mélange réactionnel et l'on effectue le dosage des produits de réaction.
Au bout de 1 heure 30 minutes de réaction, les résultats obtenus sont les suivants :

- $TT_{H2O2}$ = 100 %
- $RT_{HYDROQUINONE/H2O2}$ = 74,5 %
- $RT_{PYROCATECHINE/H2O2}$ = 13 %
- Rapport $_{HQ/PC}$ = 5,7

Exemple 62

Dans un ballon en verre tel que décrit dans l'exemple 1, on charge :

- 0,828 g (0,003 mol) de carbinol correspondant à la diphénylfuchsone
- 8,3 ml d'acétonitrile
- 145,7 mg (0,003 mol) dune solution aqueuse de peroxyde d'hydrogène à 70,5 %

A la température ambiante de 25°C, on ajoute 18 mg (0,12 mmol) d'acide perchlorique à 70 % en poids.
La température réactionnelle s'élève d'elle-même à 50°C.
Au bout de 15 minutes de réaction, les résultats obtenus sont les suivants :

- $TT_{H2O2}$ = 100 %
- $RR_{HYDROQUINONE/H2O2}$ = 100 %
- $RT_{HYDROQUINONE/H2O2}$ = 100 %

On récupère la benzophénone avec un taux de récupération de 95 %.

Exemple 63

Dans un ballon en verre de 100 ml, muni d'une agitation centrale, d'un réfrigérant, d'une ampoule de coulée et d'un thermomètre, on charge :

- 56,4 g (0,6 mol) de phénol
- 10,92 g (0,06 mol) de benzophénone
- 46,06 g (0,48 mol) d'acide méthanesulfonique

On porte ce mélange réactionnel à 110°C pendant 7 heures, tout en maintenant sous agitation de 1200 tours/mn.
On refroidit alors le mélange réactionnel, puis on ajoute 50 ml d'eau et 100 ml d'éther isopropylique. On décante la phase éthérée qui est lavée 3 fois par 50 ml d'eau.
Dans cette phase organique éthérée, on dose :

- FUCHSONE = 0,0347 mol
- BENZOPHENONE = 0,0240 mol
- PHENOL = 0,487 mol

Cette phase éthérée est placée dans le ballon précédent. On y ajoute 0,1 g d'acide perchlorique. On porte cette masse réactionnelle à 40°C et l'on y introduit en deux minutes, 0,035 mol de peroxyde d'hydrogène sous la forme d'une solution aqueuse à 70 %.
Après 1 heure à 45°C, tout en maintenant sous agitation de 1200 tours/mn, on refroidit la masse réactionnelle. On dose le peroxyde d'hydrogène résiduel ainsi que les diphénols formés :
Les résultats obtenus sont les suivants :

- $TT_{H2O2}$ = 100 %
- $TT_{FUCHSONE}$ = 100 %
- $RR_{HYDROQUINONE/H2O2}$ = 100 %

Il n'y a pas de pyrocatéchine.
On récupère la benzophénone avec un taux de récupération de 100 %.

Exemple 64

Dans un ballon de verre de 100 ml muni d'une agitation centrale, d'un réfrigérant, d'une ampoule de coulée et d'un thermomètre, on charge :

- 23,5 (0,25 mol) de phénol
- 0,015 g (0,001 mol) d'acide perchlorique à 70 % en poids
- 4,3 g (0,0617 mol) de diphénylfuchsone

On porte ce mélange à 75°C et l'on introduit, en deux minutes, par l'intermédiaire de l'ampoule de coulée, 0,017 mol de peroxyde d'hydrogène sous la forme d'une solution aqueuse à 70 %, tout en maintenant sous agitation de 1200 tours/mn.
Après 30 mn de réaction, la masse réactionnelle est refroidie ; on dose le peroxyde d'hydrogène résiduel par iodométrie et les diphénols formés par chromatographie liquide haute performance.
Les résultats obtenus sont les suivants :

- $TT_{H2O2}$ = 100 %
- $RT_{HYDROQUINONE/H2O2}$ = 93 %
- $RT_{PYROCATECHINE/H2O2}$ = 2 %

Exemple 65

Dans un appareillage tel que décrit dans l'exemple 64, on charge :

- 23,5 g (0,25 mol) de phénol

- 3,45 g (0,0125 mol) de carbinol correspondant à la diphénylfuchsone

On porte ce mélange à 65°C puis l'on coule, en deux minutes, par l'intermédiaire de l'ampoule de coulée, 0,607 g (0,0125 mol) de peroxyde d'hydrogène sous la forme d'une solution aqueuse à 70 % tout en maintenant l'agitation à 1200 tours/mn. On opère ainsi sans acide fort.
Après 1 heure, le mélange est refroidi et on dose le peroxyde d'hydrogène résiduel et les produits formés.
Les résultats obtenus sont les suivants :

- $TT_{H2O2}$ = 100 %
- $RT_{HYDROQUINONE/H2O2}$ = 45 %
- $RT_{PYROCATECHINE/H2O2}$ = 31,5 %
- $Rapport_{HQ/PC}$ = 1,43

Exemple 66

Dans un appareillage tel que décrit dans l'exemple 64, on charge :

- 23,5 g (0,25 mol) de phénol
- 3,2 g (0,0125 mol) de fuchsone
- 1,8 g (0,0125 mol) d'acide perchlorique à 70 % en poids

On porte ce mélange à 75°C et l'on coule, en deux minutes, 0,60 g (0,0125 mol) de peroxyde d'hydrogène sous la forme d'une solution aqueuse à 70 % poids, tout en agitant à 1200 tours/mn.
Après 15 minutes, la masse réactionnelle est refroidie et on dose les produits formés.
Les résultats obtenus sont les suivants :

- $TT_{H2O2}$ = 100 %
- $TT_{FUCHSONE}$ = 79 %
- $RT_{HYDROQUINONE/H2O2}$ = 80,5 %
- $RT_{PYROCATECHINE/H2O2}$ = 12 %
- $Rapport_{HQ/PC}$ = 6,7

EXEMPLES 67 A 83

Les exemples 67 à 83 illustrent la mise en oeuvre d'une fuchsone, comme catalyseur d'hydroxylation d'un composé phénolique.
Dans les exemples, les abréviations suivantes signifient :

$$TTH_2O_2 = \frac{\text{nombre de moles de peroxyde d'hydrogène transformées}}{\text{nombre de moles de peroxyde d'hydrogène introduites}}$$

$$RT_{HQ} = \frac{\text{nombre de moles d'hydroquinone formées}}{\text{nombre de moles de peroxyde d'hydrogène transformées}}\ \%$$

$$RT_{PC} = \frac{\text{nombre de moles de pyrocatéchine formées}}{\text{nombre de moles de peroxyde d'hydrogène transformées}}\ \%$$

On donne, ci-après, le protocole opératoire qui va être suivi dans les exemples 67 à 83.
Dans un ballon en verre de 100 ml muni d'une agitation centrale, d'un réfrigérant, d'une ampoule de coulée et d'un thermomètre, on charge :

- 47 g (0,50 mol) de phénol,
- x g d'une fuchsone de formule (III) et/ou (IV).

On introduit ensuite y g d'acide fort (acide perchlorique) et éventuellement z g de solvant aprotique.
Les différentes quantités (x, y et z) peuvent être déterminées à partir des indications données dans les tableaux récapitulatifs.
On porte le mélange réactionnel à la température réactionnelle choisie 75°C (sauf indication contraire), tout

en le maintenant sous agitation à 1200 tours/mn.

On introduit par l'intermédiaire de l'ampoule de coulée, en 2 minutes, la solution aqueuse de peroxyde d'hydrogène à 70,5 % en poids en une quantité également précisée dans les tableaux suivants.

On maintient le mélange réactionnel sous agitation, à 75°C, pendant la durée mentionnée dans les tableaux suivants.

On refroidit alors le mélange réactionnel et l'on effectue le dosage des produits de réaction : le peroxyde d'hydrogène résiduel est dosé par iodométrie et les diphénols formés sont dosés par chromatographie liquide, haute performance.

Exemples 67 à 70

Dans ces quatre exemples, on réalise l'hydroxylation du phénol par le peroxyde d'hydrogène, en présence d'acide perchlorique et en l'absence de solvant organique.

La fuchsone mise en oeuvre est la diphénylfuchsone répondant à la formule suivante :

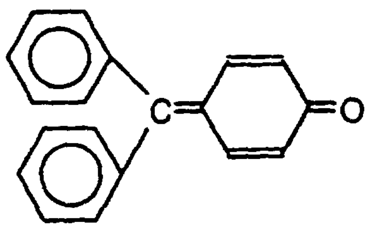

Les essais sont conduits selon le protocole opératoire précédemment défini.

Toutes les conditions et résultats obtenus sont rassemblés dans le tableau (III) suivant:

Tableau (III)

Hydroxylation du phénol par $H_2O_2$/Acide perchlorique/Diphénylfuchsone

| Réf. | Fuchsone de formule (III) [Rapport molaire fuchsone /$H_2O_2$] | Solvant organique [rapport molaire solvant organique/ phénol] | Rapport molaire $H_2O_2$/phénol | Rapport molaire $H^+/H_2O_2$ | Durée | $TT_{H_2O_2}$ | $RT_{HQ}$ | $RT_{PC}$ | Rapport HQ/PC |
|---|---|---|---|---|---|---|---|---|---|
| 67 | Diphénylfuchsone 0,240 | sans | $5,2.10^{-2}$ | $1,1.10^{-2}$ | 30 mn | 100 | 46,0 | 31,5 | 1,46 |
| 68 | Diphénylfuchsone 0,240 | sans | $5,2.10^{-2}$ | $1,1.10^{-2}$ | 30 mn | 100 | 46,0 | 31,5 | 1,46 |
| 69 | Diphénylfuchsone 0,199 | sans | $5,25.10^{-2}$ | $0,79.10^{-2}$ | 60 mn | 100 | 47,5 | 34,5 | 1,37 |
| 70 | Diphénylfuchsone 0,100 | sans | $5,01.10^{-2}$ | $0,84.10^{-2}$ | 60 mn | 100 | 42,5 | 40,0 | 1,06 |

Exemples 71 et 72

Dans cette série d'exemples, on conduit l'hydroxylation du phénol par le peroxyde d'hydrogène, en présence d'acide perchlorique et en l'absence de solvant organique.

La fuchsone mise en oeuvre est l'acide rosolique répondant à la formule suivante :

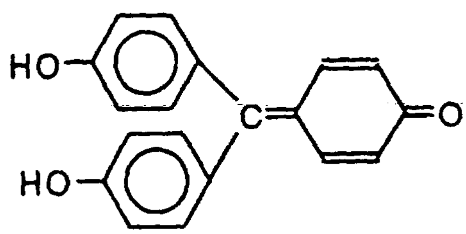

Les essais sont conduits selon le protocole opératoire précédemment défini.

Toutes les conditions et résultats obtenus sont rassemblés dans le tableau (IV) suivant:

Tableau (IV)

Hydroxylation du phénol par $H_2O_2$/Acide perchlorique/Acide rosolique

| Réf. | Fuchsone de formule(III) [Rapport molaire Fuchsone/$H_2O_2$] | Solvant organique [rapport molaire solvant organique/ phénol] | Rapport molaire $H_2O_2$/phénol | Rapport molaire $H^+/H_2O_2$ | Durée | $TT_{H_2O_2}$ | $RT_{HQ}$ | $RT_{PC}$ | Rapport HQ/PC |
|---|---|---|---|---|---|---|---|---|---|
| 71 | Acide rosolique (0,25) | sans | 5,0 | 0,47 | 180 mn | 99,5 | 40,0 | 31,5 | 1,27 |
| 72 | Acide rosolique (0,25) | sans | 5,0 | 1,15 | 30 mn | 100 | 38,0 | 31,0 | 1,22 |

Exemple 73

Dans cet exemple, on réalise l'hydroxylation du phénol par le peroxyde d'hydrogène, en présence d'acide perchlorique et d'un solvant organique polaire et peu basique tel que l'acétonitrile.
La fuchsone mise en oeuvre est la diphénylfuchsone définie dans l'exemple 67.
L'essai est conduit selon le protocole opératoire précédemment défini.
Toutes les conditions et résultats obtenus sont rassemblés dans le tableau (V) suivant :

Tableau (V)

Hydroxylation du phénol par $H_2O_2$/Acide perchlorique/Diphénylfuchsone

| Réf. | Fuchsone de formule(III) [Rapport molaire fuchsone/$H_2O_2$] | Solvant organique [rapport molaire solvant organique/ phénol] | Rapport molaire $H_2O_2$/phénol | Rapport molaire $H^+/H_2O_2$ | Durée | $TT_{H_2O_2}$ | $RT_{HQ}$ | $RT_{PC}$ | Rapport HQ/PC |
|---|---|---|---|---|---|---|---|---|---|
| 73 | Diphénylfuchsone (0,25) | Acétonitrile (0,50) | $5,1.10^{-2}$ | $1,3.10^{-2}$ | 180 mn | 98,5 | 56,0 | 28,5 | 2,0 |

Exemple 74

Dans cet exemple, on conduit l'hydroxylation du phénol par le peroxyde d'hydrogène, en présence d'acide perchlorique et d'un solvant organique peu polaire et basique tel que l'oxyde de diisopropyle.
La fuchsone mise en oeuvre est la diphénylfuchsone définie dans l'exemple 67.
L'essai est conduit selon le protocole opératoire précédemment défini.
Toutes les conditions et résultats obtenus sont rassemblés dans le tableau (VI) suivant :

Tableau (VI)

Hydroxylation du phénol par $H_2O_2$/Acide perchlorique/Diphénylfuchsone

| Réf. | Fuchsone de formule (III) [Rapport molaire Fuchsone/$H_2O_2$] | Solvant organique [rapport molaire solvant organique/ phénol] | Rapport molaire $H_2O_2$/phénol | Rapport molaire $H^+/H_2O_2$ | Durée | $TT_{H_2O_2}$ | $RT_{HQ}$ | $RT_{PC}$ | Rapport HQ/PC |
|---|---|---|---|---|---|---|---|---|---|
| 74 | Diphénylfuchsone (0,25) | Oxyde de diisopropyle (0,51) | $5,0.10^{-2}$ | $1,25.10^{-2}$ | 180 mn | 99,5 | 39,0 | 25,0 | 1,6 |

Exemple 75

Dans cet exemple, on opère comme dans les exemples 67 à 70 mais en remplaçant la diphénylfuchsone par le carbinol correspondant.

Toutes les conditions et résultats obtenus sont rassemblés dans le tableau (VII) suivant :

Tableau (VII)

Hydroxylation du phénol par $H_2O_2$/Acide perchlorique/Carbinol de diphénylfuchsone

| Réf. | Carbinol de formule (IV) [Rapport molaire Carbinol/$H_2O_2$] | Solvant organique [rapport molaire solvant organique/phénol] | Rapport molaire $H_2O_2$/phénol | Rapport molaire $H^+/H_2O_2$ | Durée | $TT_{H_2O_2}$ | $RT_{HQ}$ | $RT_{PC}$ | Rapport HQ/PC |
|---|---|---|---|---|---|---|---|---|---|
| 75 | Carbinol de diphénylfuchsone (0,245) | sans | $5,2.10^{-2}$ | $0,37.10^{-2}$ | 100 mn | 99,0 | 47,0 | 30,0 | 1,57 |

Exemples 76 à 82

Dans un ballon de verre de 100 ml muni d'une agitation centrale, d'un réfrigérant, d'une ampoule de coulée et d'un thermomètre, on charge 47 g (0,5 mol) de phénol.

On introduit ensuite de l'acide perchlorique et de la diphénylfuchsone suivant les quantités indiquées dans le tableau (VIII).

On porte ce mélange à 75°C et l'on ajoute en deux minutes, par l'intermédiaire de l'ampoule de coulée, une solution aqueuse de peroxyde d'hydrogène à 70 % suivant les quantités indiquées dans le tableau (VIII) et tout en maintenant sous agitation de 1200 tours/mn.

En fin de réaction, on refroidit le mélange réactionnel et l'on effectue le dosage des produits de réaction.

Les résultats obtenus sont les suivants :

Tableau (VIII)

Hydroxylation du phénol par $H_2O_2$/Acide perchlorique/Diphénylfuchsone

| Réf. | Fuchsone de formule (III) [Rapport molaire Fuchsone/$H_2O_2$] | Rapport molaire $H^+/H_2O_2$ | Rapport molaire $H_2O_2$/phénol | Durée | $TTH_2O_2$ | $RT_{HQ}$ | $RT_{PC}$ | Rapport HQ/PC |
|---|---|---|---|---|---|---|---|---|
| 76 | Diphénylfuchsone ($4,65.10^{-2}$) | $0,95.10^{-2}$ | $5,35.10^{-2}$ | 60 mn | 99 | 39,5 | 41,5 | 0,,95 |
| 77 | Diphénylfuchsone (0,103) | $0,85.10^{-2}$ | $5,0.10^{-2}$ | 60 mn | 100 | 42,5 | 40 | 1,06 |
| 78 | Diphénylfuchsone (0,145) | $0,95.10^{-2}$ | $5,2.10^{-2}$ | 60 mn | 100 | 44,5 | 36 | 1,25 |
| 79 | Diphénylfuchsone (0,199) | $0,80.10^{-2}$ | $5,25.10^{-2}$ | 60 mn | 100 | 48 | 34,5 | 1,38 |
| 80 | Diphénylfuchsone (0,33) | $0,75.10^{-2}$ | $5,3.10^{-2}$ | 30 mn | 100 | 54 | 29 | 1,85 |
| 81 | Diphénylfuchsone (0,57) | $0,89.10^{-2}$ | $4,65.10^{-2}$ | 30 mn | 100 | 68,5 | 18 | 3,85 |
| 82 | Diphénylfuchsone (0,77) | $0,87.10^{-2}$ | $4,7.10^{-2}$ | 30 mn | 100 | 81 | 9 | 9,1 |

Exemple 83

Dans un appareillage tel que décrit précédemment, on charge :

. 47 g (0,5 mol) de phénol
. 0,61 g (0,00237 mol) de diphénylfuchsone
. 0,23 g (0,00237 mol) d'acide sulfurique à 98 %

On porte le mélange à 75°C et, tout en agitant à 1200 tours/mn, on coule, en deux minutes, 1,21 g (0,0125 mol) d'une solution aqueuse de peroxyde d'hydrogène à 70 %.
Après 25 mn, on refroidit le mélange et on dose les produits formés.
Les résultats obtenus sont les suivants :

- $TT_{H2O2}$ = 100 %
- $RR_{HYDROQUINONE/H2O2}$ = 35,5 %
- $RR_{PYROCATEGHINE/H2O2}$ = 43 %
- rapport $_{HQ/PC}$ = 0,82

## Revendications

1. Procédé de préparation d'un composé aromatique para-dihydroxylé **caractérisé par le fait qu'**il consiste à faire réagir un agent oxydant avec une p-fuchsone.

2. Procédé selon la revendication 1 **caractérisé par le fait qu'**il consiste à faire réagir un agent oxydant avec une fuchsone répondant à la formule générale (III) et/ou (IV) :

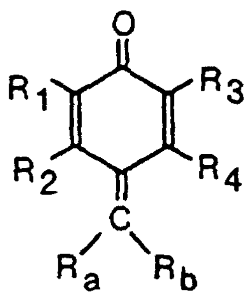
(III)

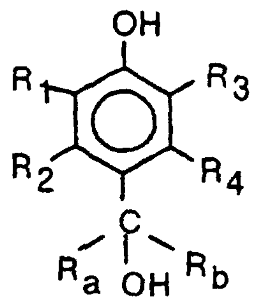
(IV)

dans ladites formules (III) et (IV),

- $R_a$ et $R_b$, identiques ou différents sont des radicaux hydrocarbonés ayant chacun de 3 à 30 atomes de carbone; les atomes de carbone de chaque radical $R_a$ et $R_b$ en position a par rapport à l'atome de carbone qui les porte, étant des carbones tertiaires,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle, de préférence un carbocycle insaturé ou aromatique ayant de 4 à 7 atomes de carbone.

3. Procédé selon la revendication 2 **caractérisé par le fait que** la fuchsone est une fuchsone répondant à la formule générale (III) et/ou (IV) dans laquelle :

- $R_1$, $R_2$, $R_3$ et $R_4$, identiques ou différents représentent $R_0$, l'un des groupes suivants :

  . un atome d'hydrogène,
  . un radical alkyle linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes

de carbone, tel que vinyle, allyle,

- un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
- un groupe acyle ayant de 2 à 6 atomes de carbone,
- un radical de formule :

$$-R_5-OH$$

$$-R_5-COOR_6$$

$$-R_5-X$$

$$-R_5-CF_3$$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

  - un radical carbocyclique saturé ou insaturé ayant de 4 à 7 atomes de carbone, de préférence un radical cyclohexyle,
  - un radical de formule

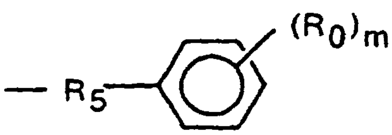

dans lequel $R_5$ représente un lien valentiel ou un radical hydrocarboné divalent, linéaire ou ramifié, saturé ou insaturé ayant de à 6 atomes de carbone tel que par exemple, méthylène, éthylène, propylène, isopropylène, isopropylidène et $R_0$ ayant la signification donnée précédemment et m est un nombre entier de 0 à 4,

  - un radical - $R_5$ - A - $R_8$ dans lequel $R_5$ a la signification donnée précédemment, $R_8$ représente un radical alkyle linéaire ou ramifié ayant de 1 à 6 atomes de carbone ou un radical de formule

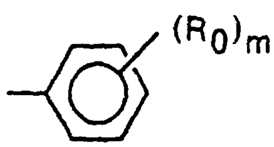

et A symbolise l'un des groupes suivants :
-O-, -COO-, -OCOO-, $-SO_2-$,

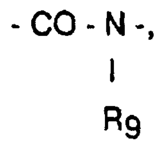

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone.

**4.** Procédé selon l'une des revendications 2 et 3 **caractérisé par le fait que** la fuchsone est une fuchsone répondant à la formule générale (III) et/ou (IV) dans laquelle :

- $R_1$, $R_2$, $R_3$ et $R_4$, identiques ou différents représentent l'un des groupes suivants :

  . un atome d'hydrogène,
  . un radical alkyle, linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- deux groupes $R_1$ et $R_2$ et/ou $R_3$ et $R_4$ placés sur deux atomes de carbone vicinaux peuvent former ensemble et avec les atomes de carbone qui les portent un cycle benzénique.

**5.** Procédé selon l'une des revendications 2 à 4 **caractérisé par le fait que** la fuchsone est une fuchsone répondant à la formule générale (III) et/ou (IV) dans laquelle l'un des radicaux $R_1$, $R_2$, $R_3$ et $R_4$ représente un groupe hydroxyle, un radical méthyle ou un radical méthoxy et les 3 autres représentent un atome d'hydrogène.

**6.** Procédé selon l'une des revendications 1 à 5 **caractérisé par le fait que** la fuchsone est une fuchsone répondant à la formule générale (III) et/ou (I'V) dans laquelle $R_a$ et $R_b$ représentent un radical alkyle ramifié ayant au moins 3 atomes de carbone ou un radical aryle ayant au moins 6 atomes de carbone, de préférence un radical tert-butyle, tert-pentyle, tert-hexyle ou phényle éventuellement substitué.

**7.** Procédé selon l'une des revendications 1 à 6 **caractérisé par le fait que** la fuchsone est une fuchsone répondant à la formule générale (IIIa) et/ou (IVa) suivante :

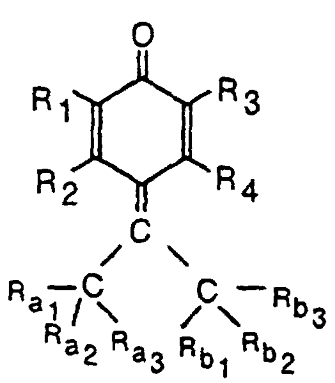
(IIIa)

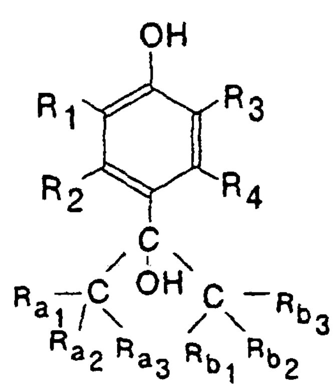
(IVa)

dans lesdites formules (IIIa) et (IVa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ d'une part et $R_{b1}$, $R_{b2}$, $R_{b3}$, d'autre part peuvent former ensemble et l'atome de carbone qui les porte, un cycle benzénique ou naphtalénique éventuellement substitué,
- $R_1$, $R_2$, $R_3$ et $R_4$, identiques ou différents représentent l'un des groupes suivants :

  . un atome d'hydrogène,
  . un radical alkyle linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- les deux groupes $R_1$ et $R_2$ et/ou $R_3$ et $R_4$ placés sur deux atomes de carbone vicinaux peuvent former ensemble et avec les atomes de carbone qui les portent un cycle benzénique.

**8.** Procédé selon l'une des revendications 1 à 7 **caractérisé par le fait que** la fuchsone est une fuchsone répondant à la formule générale (IIIa) et/ou (IVa) dans laquelle l'un des radicaux $R_1$, $R_2$, $R_3$ et $R_4$ représente un groupe hydroxyle, un radical méthyle, un radical méthoxy et les 3 autres représentent un atome d'hydrogène.

**9.** Procédé selon l'une des revendications 1 à 8 **caractérisé par le fait que** la fuchsone est une fuchsone répondant à la formule (IIIb) et/ou (IVb) suivante :

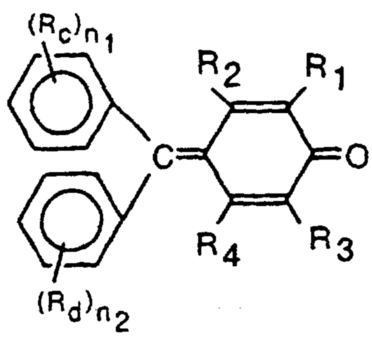

(IIIb)

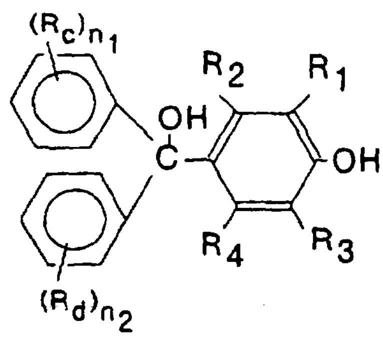

(IVb)

dans lesdites formules (IIIb) et (IVb) :

- $R_1$, $R_2$, $R_3$ et $R_4$, identiques ou différents représentent l'un des groupes suivants :

  . un atome d'hydrogène,
  . un radical alkyle linéaire ou ramifié, ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- deux groupes $R_1$ et $R_2$ et/ou $R_3$ et $R_4$ placés sur deux atomes de carbone vicinaux peuvent former ensemble et avec les atomes de carbone qui les portent un cycle benzénique.
- $R_c$ et $R_d$ identiques ou différents représentent un atome d'hydrogène ou un substituant,
- $n_1$, $n_2$ identiques ou différents est un nombre égal à 0, 1, 2 ou 3.

**10.** Procédé selon l'une des revendications 1 à 9 **caractérisé par le fait que** la fuchsone est une fuchsone répondant à la formule (IIIb) et/ou (IVb) dans laquelle $R_c$ et $R_d$, identiques ou différents représentent :

- des radicaux alkyle linéaires ou ramifiés ayant de 1 à 4 atomes de carbone,
- un radical phényle,
- des radicaux alkoxy $R_{10}$ - O dans lesquels $R_{10}$ représente un radical alkyle linéaire ou ramifié ayant de 1 à 4 atomes de carbone ou le radical phényle,
- un atomes d'halogène, de préférence de chlore, de brome ou de fluor.

**11.** Procédé selon l'une des revendications 1 à 10 **caractérisé par le fait que** la fuchsone est une fuchsone répondant à la formule (IIIb) et/ou (IVb) dans laquelle $R_c$ et $R_d$, identiques ou différents représentent un atome d'hydrogène ou un substituant tel que défini dans la revendication 38, de préférence en position 4,4' et $n_1$, $n_2$ identiques ou différents sont égaux à 0 ou 1.

**12.** Procédé selon l'une des revendications 1 à 11 **caractérisé par le fait que** la fuchsone est une fuchsone répondant à la formule (IIIb) et/ou (IVb) dans laquelle $R_c$ et $R_d$, identiques ou différents représentent un atome d'hydrogène; un radical méthyle, éthyle, tert-butyle, phényle ; un radical méthoxy ou éthoxy ; un groupe hydroxyle, de préférence en position 3,3' ou 4,4'.

**13.** Procédé selon l'une des revendications 1 à 12 **caractérisé par le fait que** l'agent oxydant est choisi parmi le peroxyde d'hydrogène ; les peracides de préférence, l'acide peracétique ; les hydroperoxydes de préférence l'hydroperoxyde de tertiobutyle, l'hydroperoxyde de cyclohexyle, l'hydroperoxyde de cumyle.

**14.** Procédé selon la revendication 13 **caractérisé par le fait que** l'agent oxydant est une solution aqueuse de peroxyde d'hydrogène.

**15.** Procédé selon l'une des revendications 1 à 14 **caractérisé par le fait que** la quantité de peroxyde d'hydrogène est de préférence égale à la quantité stoechiométrique soit 1 mole de $H_2O_2$ pour 1 mole de fuchsone de formule (III) et/ou (IV).

**16.** Procédé selon l'une des revendications 1 à 15 **caractérisé par le fait que** l'on fait réagir la fuchsone et l'agent oxydant, en présence d'un catalyseur acide qui est un acide protonique fort présentant un pKa dans l'eau inférieur à - 0,1 et, de préférence, inférieur à - 1,0.

**17.** Procédé selon l'une des revendications 1 à 16 **caractérisé par le fait que** l'acide fort est un oxyacide halogéné ou non, de préférence, l'acide sulfurique, l'acide pyrosulfurique, l'acide perchlorique, les acides halogénosulfoniques, de préférence, l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques ; et encore plus préférentiellement l'acide perchlorique, l'acide trifluorométhanesulfonique ou l'acide méthanesulfonique.

**18.** Procédé selon l'une des revendications 1 à 17 **caractérisé par le fait que** la quantité d'acide exprimée par le rapport du nombre d'équivalents de protons au nombre de moles de peroxyde d'hydrogène varie entre environ $1.10^{-4}$ et environ 1,0, de préférence, entre $1.10^{-3}$ et 0,1.

**19.** Procédé selon l'une des revendications 1 à 18 **caractérisé par le fait que** la réaction est conduite dans un solvant réactionnel qui est un composé phénolique de formule (I) et/ou un solvant organique.

**20.** Procédé selon la revendication 19 **caractérisé par le fait que** le solvant organique est choisi parmi :

- les nitriles alphatiques ou aromatiques, de préférence, l'acétonitrile, le propionitrile, le butanenitrile, l'isobutanenitrile, le benzonitrile, le cyanure de benzyle,
- les éther-oxydes aliphatiques, cycloaliphatiques ou aromatiques et, de préférence, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne,
- les hydrocarbures aliphatiques chlorés, de préférence, le dichlorométhane, le tétrachlorométhane, le dichloroéthane,
- les alcools, de préférence, le méthanol, l'éthanol, l'isopropanol, le tertiobutanol.

**21.** Procédé selon l'une des revendications 1 à 20 **caractérisé par le fait que** la température de la réaction est comprise entre 20°C et 150°C, de préférence entre 30°C et 80°C.

**22.** Procédé selon l'une des revendications 1 à 21 **caractérisé par le fait que** l'on oxyde, à l'aide d'une solution de peroxyde d'hydrogène, la diphénylfuchsone ou l'acide rosolique, la diphénylméthylfuchsone en vue d'obtenir respectivement l'hydroquinone ou la méthylhydroquinone.

**23.** Procédé selon l'une des revendications 1 à 22 **caractérisé par le fait que** la fuchsone mise en oeuvre est une fuchsone obtenue selon un procédé qui consiste à faire réagir un composé phénolique présentant au moins un atome d'hydrogène en position para et un composé cétonique non énolisable, en présence d'une quantité efficace d'un acide protonique présentant une fonction d'acidité $-H_o$ d'au moins 5 et éventuellement en présence d'une quantité efficace d'un composé soufré ionisable.

**24.** Procédé selon la revendication 23 **caractérisé par le fait que** le composé phénolique présentant au moins un atome d'hydrogène en position para est un composé phénolique répondant à la formule générale (I) :

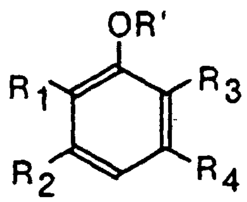
(I)

dans ladite formule (I) :

- la position en para est libre,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle,
- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

**25.** Procédé selon l'une des revendications 23 et 24 **caractérisé par le fait que** le composé phénolique répond à la formule générale (I) dans laquelle :

- le radical R' représente l'un des groupes suivants :
  . un atome d'hydrogène
  . un radical alkyle linéaire ou ramifié ayant de 1 à 6 atomes de carbone, de préférence de 1 à 4 atomes de carbone,
  . un radical cyclohexyle,
  . un radical phényle,
  . un radical benzyle,

- $R_1$, $R_2$, $R_3$ et $R_4$, identiques ou différents représentent $R_0$, l'un des groupes suivants :
  . un atome d'hydrogène,
  . un radical alkyle linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,

. un radical de formule :

$$-R_5\text{-OH}$$

$$-R_5\text{-COOR}_6$$

$$-R_5\text{-X}$$

$$-R_5\text{-CF}_3$$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

  . un radical carbocyclique saturé ou insaturé ayant de 4 à 7 atomes de carbone, de préférence un radical cyclohexyle,

  . un radical de formule

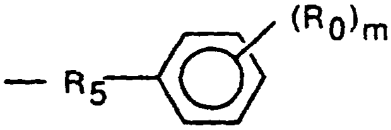

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

  . un radical - $R_5$ - A - $R_8$ dans lequel $R_5$ a la signification donnée précédemment, $R_8$ représente un radical alkyle linéaire ou ramifié ayant de 1 à 6 atomes de carbone ou un radical de formule

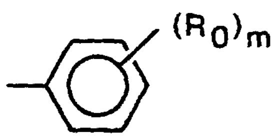

et A symbolise l'un des groupes suivants :
-O-, -COO-, -OCOO-, $-SO_2-$,

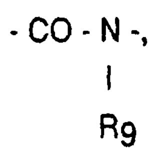

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_3$ et $R_4$ placés sur deux atomes de carbone vicinaux peuvent former ensemble

et avec les atomes de carbone qui les portent un carbocycle insaturé ou aromatique ayant de 4 à 7 atomes de carbone et, de préférence, 6 atomes de carbone.

**26.** Procédé selon l'une des revendications 23 à 25 **caractérisé par le fait que** le composé phénolique répond à la formule générale (I) dans laquelle :

- R' représente un atome d'hydrogène
- $R_1$, $R_2$, $R_3$ et $R_4$, identiques ou différents représentent l'un des groupes suivants :
  - . un atome d'hydrogène,
  - . un radical alkyle, linéaire ou ramifié ayant de 1 à 4 atomes de carbone,.
  - . un radical alkoxy linéaire ou ramifié ayant de 1 à 4 atomes de carbone,
  - . un groupe hydroxyle,
  - . un atome d'halogène,
  - . un groupe $-CF_3$
  - . un radical cyclohexyle,
  - . un radical phényle,
- deux groupes $R_1$ et $R_2$ et/ou $R_3$ et $R_4$ placés sur deux atomes de carbone vicinaux peuvent former ensemble et avec les atomes de carbone qui les portent un cycle benzénique.

**27.** Procédé selon l'une des revendications 23 à 26 **caractérisé par le fait que** le composé phénolique répond à la formule générale (I) laquelle R' représente un atome d'hydrogène et l'un des radicaux $R_1$, $R_2$, $R_3$ et $R_4$ représente un groupe hydroxyle, un radical méthyle ou un radical méthoxy et les 3 autres représentent un atome d'hydrogène.

**28.** Procédé selon l'une des revendications 23 à 27 **caractérisé par le fait que** le composé phénolique est le phénol, l'anisole, l'orthocrésol, le métacrésol, le 2-méthoxyphénol, le 2-éthylphénol, le 3-éthylphénol, 2-propylphénol, le 2-sec-butylphénol, le 2-tert-butylphénol, le 3-tert-butylphénol, le 2-méthoxyphénol, le 3-méthoxyphénol, le salicylate de méthyle, le 2-chlorophénol, le 3-chloro-phénol ; le 2,3-diméthylphénol, le 2,5-diméthylphénol, le 2,6-diméthylphénol, le 3,5-diméthyphénol, le 2,3-dichlorophénol, le 2,5-dichlorophénol, le 2,6-dichlorophénol, le 3,5-dichlorophénol, le 2,6-ditert-butylphénol, le 3,5-ditert-butylphénol ; le 2,3,5-triméthylphénol, le 2,3,6-triméthylphénol, le 2,3,5-trichlorophénol, le 2,3,6-trichlorophénol ; le 1-hydroxynaphtalène ; le 2-phénoxyphénol, le 3-phénoxyphénol.

**29.** Procédé selon l'une des revendications 23 à 28 **caractérisé par le fait que** le composé cétonique non énolisable est un composé cétonique répondant à la formule générale (II) :

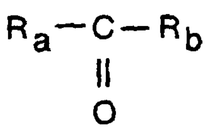

(II)

dans ladite formule (II) :

- $R_a$ et $R_b$, identiques ou différents sont des radicaux hydrocarbonés ayant chacun de 3 à 30 atomes de carbone; les atomes de carbone de chaque radical $R_a$ et $R_b$ en position $\alpha$ par rapport au groupe carbonyle étant des carbones tertiaires.

**30.** Procédé selon l'une des revendications 23 à 29 **caractérisé par le fait que** le composé cétonique répond à la formule générale (IIa) :

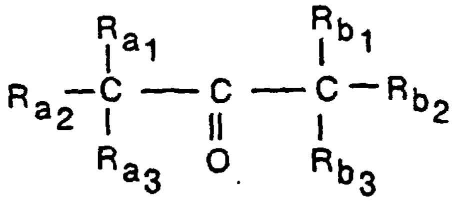

(IIa)

dans ladite formule (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ et/ou $R_{b1}$, $R_{b2}$, $R_{b3}$ peuvent former ensemble et avec l'atome de carbone qui les porte un cycle benzénique ou naphtalénique éventuellement substitué.

**31.** Procédé selon l'une des revendications 23 à 30 **caractérisé par le fait que** le composé cétonique répond à la formule générale (IIb) :

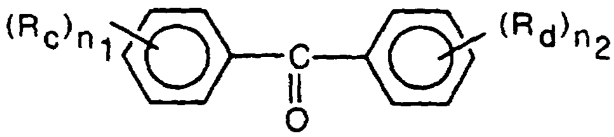

(IIb)

dans ladite formule (IIb) :

- $R_c$ et $R_d$ identiques ou différents représentent un atome d'hydrogène ou un substituant,
- $n_1$, $n_2$ identiques ou différents est un nombre égal à 0, 1, 2 ou 3.

**32.** Procédé selon l'une des revendications 23 à 31 **caractérisé par le fait que** le composé cétonique répond à la formule générale (IIb) dans laquelle $R_c$ et $R_d$, identiques ou différents représentent :

- des radicaux alkyle linéaires ou ramifiés ayant de 1 à 4 atomes de carbone,
- un radical phényle,
- des radicaux alkoxy $R_{10}$ - O dans lesquels $R_{10}$ représente un radical alkyle linéaire ou ramifié ayant de 1 à 4 atomes de carbone ou le radical phényle,
- un groupe hydroxyle,
- un atome de fluor.

**33.** Procédé selon l'une des revendications 23 à 32 **caractérisé par le fait que** le composé cétonique répond à la formule générale (IIb) dans laquelle $R_c$ et $R_d$, identiques ou différents représentent un atome d'hydrogène ou un substituant tel que défini dans la revendication 32, de préférence en position 4,4' et $n_1$, $n_2$ identiques ou différents sont égaux à 0 ou 1.

**34.** Procédé selon l'une des revendications 23 à 33 **caractérisé par le fait que** le composé cétonique répond à la formule générale (IIb) dans laquelle $R_c$ et $R_d$, identiques ou différents représentent un atome d'hydrogène ; un radical méthyle, éthyle, tert-butyle, phényle ; un radical méthoxy ou éthoxy; un groupe hydroxyle, de préférence en position 3,3' ou 4,4'.

**35.** Procédé selon l'une des revendications 23 à 34 **caractérisé par le fait que** le composé cétonique de formule générale (IIb) est :

- la benzophénone
- la méthyl-2 benzophénone
- la diméthyl-2,4 benzophénone
- la diméthyl-4,4' benzophénone

- la diméthyl-2,2' benzophénone
- la diméthoxy-4,4' benzophénone
- l'hydroxy-4 benzophénone
- la dihydroxy-4-4' benzophénone
- le benzoyl-4 biphényle

**36.** Procédé selon l'une des revendications 23 à 35 **caractérisé par le fait que** le catalyseur acide présente une fonction d'acidité - Ho comprise entre 5 et 20, de préférence entre 10 et 15.

**37.** Procédé selon la revendication 36 **caractérisé par le fait que** le catalyseur acide est un acide protonique fort, de préférence l'acide fluorhydrique, l'acide perchlorique, l'acide trifluorométhanesulfonique, l'acide paratoluènesulfonique, l'acide chlorosulfonique, l'acide fluorosulfonique, l'acide méthanesulfonique, l'acide benzène sulfonique et encore plus préférentiellement, l'acide fluorhydrique, l'acide perchlorique, l'acide trifluorométhanesulfonique ou l'acide méthanesulfonique.

**38.** Procédé selon la revendication 36 **caractérisé par le fait que** le catalyseur acide est une résine sulfonique, de préférence, un copolymère styrène-divinylbenzène sulfoné, un copolymère phénol-formaldéhyde sulfoné, un copolymère tétrafluoroéthylène-perfluoro[2-(fluorosulfonyléthoxy)-propyl] vinyl éther et, de préférence, les résines suivantes : TEMEX 50, AMBERLYST 15, AMBERLYST 35, AMBERLYST 36, DOWEX 50 W ; DUOLITE ARC 9359 : NAFION.

**39.** Procédé selon la revendication 36 **caractérisé par le fait que** le catalyseur acide est choisi parmi : la silice-alumine, la silice-$Ga_2O_3$, la silice-$B_2O_3$ ; les argiles acides ; les argiles pontées les zéolithes naturelles ou synthétiques ; les phosphates de bore, d'aluminium ou de gallium ; les phosphates lamellaires de métaux tétravalents répondant à la formule $\alpha$-$M(HPO_4)_2$, $pH_2O$ dans laquelle M représente un métal tétravalent choisi parmi le titane, le germanium, le zirconium ou l'étain et p est un nombre inférieur à 2; les phosphonates lamellaires de métaux tétravalents, de préférence les phosphonates lamellaires de zirconium ; les oxydes rendus acides par un traitement,de préférence les oxydes sulfatés de titane, de zirconium ou de fer.

**40.** Procédé selon la revendication 36 **caractérisé par le fait que** le catalyseur acide utilisé est choisi parmi :

- les argiles naturelles présentant une structure dite "TOT" ou tétraèdre-octaèdre-tétraèdre, de préférence de la classe des smectites, et encore plus préférentiellement les montmorillonites.
- les argiles commerciales, déjà acides ou non, ou traitée par une solution aqueuse d'un acide.
- les argiles pontées préparées à partir de smectites, de préférence à partir de beidellites, en particulier de beidellites synthètiques ou de montmorillonites ; le pontage de l'argile étant de préférence un pontage à l'aluminium.

**41.** Procédé selon la revendication 36 **caractérisé par le fait que** le catalyseur acide est choisi parmi :

- les zéolithes naturelles telles que la chabazite, la clinoptilolite, l'érionite, la mordénite, la phillipsite, l'offrétite,
- les zéolithes synthétiques telles que la zéolithe ZSM-5, la zéolithe Y, la ferrierite la zéolithe X de type faujasite, la zéolithe de type L, la mordénite, la zéolithe ZSM-11; la mazzite, l'offrétite, de préférence une zéolithe US-Y ou une zéolithe ZSM-5.

**42.** Procédé selon la revendication 41 **caractérisé par le fait que** la zéolithe mise en oeuvre est une zéolithe acidifiée.

**43.** Procédé selon l'une des revendications 23 à 42 **caractérisé par le fait que** le composé soufré ionisable est un composé minéral soufré ou un composé organique soufré, de préférence choisi parmi :

- les halogénures de soufre,
- les thiosulfates d'ammonium, de métal alcalin ou alcalino-terreux,
- l'hydrogène sulfuré ou ses précurseurs,
- les mercaptans de préférence les alkylmercarptans, phénylmercaptans et phénylalkylmercaptans,
- les thioalcools,
- les thiophénols,
- les acides thioorganiques, leurs sels ou leurs esters,
- les résines échangeuses de cations modifiées par réaction avec une alkyl mercaptoamine, de préférence les

résines à squelette polystyrénique porteuses de groupes sulfoniques ayant réagi avec une alkyl mercaptoamine, de préférence celle avec un groupe amine primaire et encore plus préférentiellement celle ayant de 1 à 4 atomes de carbone.

**44.** Procédé selon la revendication 43 **caractérisé par le fait que** le composé soufré est choisi parmi :

- le monochlorure de soufre ou le bichlorure de soufre,
- le thiosulfate d'ammonium, de sodium ou de potassium,
- l'hydrogène sulfuré ou le sulfure de calcium ou de potassium,
- l'éthylmercaptan, le butylmercaptan, le 1-nonylmercaptan, le benzylmercaptan,
- le 2-mercaptoéthanol, le 3-mercapto-2-butanol,
- le thiophénol, l'o-thiocrésol, le m-thiocrésol, le p-thiocrésol, le thioxylénol, le p-méthylthiophénol, l'o-éthylthiophénol, le thiohydroquinone, le thionaphtol,
- l'acide thioacétique, l'acide thiopropionique, l'acide thiolactique, l'acide thiosalicylique, l'acide 2-mercaptoéthanesulfonique, l'acide 3-mercaptopropanesulfonique, l'acide mercaptosuccinique, l'acide thioglycolique, le thioglycolate de méthyle, d'éthyle,
- les résines à squelette polystyrénique porteuses de groupes sulfoniques ayant réagi avec la 2-mercaptoéthylamine, la 2-mercaptoisopropylamine, la 3-mercaptobutylamine.

**45.** Procédé selon l'une des revendications 23 à 44 **caractérisé par le fait que** le rapport molaire entre le composé phénolique de formule (I) et le composé cétonique de formule (II) est compris entre 1 et 20, de préférence entre 1 et 10.

**46.** Procédé selon l'une des revendications 23, 36 à 38 **caractérisé par le fait que** la quantité de catalyseur acide exprimée par le rapport d'équivalents de protons au nombre de moles de composé cétonique de formule (II) varie entre $1.10^{-3}$ et 1,0, de préférence entre $5.10^{-3}$ et 0,5.

**47.** Procédé selon la revendication 36 ou 37 **caractérisé par le fait que** ledit rapport est compris entre 1,0 et 20, de préférence entre 5,0 et 10 lorsque l'acide est utilisé comme milieu réactionnel.

**48.** Procédé selon l'une des revendications 39 à 42 **caractérisé par le fait que** la quantité de catalyseur acide mis en oeuvre représente en poids par rapport au composé phénolique de formule (I) engagé, de 0,1 à 20 %, de préférence de 0,5 à 10 %.

**49.** Procédé selon l'une des revendications 23 à 48 **caractérisé par le fait que** la quantité de composé soufré mis en oeuvre exprimée par rapport au composé cétonique de formule (II) est telle que le rapport molaire entre le composé soufré et le composé cétonique de formule (II) soit compris entre 0,001 et 1,0, de préférence entre 0,005 et 0,20.

**50.** Procédé selon l'une des revendications 23 à 49 **caractérisé par le fait que** la température de la réaction est comprise entre 45°C et 200°C, de préférence entre 60°C et 150°C.

**51.** Procédé selon l'une des revendications 23 à 50 **caractérisé par le fait que** l'on prépare la diphénylfuchsone par réaction du phénol et de la benzophénone ; l'acide rosolique par réaction du phénol et de la dihydroxy-4,4' benzophénone ; la diphénylméthylfuchsone par réaction de l'ortho-crésol et de la benzophénone.

**52.** Procédé selon la revendication 23 **caractérisé par le fait que** le composé aromatique p-dihydroxylé est obtenu en préparant une fuchsone selon le procédé décrit dans l'une des revendications 23 à 51 et puis en l'oxydant par addition de l'agent oxydant dans le milieu réactionnel, sans que la fuchsone soit isolée.

**53.** Procédé de préparation d'un composé aromatique p-dihydroxylé, par oxydation d'un composé phénolique présentant un atome d'hydrogène en position para avec le peroxyde d'hydrogène, en présence d'une quantité efficace d'un acide protonique fort présentant un pKa dans l'eau inférieur à - 0,1 et, de préférence, inférieur à - 1,0, **caractérisé par le fait que** la réaction est conduite en présence d'une quantité efficace d'une p-fuchsone.

**54.** Procédé selon la revendication 53 **caractérisé par le fait que** le composé phénolique est un composé phénolique de formule (I) décrit dans l'une des revendications 24 à 28.

**55.** Procédé selon l'une des revendications 53 et 54 **caractérisé par le fait que** la p-fuchsone est une fuchsone

répondant à la formule (III) et/ou (IV) décrite dans l'une des revendications 2 à 12.

**56.** Procédé selon l'une des revendications 53 à 55 **caractérisé par le fait que** la p-fuchsone est obtenue selon le procédé décrit dans l'une des revendications 23 à 51.

**57.** Procédé selon l'une des revendications 53 à 56 **caractérisé par le fait que** la quantité de fuchsone de formule (III) et/ou (IV) est inférieure à 1,0 mole par mole de peroxyde d'hydrogène, de préférence comprise entre $1.10^{-3}$ et 1,0 et, encore plus préférentiellement entre 0,05 et 0,5.

**58.** Procédé selon l'une des revendications 53 à 57 **caractérisé par le fait que** l'acide fort est un acide défini dans l'une des revendications 16 et 17.

**59.** Procédé selon l'une des revendications 53 à 58 **caractérisé par le fait que** la quantité d'acide est telle que le rapport $H^+/H_2O_2$ est compris entre environ $1.10^{-4}$ et environ 1,0, de préférence, entre $1.10^{-3}$ et 0,1.

**60.** Procédé selon l'une des revendications 53 à 59 **caractérisé par le fait que** le rapport molaire $H_2O_2$/composé phénolique de formule (I) est compris entre 0,01 et 0,3, de préférence entre 0,05 et 0,10.

**61.** Procédé selon l'une des revendications 53 à 60 **caractérisé par le fait que** la réaction est conduite en présence d'un solvant organique, aprotique, polaire, présentant une polarité telle que sa constante diélectrique est supérieure ou égale à 20, de préférence comprise entre 25 et 75 et une basicité telle qu'il possède un "nombre donneur" inférieur à 25, de préférence inférieur ou égal à 20, et encore plus préférentiellement compris entre 2 et 17.

**62.** Procédé selon la revendication 61 **caractérisé par le fait que** le solvant organique polaire est choisi parmi : les composés nitrés, de préférence, le nitrométhane, le nitroéthane, le nitro-1 propane, le nitro-2 propane ou leurs mélanges, le nitrobenzène ; les nitriles aliphatiques ou aromatiques, de préférence, l'acétonitrile, le propionitrile, le butanenitrile, l'isobutanenitrile, le benzonitrile, le cyanure de benzyle ; la tétraméthylène sulfone et le carbonate de propylène.

**63.** Procédé selon l'une des revendications 61 et 62 **caractérisé par le fait que** la quantité de solvant mis en oeuvre est telle que le rapport molaire entre le nombre de moles de solvant organique et le nombre de moles de composé phénolique de formule (I) varie entre 0,1 et 2,0, de préférence, entre 0,25 et 1,0.

**64.** Procédé selon l'une des revendications 53 à 63 **caractérisé par le fait que** la réaction est conduite en présence d'un solvant organique, aprotique, polaire, présentant une polarité telle que sa constante diélectrique est inférieure à environ 20, de préférence, comprise entre 2 et 15 et une basicité telle qu'il possède un "nombre donneur" supérieur ou égal à 15 et inférieur à 25, de préférence, compris entre 15 et 25.

**65.** Procédé selon la revendication 64 **caractérisé par le fait que** le solvant organique polaire est choisi parmi : les éther-oxydes aliphatiques, cycloaliphatiques ou aromatiques, de préférence, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne; les esters neutres phosphoriques, de préférence, le phosphate de triméthyle, le phosphate de triéthyle, le phosphate de butyle, le phosphate de triisobutyle, le phosphate de tripentyle; le carbonate d'éthylène.

**66.** Procédé selon l'une des revendications 64 et 65 **caractérisé par le fait que** la quantité de solvant mis en oeuvre est telle que le rapport molaire entre le nombre de moles de solvant organique et le nombre de moles de composé phénolique de formule (I) varie entre 0,01 et 0,25, de préférence, entre 0,025 et 0,15.

**67.** Procédé selon l'une des revendications 56 à 66 **caractérisé par le fait que** l'on opère à une température comprise entre 45°C et 150°C, de préférence, entre 60°C et 110°C.

**68.** Procédé selon l'une des revendications 56 à 67 **caractérisé par le fait que** l'on fait réagir le phénol et une solution de peroxyde d'hydrogène, en présence d'une quantité efficace d'un acide fort et d'une quantité efficace de diphénylfuchsone et/ou de son carbinol.

## Patentansprüche

1. Verfahren zur Herstellung einer para-dihydroxylierten aromatischen Verbindung, **dadurch gekennzeichnet, daß** es darin besteht, ein Oxidationsmittel mit einem p-Fuchson zur Reaktion zu bringen.

2. Verfahren nach Anspruch 1, **dadurch gekennzeichnet, daß** es darin besteht, ein Oxidationsmittel mit einem Fuchson zur Reaktion zu bringen, das der allgemeinen Formel (III) und/oder (IV) entspricht:

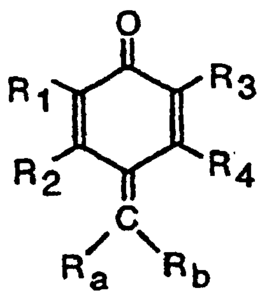 (III) 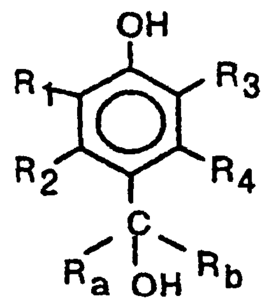 (IV)

wobei in den genannten Formeln (III) und (IV)

- $R_a$ und $R_b$, gleich oder verschieden, Kohlenwasserstoff-Reste mit jeweils 3 bis 30 Kohlenstoffatomen sind, wobei die Kohlenstoffatome von jedem Rest $R_a$ und $R_b$ in Position $\alpha$ zu dem Kohlenstoffatom, das sie trägt, tertiäre Kohlenstoffe sind,
- $R_1$, $R_2$, $R_3$ und $R_4$, gleich oder verschieden, ein Wasserstoffatom oder irgendeinen Substituenten darstellen,
- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen Ring bilden können, vorzugsweise einen ungesättigten oder aromatischen Carbocyclus mit 4 bis 7 Kohlenstoffatomen.

3. Verfahren nach Anspruch 2, **dadurch gekennzeichnet, daß** das Fuchson ein solches Fuchson ist, das der allgemeinen Formel (III) und/oder (IV) entspricht, in der

- $R_1$, $R_2$, $R_3$ und $R_4$, gleich oder verschieden, $R_0$ darstellen, eine der folgenden Gruppen:

  · ein Wasserstoffatom
  · einen linearen oder verzweigten Rest Alkyl mit 1 bis 6 Kohlenstoffatomen, vorzugsweise 1 bis 4 Kohlenstoffatomen, wie Methyl, Ethyl, Propyl, Isopropyl, Butyl, sek.-Butyl, tert.-Butyl,
  · einen linearen oder verzweigten Rest Alkenyl mit 2 bis 6 Kohlenstoffatomen, vorzugsweise 2 bis 4 Kohlenstoffatomen, wie Vinyl, Allyl,
  · einen linearen oder verzweigten Rest Alkoxy mit 1 bis 6 Kohlenstoffatomen, vorzugsweise 1 bis 4 Kohlenstoffatomen, wie die Reste Methoxy, Ethoxy, Propoxy, Isopropoxy, Butoxy,
  · eine Gruppe Acyl mit 2 bis 6 Kohlenstoffatomen,
  · einen Rest der Formel

$$-R_5-OH$$

$$-R_5-COOR_6$$

$$-R_5-X$$

$$-R_5-CF_3$$

wobei in den genannten Formeln $R_5$ eine Valenzbindung oder einen linearen oder verzweigten, gesättigten oder ungesättigten, divalenten Kohlenwasserstoff-Rest mit 1 bis 6 Kohlenstoffatomen darstellt, wie beispielsweise Methylen, Ethylen, Propylen, Isopropylen, Isopropyliden; $R_6$ einen linearen oder verzweigten Rest Alkyl mit 1 bis 6 Kohlenstoffatomen darstellt; X ein Halogenatom symbolisiert, vorzugsweise ein Atom von Chlor, Brom oder Fluor;

- $R_1$, $R_2$, $R_3$ und $R_4$, gleich oder verschieden, $R_7$ darstellen, einen der folgenden komplexeren Reste:

  · einen gesättigten oder ungesättigten carbocyclischen Rest mit 4 bis 7 Kohlenstoffatomen, vorzugsweise einen Rest Cyclohexyl,
  · einen Rest der Formel

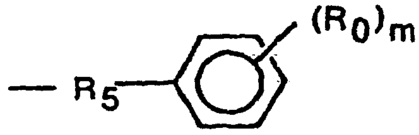

worin $R_5$ eine Valenzbindung oder einen linearen oder verzweigten, gesättigten oder ungesättigten, divalenten Kohlenwasserstoff-Rest mit 1 bis 6 Kohlenstoffatomen darstellt, wie beispielsweise Methylen, Ethylen, Propylen, Isopropylen, Isopropyliden; und $R_0$ die vorstehend angegebene Bedeutung besitzt und m eine ganze Zahl von 0 bis 4 ist, einen Rest $-R_5-A-R_8$ darstellt, worin $R_5$ die vorstehend angegebene Bedeutung besitzt, $R_8$ einen linearen oder verzweigten Rest Alkyl mit 1 bis 6 Kohlenstoffatomen oder einen Rest der Formel

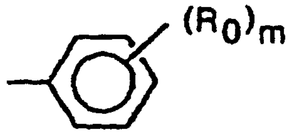

darstellt, und A eine der folgenden Gruppen symbolisiert -O-, -COO-, -OCOO-, $-SO_2-$,

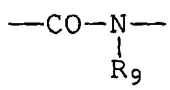

worin in diesen Formeln $R_9$ ein Wasserstoffatom oder einen Rest Alkyl mit 1 bis 4 Kohlenstoffatomen, einen Rest Cyclohexyl oder Phenyl darstellt,

- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen ungesättigten oder aromatischen Carbocyclus mit 4 bis 7 Kohlenstoffatomen und vorzugsweise 6 Kohlenstoffatomen bilden können.

**4.** Verfahren nach irgendeinem der Ansprüche 2 und 3, **dadurch gekennzeichnet, daß** das Fuchson ein solches Fuchson ist, das der allgemeinen Formel (III) und/oder (IV) entspricht, in der

- $R_1$, $R_2$, $R_3$ und $R_4$, gleich oder verschieden, eine der folgenden Gruppen darstellen:

  · ein Wasserstoffatom,
  · einen linearen oder verzweigten Rest Alkyl mit 1 bis 4 Kohlenstoffatomen,
  · einen linearen oder verzweigten Rest Alkoxy mit 1 bis 4 Kohlenstoffatomen,
  · eine Gruppe Hydroxyl,
  · ein Halogenatom,
  · eine Gruppe $-CF_3$,
  · einen Rest Cyclohexyl,
  · einen Rest Phenyl,

- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen benzolischen Ring bilden können.

**5.** Verfahren nach einem der Ansprüche 2 bis 4, **dadurch gekennzeichnet, daß** das Fuchson ein solches Fuchson ist, das der allgemeinen Formel (III) und/oder (IV) entspricht, in der einer der Reste $R_1$, $R_2$, $R_3$ und $R_4$ eine Gruppe Hydroxyl, einen Rest Methyl oder einen Rest Methoxy darstellt und die anderen drei ein Wasserstoffatom bedeuten.

**6.** Verfahren nach einem der Ansprüche 1 bis 5, **dadurch gekennzeichnet, daß** das Fuchson ein solches Fuchson ist, das der allgemeinen Formel (III) und/oder (IV) entspricht, in der $R_a$ und $R_b$ einen verzweigten Rest Alkyl mit mindestens 3 Kohlenstoffatomen oder einen Rest Aryl mit mindestens 6 Kohlenstoffatomen darstellen, vorzugsweise einen Rest tert.-Butyl, tert.-Pentyl, tert.-Hexyl oder Phenyl, gegebenenfalls substituiert.

**7.** Verfahren nach einem der Ansprüche 1 bis 6, **dadurch gekennzeichnet, daß** das Fuchson ein solches Fuchson ist, das der folgenden allgemeinen Formel (IIIa) und/oder (IVa) entspricht:

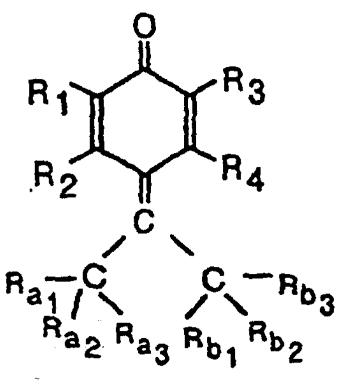
(IIIa)

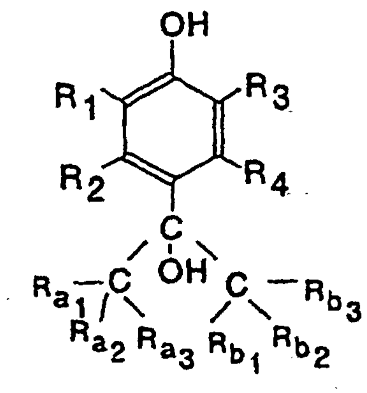
(IVa)

wobei in den genannten Formeln (IIIa) und (IVa)

- $R_{a1}$, $R_{a2}$, $R_{a3}$ und $R_{b1}$, $R_{b2}$, $R_{b3}$, gleich oder verschieden, lineare oder verzweigte Reste Alkyl mit 1 bis 10 Kohlenstoffatomen, Reste Cyclohexyl, Phenyl oder Naphthyl, gegebenenfalls substituiert, darstellen,
- $R_{a1}$, $R_{a2}$, $R_{a3}$ einerseits und $R_{b1}$, $R_{b2}$, $R_{b3}$ andererseits zusammen und mit dem Kohlenstoffatom, das sie trägt, einen benzolischen oder naphthalinischen Ring bilden können, gegebenenfalls substituiert,
- $R_1$, $R_2$, $R_3$ und $R_4$, gleich oder verschieden, eine der folgenden Gruppen darstellen:
  - ein Wasserstoffatom,
  - einen linearen oder verzweigten Rest Alkyl mit 1 bis 4 Kohlenstoffatomen,
  - einen linearen oder verzweigten Rest Alkoxy mit 1 bis 4 Kohlenstoffatomen,
  - eine Gruppe Hydroxyl,
  - ein Halogenatom,
  - eine Gruppe $-CF_3$,
  - einen Rest Cyclohexyl,
  - einen Rest Phenyl,
- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen benzolischen Ring bilden können.

**8.** Verfahren nach einem der Ansprüche 1 bis 7, **dadurch gekennzeichnet, daß** das Fuchson ein solches Fuchson ist, das der allgemeinen Formel (IIIa) und/oder (IVa) entspricht, in der einer der Reste $R_1$, $R_2$, $R_3$ und $R_4$ eine Gruppe Hydroxyl, einen Rest Methyl, einen Rest Methoxy darstellt und die anderen drei ein Wasserstoffatom bedeuten.

**9.** Verfahren nach einem der Ansprüche 1 bis 8, **dadurch gekennzeichnet, daß** das Fuchson ein solches Fuchson ist, das der folgenden allgemeinen Formel (IIIb) und/oder (IVb) entspricht:

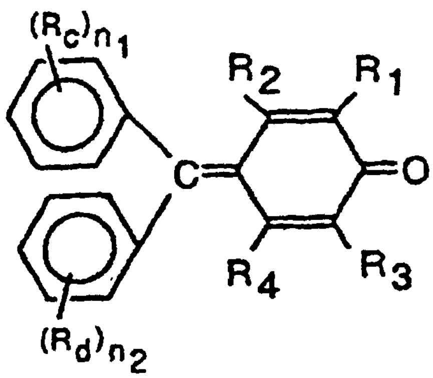

(IIIb)

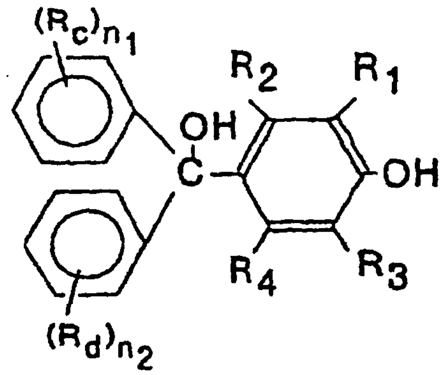

(IVb)

wobei in den genannten Formeln (IIIb) und (IVb)

- $R_1$, $R_2$, $R_3$ und $R_4$, gleich oder verschieden, eine der folgenden Gruppen darstellen:

  · ein Wasserstoffatom,
  · einen linearen oder verzweigten Rest Alkyl mit 1 bis 4 Kohlenstoffatomen,
  · einen linearen oder verzweigten Rest Alkoxy mit 1 bis 4 Kohlenstoffatomen,
  · eine Gruppe Hydroxyl,
  · ein Halogenatom,
  · eine Gruppe $-CF_3$,
  · einen Rest Cyclohexyl,
  · einen Rest Phenyl,

- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen benzolischen Ring bilden können,
- $R_c$ und $R_d$, gleich oder verschieden, ein Wasserstoffatom oder einen Substituenten darstellen,
- $n_1$, $n_2$, gleich oder verschieden, eine ganze Zahl von 0, 1, 2 oder 3 sind.

**10.** Verfahren nach einem der Ansprüche 1 bis 9, **dadurch gekennzeichnet, daß** das Fuchson ein solches Fuchson ist, das der allgemeinen Formel (IIIb) und/oder (IVb) entspricht, in der $R_c$ und $R_d$, gleich oder verschieden, darstellen:

- lineare oder verzweigte Reste Alkyl mit 1 bis 4 Kohlenstoffatomen,
- einen Rest Phenyl,
- Reste Alkoxy $R_{10}$-O, worin $R_{10}$ einen linearen oder verzweigten Rest Alkyl mit 1 bis 4 Kohlenstoffatomen oder den Rest Phenyl darstellt,
- ein Halogenatom, vorzugsweise Chlor, Brom oder Fluor.

**11.** Verfahren nach einem der Ansprüche 1 bis 10, **dadurch gekennzeichnet, daß** das Fuchson ein solches Fuchson ist, das der allgemeinen Formel (IIIb) und/oder (IVb) entspricht, in der $R_c$ und $R_d$, gleich oder verschieden, ein Wasserstoffatom oder einen wie in Anspruch 38 definierten Substituenten, vorzugsweise in Position 4,4' darstellen und $n_1$, $n_2$, gleich oder verschieden, gleich 0 oder 1 sind.

**12.** Verfahren nach einem der Ansprüche 1 bis 11, **dadurch gekennzeichnet, daß** das Fuchson ein solches Fuchson ist, das der allgemeinen Formel (IIIb) und/oder (IVb) entspricht, in der $R_c$ und $R_d$, gleich oder verschieden, ein Wasserstoffatom; einen Rest Methyl, Ethyl, tert.-Butyl, Phenyl; einen Rest Methoxy oder Ethoxy; eine Gruppe Hydroxyl, vorzugsweise in Position 3,3' oder 4,4' darstellen.

**13.** Verfahren nach einem der Ansprüche 1 bis 12, **dadurch gekennzeichnet, daß** das Oxidationsmittel unter Wasserstoffperoxid; den Persäuren, vorzugsweise Peressigsäure; den Hydroperoxiden, vorzugsweise tert.-Butyl-hydroperoxid, Cyclohexyl-hydroperoxid, Cumyl-hydroperoxid ausgewählt wird.

**14.** Verfahren nach Anspruch 13, **dadurch gekennzeichnet, daß** das Oxidationsmittel eine wäßrige Lösung von Wasserstoffperoxid ist.

**15.** Verfahren nach einem der Ansprüche 1 bis 14, **dadurch gekennzeichnet, daß** die Menge von Wasserstoffperoxid vorzugsweise gleich der stöchiometrischen Menge ist, das sind 1 mol $H_2O_2$ pro 1 mol Fuchson der Formel (III) und/oder (IV).

**16.** Verfahren nach einem der Ansprüche 1 bis 15, **dadurch gekennzeichnet, daß** man das Oxidationsmittel und das Fuchson in Anwesenheit eines sauren Katalysators zur Reaktion bringt, der eine starke protonische Säure mit einem pKa in Wasser von unter -0,1 und vorzugsweise unter -1,0 ist.

**17.** Verfahren nach einem der Ansprüche 1 bis 16, **dadurch gekennzeichnet, daß** die starke Säure eine halogenierte oder nicht halogenierte Sauerstoffsäure ist, vorzugsweise Schwefelsäure, Pyroschwefelsäure, Perchlorsäure, die Halogensulfonsäuren, vorzugsweise Fluorsulfonsäure, Chlorsulfonsäure oder Trifluormethansulfonsäure, Methansulfonsäure, Ethansulfonsäure, Ethandisulfonsäure, Benzolsulfonsäure, die Benzoldisulfonsäuren, die Toluolsulfonsäuren, die Naphthalinsulfonsäuren und die Naphthalindisulfonsäuren; und noch bevorzugter Perchlorsäure, Trifluormethansulfonsäure oder Methansulfonsäure.

**18.** Verfahren nach einem der Ansprüche 1 bis 17, **dadurch gekennzeichnet, daß** die Menge an Säure, ausgedrückt durch das Verhältnis der Anzahl der Äquivalente von Protonen zur Anzahl der Mole von Wasserstoffperoxid zwischen etwa $1.10^{-4}$ und etwa 1,0, vorzugsweise zwischen $1.10^{-3}$ und 0,1 variiert.

**19.** Verfahren nach einem der Ansprüche 1 bis 18, **dadurch gekennzeichnet, daß** die Reaktion in einem Reaktionslösungsmittel durchgeführt wird, das eine phenolische Verbindung der Formel (I) und/oder ein organisches Lösungsmittel ist.

**20.** Verfahren nach Anspruch 19, **dadurch gekennzeichnet, daß** das organische Lösungsmittel ausgewählt wird unter:

- den aliphatischen oder aromatischen Nitrilen, vorzugsweise Acetonitril, Propionitril, Butannitril, Isobutannitril, Benzonitril, Benzylcyanid,
- den aliphatischen, cycloaliphatischen oder aromatischen Etheroxiden, vorzugsweise Diethyloxid, Dipropyloxid, Diisopropyloxid, Dibutyloxid, Methyl-tert.-butylether, Dipentyloxid, Diisopentyloxid, Dimethylether von Ethylenglycol (oder 1,2-Dimethoxyethan), Dimethylether von Diethylenglycol (oder 1,5-Dimethoxy-3-oxa-pentan), Dioxan, Tetrahydrofuran,
- den chlorierten aliphatischen Kohlenwasserstoffen, vorzugsweise Dichlormethan, Tetrachlormethan, Dichlorethan,
- den Alkoholen, vorzugsweise Methanol, Ethanol, Isopropanol, tert.-Butanol.

**21.** Verfahren nach einem der Ansprüche 1 bis 20, **dadurch gekennzeichnet, daß** die Temperatur der Reaktion zwischen 20 °C und 150 °C, vorzugsweise zwischen 30 °C und 80 °C beträgt.

**22.** Verfahren nach einem der Ansprüche 1 bis 21, **dadurch gekennzeichnet, daß** man Diphenylfuchson oder Rosolsäure, Diphenylmethylfuchson mit Hilfe einer Lösung von Wasserstoffperoxid im Hinblick darauf oxidiert, jeweils Hydrochinon oder Methylhydrochinon zu erhalten.

**23.** Verfahren nach einem der Ansprüche 1 bis 22, **dadurch gekennzeichnet, daß** das eingesetzte Fuchson ein solches Fuchson ist, das nach einem Verfahren erhalten wird, das darin besteht, eine phenolische Verbindung mit mindestens einem Wasserstoffatom in para-Position und eine ketonische, nicht enolisierbare Verbindung in Anwesenheit einer wirksamen Menge von einer protonischen Säure, die eine Funktion der Azidität $-H_o$ von mindestens 5 aufweist, und gegebenenfalls in Anwesenheit einer wirksamen Menge einer ionisierbaren Schwefelverbindung zur Reaktion zu bringen.

**24.** Verfahren nach Anspruch 23, **dadurch gekennzeichnet, daß** die phenolische Verbindung, die mindestens ein Wasserstoffatom in para-Position aufweist, eine phenolische Verbindung ist, die der allgemeinen Formel (I) entspricht:

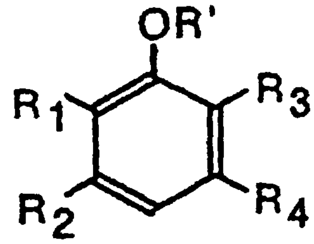

(I)

wobei in der genannten Formel (I):

- die para-Position frei ist,
- $R_1$, $R_2$, $R_3$ und $R_4$, gleich oder verschieden, ein Wasserstoffatom oder irgendeinen Substituenten darstellen,
- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen Ring bilden können,
- R' ein Wasserstoffatom oder einen Kohlenwasserstoff-Rest mit 1 bis 24 Kohlenstoffatomen darstellt, der ein linearer oder verzweigter, gesättigter oder ungesättigter, acyclischer aliphatischer Rest; ein gesättigter oder ungesättigter, monocyclischer oder polycyclischer, cycloaliphatischer Rest; ein linearer oder verzweigter, gesättigter oder ungesättigter, aliphatischer Rest sein kann, der einen cyclischen Substituenten trägt.

**25.** Verfahren nach einem der Ansprüche 23 und 24, **dadurch gekennzeichnet, daß** die phenolische Verbindung der allgemeinen Formel (I) entspricht, in der:

- der Rest R' eine der folgenden Gruppen darstellt:

  · ein Wasserstoffatom,
  · einen linearen oder verzweigten Rest Alkyl mit 1 bis 6 Kohlenstoffatomen, vorzugsweise 1 bis 4 Kohlenstoffatomen,
  · einen Rest Cyclohexyl,
  · einen Rest Phenyl,
  · einen Rest Benzyl,

- $R_1$, $R_2$, $R_3$ und $R_4$, gleich oder verschieden, $R_0$ darstellen, eine der folgenden Gruppen:

  · ein Wasserstoffatom
  · einen linearen oder verzweigten Rest Alkyl mit 1 bis 6 Kohlenstoffatomen, vorzugsweise 1 bis 4 Kohlenstoffatomen, wie Methyl, Ethyl, Propyl, Isopropyl, Butyl, sek.-Butyl, tert.-Butyl,
  · einen linearen oder verzweigten Rest Alkenyl mit 2 bis 6 Kohlenstoffatomen, vorzugsweise 2 bis 4 Kohlenstoffatomen, wie Vinyl, Allyl,
  · einen linearen oder verzweigten Rest Alkoxy mit 1 bis 6 Kohlenstoffatomen, vorzugsweise 1 bis 4 Kohlenstoffatomen, wie die Reste Methoxy, Ethoxy, Propoxy, Isopropoxy, Butoxy,
  · eine Gruppe Acyl mit 2 bis 6 Kohlenstoffatomen,
  · einen Rest der Formel

$$-R_5-OH$$

$$-R_5-COOR_6$$

$$-R_5-X$$

$$-R_5-CF_3$$

wobei in den genannten Formeln $R_5$ eine Valenzbindung oder einen linearen oder verzweigten, gesättigten oder ungesättigten, divalenten Kohlenwasserstoff-Rest mit 1 bis 6 Kohlenstoffatomen darstellt, wie beispielsweise Methylen, Ethylen, Propylen, Isopropylen, Isopropyliden; $R_6$ einen linearen oder verzweigten Rest Alkyl mit 1 bis 6 Kohlenstoffatomen darstellt; X ein Halogenatom symbolisiert, vorzugsweise ein Atom von Chlor, Brom oder Fluor;

- $R_1$, $R_2$, $R_3$ und $R_4$, gleich oder verschieden, $R_7$ darstellen, einen der folgenden komplexeren Reste:

  · einen gesättigten oder ungesättigten carbocyclischen Rest mit 4 bis 7 Kohlenstoffatomen, vorzugsweise einen Rest Cyclohexyl,
  · einen Rest der Formel

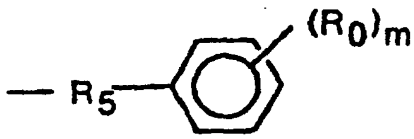

worin $R_5$ eine Valenzbindung oder einen linearen oder verzweigten, gesättigten oder ungesättigten, divalenten Kohlenwasserstoff-Rest mit 1 bis 6 Kohlenstoffatomen darstellt, wie beispielsweise Methylen, Ethylen, Propylen, Isopropylen, Isopropyliden; und $R_0$ die vorstehend angegebene Bedeutung besitzt und m eine ganze Zahl von 0 bis 4 ist, einen Rest $-R_5-A-R_8$ darstellt, worin $R_5$ die vorstehend angegebene Bedeutung besitzt, $R_8$ einen linearen oder verzweigten Rest Alkyl mit 1 bis 6 Kohlenstoffatomen oder einen Rest der Formel

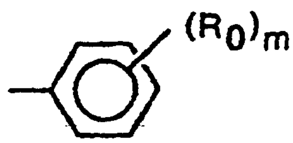

darstellt, und A eine der folgenden Gruppen symbolisiert -O-, -COO-, -OCOO-, $-SO_2-$,

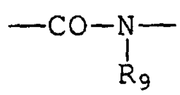

worin in diesen Formeln $R_9$ ein Wasserstoffatom oder einen Rest Alkyl mit 1 bis 4 Kohlenstoffatomen, einen Rest Cyclohexyl oder Phenyl darstellt,

- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen ungesättigten oder aromatischen Carbocyclus mit 4 bis 7 Kohlenstoffatomen und vorzugsweise 6 Kohlenstoffatomen bilden können.

**26.** Verfahren nach einem der Ansprüche 23 bis 25, **dadurch gekennzeichnet, daß** die phenolische Verbindung der allgemeinen Formel (I) entspricht, in der:

- der Rest R' ein Wasserstoffatom darstellt,
- $R_1$, $R_2$, $R_3$ und $R_4$, gleich oder verschieden, eine der folgenden Gruppen darstellen:

  · ein Wasserstoffatom,
  · einen linearen oder verzweigten Rest Alkyl mit 1 bis 4 Kohlenstoffatomen,
  · einen linearen oder verzweigten Rest Alkoxy mit 1 bis 4 Kohlenstoffatomen,
  · eine Gruppe Hydroxyl,
  · ein Halogenatom,
  · eine Gruppe $-CF_3$,

- einen Rest Cyclohexyl,
- einen Rest Phenyl,

- zwei Gruppen $R_1$ und $R_2$ und/oder $R_3$ und $R_4$, die an zwei vicinalen Kohlenstoffatomen angeordnet sind, zusammen und mit den Kohlenstoffatomen, die sie tragen, einen benzolischen Ring bilden können.

**27.** Verfahren nach einem der Ansprüche 23 bis 26, **dadurch gekennzeichnet, daß** die phenolische Verbindung der allgemeinen Formel (I) entspricht, in der R' ein Wasserstoffatom ist und einer der Reste $R_1$, $R_2$, $R_3$ und $R_4$ eine Gruppe Hydroxyl, einen Rest Methyl oder einen Rest Methoxy darstellt und die anderen drei ein Wasserstoffatom bedeuten.

**28.** Verfahren nach einem der Ansprüche 23 bis 27, **dadurch gekennzeichnet, daß** die phenolische Verbindung Phenol, Anisol, ortho-Kresol, meta-Kresol, 2-Methoxyphenol, 2-Ethylphenol, 3-Ethylphenol, 2-Propylphenol, 2-sek. -Butylphenol, 2-tert.-Butylphenol, 3-tert.-Butylphenol, 2-Methoxyphenol, 3-Methoxyphenol, Salicylsäuremethylester, 2-Chlorphenol, 3-Chlorphenol; 2,3-Dimethylphenol, 2,5-Dimethylphenol, 2,6-Dimethylphenol, 3,5-Dimethylphenol, 2,3-Dichlorphenol, 2,5-Dichlorphenol, 2,6-Dichlorphenol, 3,5-Dichlorphenol, 2,6-Di-tert.-butylphenol, 3,5-Di-tert.-butylphenol; 2,3,5-Trimethylphenol, 2,3,6-Trimethylphenol, 2,3,5-Trichlorphenol, 2,3,6-Trichlorphenol; 1-Hydroxynaphthalin; 2-Phenoxyphenol, 3-Phenoxyphenol ist.

**29.** Verfahren nach einem der Ansprüche 23 bis 28, **dadurch gekennzeichnet, daß** die ketonische, nicht enolisierbare Verbindung eine ketonische Verbindung ist, die der allgemeinen Formel (II) entspricht:

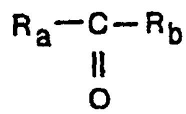

(II)

wobei in der genannten Formel (II):

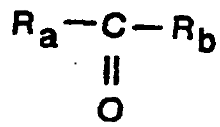

(II)

- $R_a$ und $R_b$, gleich oder verschieden, Kohlenwasserstoff-Reste mit jeweils 3 bis 30 Kohlenstoffatomen sind, wobei die Kohlenstoffatome von jedem Rest $R_a$ und $R_b$ in Position $\alpha$ zur Carbonylgruppe tertiäre Kohlenstoffe sind.

**30.** Verfahren nach einem der Ansprüche 23 bis 29, **dadurch gekennzeichnet, daß** die ketonische Verbindung der allgemeinen Formel (IIa) entspricht:

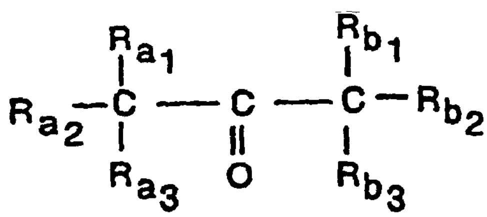

(IIa)

wobei in der genannten Formel (IIa):

- $R_{a1}$, $R_{a2}$, $R_{a3}$ und $R_{b1}$, $R_{b2}$, $R_{b3}$, gleich oder verschieden, lineare oder verzweigte Reste Alkyl mit 1 bis 10

Kohlenstoffatomen, Reste Cyclohexyl, Phenyl oder Naphthyl, gegebenenfalls substituiert, darstellen,

- $R_{a1}$, $R_{a2}$, $R_{a3}$ und/oder $R_{b1}$, $R_{b2}$, $R_{b3}$ zusammen und mit dem Kohlenstoffatom, das sie trägt, einen benzolischen oder naphthalinischen Ring bilden können, gegebenenfalls substituiert.

**31.** Verfahren nach einem der Ansprüche 23 bis 30, **dadurch gekennzeichnet, daß** die ketonische Verbindung der allgemeinen Formel (IIb) entspricht:

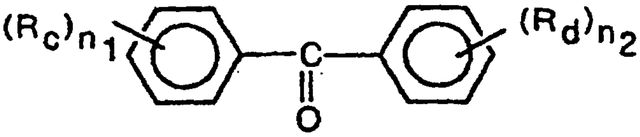

(IIb)

wobei in der genannten Formel (IIb):

- $R_c$ und $R_d$, gleich oder verschieden, ein Wasserstoffatom oder einen Substituenten darstellen,
- $n_1$, $n_2$, gleich oder verschieden, eine ganze Zahl von 0, 1, 2 oder 3 sind.

**32.** Verfahren nach einem der Ansprüche 23 bis 31, **dadurch gekennzeichnet, daß** die ketonische Verbindung der allgemeinen Formel (IIb) entspricht, in der $R_c$ und $R_d$, gleich oder verschieden, darstellen:

- lineare oder verzweigte Reste Alkyl mit 1 bis 4 Kohlenstoffatomen,
- einen Rest Phenyl,
- Reste Alkoxy $R_{10}$-O, worin $R_{10}$ einen linearen oder verzweigten Rest Alkyl mit 1 bis 4 Kohlenstoffatomen oder den Rest Phenyl darstellt,
- eine Gruppe Hydroxyl,
- ein Fluoratom.

**33.** Verfahren nach einem der Ansprüche 23 bis 32, **dadurch gekennzeichnet, daß** die ketonische Verbindung der allgemeinen Formel (IIb) entspricht, in der $R_c$ und $R_d$, gleich oder verschieden, ein Wasserstoffatom oder einen wie in Anspruch 32 definierten Substituenten darstellen, vorzugsweise in Position 4,4', und $n_1$, $n_2$, gleich oder verschieden, gleich 0 oder 1 sind.

**34.** Verfahren nach einem der Ansprüche 23 bis 33, **dadurch gekennzeichnet, daß** die ketonische Verbindung der allgemeinen Formel (IIb) entspricht, in der $R_c$ und $R_d$, gleich oder verschieden, ein Wasserstoffatom; einen Rest Methyl, Ethyl, tert.-Butyl, Phenyl; einen Rest Methoxy oder Ethoxy; eine Gruppe Hydroxyl darstellen, vorzugsweise in Position 3,3' oder 4,4'.

**35.** Verfahren nach einem der Ansprüche 23 bis 34, **dadurch gekennzeichnet, daß** die ketonische Verbindung der allgemeinen Formel (IIb) ist:

- Benzophenon,
- 2-Methylbenzophenon,
- 2,4-Dimethylbenzophenon,
- 4,4'-Dimethylbenzophenon,
- 2,2'-Dimethylbenzophenon,
- 4,4'-Dimethoxybenzophenon,
- 4-Hydroxybenzophenon,
- 4,4'-Dihydroxybenzophenon,
- 4-Benzoyl-biphenyl.

**36.** Verfahren nach einem der Ansprüche 23 bis 35, **dadurch gekennzeichnet, daß** der saure Katalysator eine Funktion der Azidität $-H_0$ zwischen 5 und 20, vorzugsweise zwischen 10 und 15 aufweist.

**37.** Verfahren nach Anspruch 36, **dadurch gekennzeichnet, daß** der saure Katalysator eine starke protonische Säure ist, vorzugsweise Fluorwasserstoffsäure, Perchlorsäure, Trifluormethansulfonsäure, para-Toluolsulfonsäure, Chlorsulfonsäure, Fluorsulfonsäure, Methansulfonsäure, Benzolsulfonsäure, und noch bevorzugter Fluorwasserstoffsäure, Perchlorsäure, Trifluormethansulfonsäure oder Methansulfonsäure.

**38.** Verfahren nach Anspruch 36, **dadurch gekennzeichnet, daß** der saure Katalysator ein sulfonisches Harz ist, vorzugsweise ein sulfoniertes Copolymer Styrol-Divinylbenzol, ein sulfoniertes Copolymer Phenol-Formaldehyd, ein Copolymer Tetrafluorethylenperfluor-[2-(fluorsulfonylethoxy)-propyl]-vinylether und vorzugsweise die folgenden Harze: TEMEX 50, AMBERLYST 15, AMBERLYST 35, AMBERLYST 36, DOWEX 50 W; DUOLITE ARC 9359; NAFION.

**39.** Verfahren nach Anspruch 36, **dadurch gekennzeichnet, daß** der saure Katalysator ausgewählt wird unter: Kieselerde-aluminat, Kieselerde-$Ga_2O_3$, Kieselerde-$B_2O_3$; den sauren Tonen; den Brückentonen (argiles pontées); den natürlichen oder synthetischen Zeolithen; den Phosphaten von Bor, Aluminium oder Gallium; den lamellaren Phosphaten von tetravalenten Metallen, entsprechend der Formel $\alpha$-$M(HPO_4)_2$,$pH_2O$, worin M ein tetravalentes Metall darstellt, ausgewählt unter Titan, Germanium, Zirkonium oder Zinn, und p eine ganze Zahl von unter 2 bedeutet; den lamellaren Phosphaten von trivalenten Metallen, vorzugsweise den lamellaren Phosphaten von Zirkonium; den Oxiden, die durch eine Behandlung sauer gemacht wurden, vorzugsweise den sulfatierten Oxiden von Titan, Zirkonium oder Eisen.

**40.** Verfahren nach Anspruch 36, **dadurch gekennzeichnet, daß** der verwendete saure Katalysator ausgewählt wird unter:

- den natürlichen Tonen mit einer sogenannten Struktur "TOT" oder Tetraeder-Oktatetraeder, vorzugsweise aus der Klasse der Smektite und noch bevorzugter der Montmorillonite,
- den handelsüblichen Tonen, die bereits sauer sind oder auch nicht, oder die durch eine wäßrige Lösung einer Säure behandelt wurden,
- den Brückentonen, hergestellt ausgehend von Smektiten, vorzugsweise ausgehend von Beidelliten, insbesondere von synthetischen Beidelliten oder von Montmorilloniten; die Brücke des Tones ist vorzugsweise eine Brücke mit Aluminium.

**41.** Verfahren nach Anspruch 36, **dadurch gekennzeichnet, daß** der saure Katalysator ausgewählt wird unter:

- den natürlichen Zeolithen wie Chabazit, Clinoptilolit, Erionit, Mordenit, Phillipsit, Offretit,
- den synthetischen Zeolithen wie Zeolith ZSM-5, Zeolith Y, Ferrierit, Zeolith X vom Typ Faujasit, Zeolith vom Typ L, Mordenit, Zeolith ZSM-11; Mazzit, Offretit, vorzugsweise ein Zeolith US-Y oder ein Zeolith ZSM-5.

**42.** Verfahren nach Anspruch 41, **dadurch gekennzeichnet, daß** der eingesetzte Zeolith ein sauer gemachter Zeolith ist.

**43.** Verfahren nach einem der Ansprüche 23 bis 42, **dadurch gekennzeichnet, daß** die ionisierbare Schwefelverbindung eine mineralische Schwefelverbindung oder eine organische Schwefelverbindung ist, vorzugsweise ausgewählt unter:

- den Schwefelhalogeniden,
- den Thiosulfaten von Ammonium, Alkalimetallen oder Erdalkalimetallen,
- Schwefelwasserstoff oder seinen Vorläufern,
- den Mercaptanen, vorzugsweise den Alkylmercaptanen, Phenylmercaptanen und Phenylalkylmercaptanen,
- den Thioalkoholen,
- den Thiophenolen,
- den thioorganischen Säuren, ihren Salzen oder ihren Estern,
- den Kationenaustauscherharzen, modifiziert durch Reaktion mit einem Alkyl-mercaptoamin, vorzugsweise den Harzen mit einem Polystyrolgerüst, die sulfonische Gruppen tragen und mit einem Alkyl-mercaptoamin reagiert haben, vorzugsweise mit einem solchen, das eine primären Aminogruppe aufweist und noch bevorzugter, das 1 bis 1 Kohlenstoffatome besitzt.

**44.** Verfahren nach Anspruch 43, **dadurch gekennzeichnet, daß** die Schwefelverbindung ausgewählt wird unter:

- Schwefel-monochlorid oder Schwefel-dichlorid,
- Thiosulfat von Ammonium, Natrium oder Kalium,
- Schwefelwasserstoff oder Sulfid von Calcium oder Kalium,

- Ethylmercaptan, Butylmercaptan, 1-Nonylmercaptan, Benzylmercaptan,
- 2-Mercaptoethanol, 3-Mercapto-2-butanol,
- Thiophenol, o-Thiokresol, m-Thiokresol, p-Thiokresol, Thioxylenol, p-Methylthiophenol, o-Ethylthiophenol, Thiohydrochinon, Thionaphthol,
- Thioessigsäure, Thiopropionsäure, Thiomilchsäure, Thiosalicylsäure, 2-Mercaptoethansulfonsäure, 3-Mercaptopropansulfonsäure, Mercaptobernsteinsäure, Thioglycolsäure, Thioglycolat von Methyl, Ethyl,
- den Harzen mit einem Polystyrolgerüst, die sulfonische Gruppen tragen und die mit 2-Mercapto-ethylamin, 2-Mercapto-isopropylamin, 3-Mercapto-butylamin reagiert haben.

**45.** Verfahren nach einem der Ansprüche 23 bis 44, **dadurch gekennzeichnet, daß** das molare Verhältnis zwischen der phenolischen Verbindung der Formel (I) und der ketonischen Verbindung der Formel (II) zwischen 1 und 20, vorzugsweise zwischen 1 und 10 beträgt.

**46.** Verfahren nach einem der Ansprüche 23, 36 bis 38, **dadurch gekennzeichnet, daß** die Menge von saurem Katalysator, ausgedrückt durch das Verhältnis der Äquivalente von Protonen zur Anzahl der Mole von ketonischer Verbindung der Formel (II) zwischen $1.10^{-3}$ und 1,0, vorzugsweise zwischen $5.10^{-3}$ und 0,5 variiert.

**47.** Verfahren nach Anspruch 36 oder 37, **dadurch gekennzeichnet, daß** das genannte Verhältnis zwischen 1,0 und 20, vorzugsweise zwischen 5,0 und 10 beträgt, wenn die Säure als Reaktionsmedium verwendet wird.

**48.** Verfahren nach einem der Ansprüche 39 bis 42, **dadurch gekennzeichnet, daß** die Menge von eingesetztem sauren Katalysator, dargestellt in Gewicht und bezogen auf die eingesetzte phenolische Verbindung der Formel (I), 0,1 bis 20 %, vorzugsweise 0,5 bis 10 % beträgt.

**49.** Verfahren nach einem der Ansprüche 23 bis 48, **dadurch gekennzeichnet, daß** die Menge von eingesetzter Schwefelverbindung, ausgedrückt im Verhältnis zur ketonischen Verbindung der Formel (II) derart ist, daß das molare Verhältnis zwischen der Schwefelverbindung und der ketonischen Verbindung der Formel (II) zwischen 0,001 und 1,0, vorzugsweise zwischen 0,005 und 0,20 beträgt.

**50.** Verfahren nach einem der Ansprüche 23 bis 49, **dadurch gekennzeichnet, daß** die Temperatur der Reaktion zwischen 45 °C und 200 °C, vorzugsweise zwischen 60 °C und 150 °C beträgt.

**51.** Verfahren nach einem der Ansprüche 23 bis 50, **dadurch gekennzeichnet, daß** man das Diphenylfuchson durch Reaktion von Phenol und Benzophenon; die Rosolsäure durch Reaktion von Phenol und 4,4'-Dihydroxy-benzophenon; das Diphenylmethylfuchson durch Reaktion von ortho-Kresol und Benzophenon herstellt.

**52.** Verfahren nach Anspruch 23, **dadurch gekennzeichnet, daß** die para-dihydroxylierte aromatische Verbindung erhalten wird, indem man ein Fuchson nach dem in einem der Ansprüche 23 bis 51 beschriebenen Verfahren herstellt und dieses anschließend durch Zugabe eines Oxidationsmittels zu dem Reaktionsmedium oxidiert, ohne daß das Fuchson isoliert wird.

**53.** Verfahren zur Herstellung einer para-dihydroxylierten aromatische Verbindung durch Oxidation einer phenolischen Verbindung, die ein Wasserstoffatom in para-Position aufweist, mit Wasserstoffperoxid in Anwesenheit einer wirksamen Menge einer starken protonischen Säure, die einen pKa in Wasser von unter -0,1 und vorzugsweise unter -1,0 aufweist, **dadurch gekennzeichnet, daß** die Reaktion in Anwesenheit einer wirksamen eines p-Fuchsons durchgeführt wird.

**54.** Verfahren nach Anspruch 53, **dadurch gekennzeichnet, daß** die phenolische Verbindung eine phenolische Verbindung der Formel (I) ist, wie in einem der Ansprüche 24 bis 28 beschrieben.

**55.** Verfahren nach einem der Ansprüche 53 und 54, **dadurch gekennzeichnet, daß** das p-Fuchson ein Fuchson ist, das der allgemeinen Formel (III) und/oder (IV) entspricht, wie in einem der Ansprüche 2 bis 12 beschrieben.

**56.** Verfahren nach einem der Ansprüche 53 bis 55, **dadurch gekennzeichnet, daß** das p-Fuchson nach dem Verfahren erhalten wird, wie in einem der Ansprüche 23 bis 51 beschrieben.

**57.** Verfahren nach einem der Ansprüche 53 bis 56, **dadurch gekennzeichnet, daß** die Menge von Fuchson der Formel (III) und/oder (IV) unter 1,0 mol pro mol Wasserstoffperoxid, vorzugsweise zwischen $1.10^{-3}$ und 1,0 mol

und noch bevorzugter zwischen 0,05 und 0,5 mol beträgt.

**58.** Verfahren nach einem der Ansprüche 53 bis 57, **dadurch gekennzeichnet, daß** die starke Säure eine Säure ist, wie in einem der Ansprüche 16 und 17 definiert.

**59.** Verfahren nach einem der Ansprüche 53 bis 58, **dadurch gekennzeichnet, daß** die Menge der Säure derart ist, daß das Verhältnis $H^+/H_2O_2$ zwischen etwa $1.10^{-4}$ und etwa 1,0, vorzugsweise zwischen $1.10^{-3}$ und 0,1 beträgt.

**60.** Verfahren nach einem der Ansprüche 53 bis 59, **dadurch gekennzeichnet, daß** das molare Verhältnis $H_2O_2$/ phenolische Verbindung der Formel (I) zwischen 0,01 und 0,3, vorzugsweise zwischen 0,05 und 0,10 beträgt.

**61.** Verfahren nach einem der Ansprüche 53 bis 60, **dadurch gekennzeichnet, daß** die Reaktion in Anwesenheit eines polaren, aprotischen organischen Lösungsmittels durchgeführt wird, das eine solche Polarität, daß seine Dielektrizitätskonstante über oder gleich 20, vorzugsweise zwischen 25 und 75 beträgt, und eine solche Basizität aufweist, daß es eine "Donatorzahl" von unter 25, vorzugsweise von unter oder gleich 20 und noch mehr bevorzugt zwischen 2 und 17 besitzt.

**62.** Verfahren nach Anspruch 61, **dadurch gekennzeichnet, daß** das polare organische Lösungsmittel ausgewählt wird unter: den nitrierten Verbindungen, vorzugsweise Nitromethan, Nitroethan, 1-Nitropropan, 2-Nitropropan oder deren Mischungen, Nitrobenzol; den aliphatischen oder aromatischen Nitrilen, vorzugsweise Acetonitril, Propionitril, Butannitril, Isobutannitril, Benzonitril, Benzylcyanid; Tetramethylensulfon und Propylen-carbonat.

**63.** Verfahren nach einem der Ansprüche 61 und 62, **dadurch gekennzeichnet, daß** die Menge von eingesetztem Lösungsmittel derart ist, daß das molare Verhältnis zwischen der Anzahl von Molen des organischen Lösungsmittels und der Anzahl von Molen der phenolischen Verbindung der Formel (I) zwischen 0,1 und 2,0, vorzugsweise zwischen 0,25 und 1,0 variiert.

**64.** Verfahren nach einem der Ansprüche 53 bis 63, **dadurch gekennzeichnet, daß** die Reaktion in Anwesenheit eines polaren, aprotischen organischen Lösungsmittels durchgeführt wird, das eine solche Polarität, daß seine Dielektrizitätskonstante unter etwa 20, vorzugsweise zwischen 2 und 15 beträgt, und eine solche Basizität aufweist, daß es eine "Donatorzahl" von über oder gleich 15 und unter 25, vorzugsweise zwischen 15 und 25 besitzt.

**65.** Verfahren nach Anspruch 64, **dadurch gekennzeichnet, daß** das polare organische Lösungsmittel ausgewählt wird unter: den aliphatischen, cycloaliphatischen oder aromatischen Ether-oxiden, vorzugsweise Diethyloxid, Dipropyloxid, Diisopropyloxid, Dibutyloxid, Methyl-tert.-butylether, Dipentyloxid, Diisopentyloxid, Dimethylether von Ethylenglycol (oder 1,2-Dimethoxyethan), Dimethylether von Diethylenglycol (oder 1,5-Dimethoxy-3-oxa-pentan), Dioxan, Tetrahydrofuran; den neutralen Phosphorestern, vorzugsweise Trimethylphosphat, Triethylphosphat, Butylphosphat, Triisobutylphosphat, Tripentylphosphat; Ethylen-carbonat.

**66.** Verfahren nach einem der Ansprüche 64 und 65, **dadurch gekennzeichnet, daß** die Menge von eingesetztem Lösungsmittel derart ist, daß das molare Verhältnis zwischen der Anzahl von Molen des organischen Lösungsmittels und der Anzahl von Molen der phenolischen Verbindung der Formel (I) zwischen 0,01 und 0,25, vorzugsweise zwischen 0,025 und 0,15 variiert.

**67.** Verfahren nach einem der Ansprüche 56 bis 66, **dadurch gekennzeichnet, daß** man bei einer Temperatur zwischen 45 °C und 150 °C, vorzugsweise zwischen 60 °C und 110 °C arbeitet.

**68.** Verfahren nach einem der Ansprüche 56 bis 67, **dadurch gekennzeichnet, daß** man das Phenol und eine Lösung von Wasserstoffperoxid in Anwesenheit einer wirksamen Menge einer starken Säure und einer wirksamen Menge von Diphenylfuchson und/oder seinem Carbinol zur Reaktion bringt.

## Claims

**1.** A process for the preparation of a para-dihydroxylated aromatic compound, **characterised in that** it consists in reacting an oxidising agent with a p-fuchsone.

**2.** A process according to Claim 1, **characterised in that** it consists in reacting an oxidising agent with a fuchsone

corresponding to general formula (III) and/or (IV):

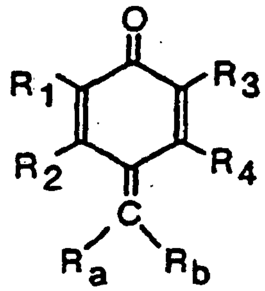 (III) 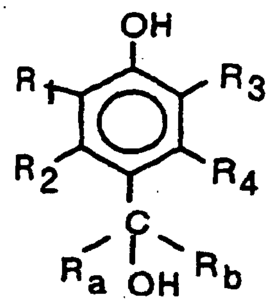 (IV)

in which formulae (III) and (IV):

- $R_a$ and $R_b$ which are identical or different are hydrocarbon radicals, each having from 3 to 30 carbon atoms; the carbon atoms of each radical $R_a$ and $R_b$ in $\alpha$ position in relation to the carbon atom bearing them being tertiary carbons,
- $R_1$, $R_2$, $R_3$ and $R_4$ which are identical or different represent a hydrogen atom or any substituent,
- two groups $R_1$ and $R_2$ and/or $R_3$ and $R_4$ placed on two neighbouring carbon atoms can form together and with the carbon atoms which bear them a ring, preferably an aromatic or unsaturated carbocycle with 4 to 7 carbon atoms.

**3.** A process according to claim 2, **characterised in that** the fuchsone is a fuchsone corresponding to general formula (III) and/or (IV) wherein:

- $R_1$, $R_2$, $R_3$ and $R_4$ which are identical or different represent $R_0$, one of the following groups:

  · a hydrogen atom,
  · a branched or straight chain alkyl radical having from 1 to 6 carbon atoms, preferably from 1 to 4 carbon atoms, such as methyl, ethyl, propyl, isopropyl, butyl, sec-butyl, tert-butyl,
  · a branched or straight chain alkenyl radical having from 2 to 6 carbon atoms, preferably from 2 to 4 carbon atoms, such as vinyl, allyl,
  · a branched or straight chain alkoxy radical having from 1 to 6 carbon atoms, preferably from 1 to 4 carbon atoms, such as the methoxy, ethoxy, propoxy, isopropoxy, butoxy radicals,
  · an acyl group having from 2 to 6 carbon atoms,
  · a radical of the formula:

$$-R_5-OH$$

$$-R_5-COOR_6$$

$$-R_5-X$$

$$-R_5-CF_3$$

  in which formulae, $R_5$ represents a valence bond or a saturated or unsaturated, branched or straight chain, divalent hydrocarbon radical having from 1 to 6 carbon atoms, such as methylene, ethylene, propylene, isopropylene, isopropilydene, for example; $R_6$ represents a branched or straight chain alkyl radical having 1 to 6 carbon atoms; X symbolises a halogen atom, preferably a chlorine, bromine or fluorine atom.
- $R_1$, $R_2$, $R_3$ and $R_4$ which are identical or different represent $R_7$, one of the following more complex radicals:

  · a saturated or unsaturated carbocyclic radical having from 4 to 7 carbon atoms, preferably a cyclohexyl

radical,

- a radical of the formula

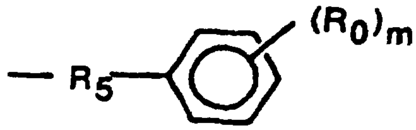

in which $R_5$ represents a valence bond or a saturated or unsaturated, branched or straight chain, divalent hydrocarbon radical having from 1 to 6 carbon atoms, such as methylene, ethylene, propylene, isopropylene, isopropylidene, for example, and $R_0$ having the meaning given hereinabove, and m is an integer of from 0 to 4,

- a radical - $R_5$ - A - $R_8$, in which $R_5$ has the meaning given hereinabove, $R_8$ represents a branched or straight chain alkyl radical having from I to 6 carbon atoms or a radical of the formula

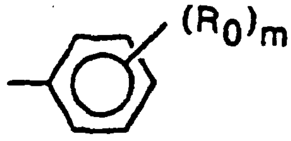

and A symbolises one of the following groups:
-O-, -COO-, -OCOO-, $-SO_2-$,

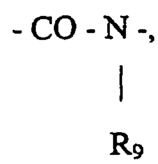

in which formulae, $R_9$ represents a hydrogen atom or an alkyl radical having from 1 to 4 carbon atoms, a cyclohexyl or phenyl radical,

- two groups $R_1$ and $R_2$ and/or $R_3$ and $R_4$ placed on two neighbouring carbon atoms can form together and with the carbon atoms which bear them an unsaturated or aromatic carbocycle having from 4 to 7 carbon atoms, and preferably 6 carbon atoms.

**4.** A process according to one of claims 2 and 3, **characterised in that** the fuchsone is a fuchsone corresponding to general formula (III) and/or (IV) in which:

- $R_1$, $R_2$, $R_3$ and $R_4$ which are identical or different represent one of the following groups:

  · a hydrogen atom,
  · a branched or straight chain alkyl radical having from 1 to 4 carbon atoms,
  · a branched or straight chain alkoxy radical having from 1 to 4 carbon atoms,
  · a hydroxyl group,
  · a halogen atom,
  · a group $-CF_3$,
  · a cyclohexyl radical,
  · a phenyl radical,

- two groups $R_1$ and $R_2$ and/or $R_3$ and $R_4$ placed on two neighbouring carbon atoms can form together and with the carbon atoms which bear them a benzene ring.

**5.** A process according to one of Claims 2 to 4, **characterised in that** the fuchsone is a fuchsone corresponding to the general formula (III) and/or (IV) in which one of the radicals $R_1$, $R_2$, $R_3$ and $R_4$ represents a hydroxyl group, a methyl radical or a methoxy radical, and the 3 others represent a hydrogen atom.

**6.** A process according to one of Claims 1 to 5, **characterised in that** the fuchsone is a fuchsone corresponding to general formula (III) and/or (IV) wherein $R_a$ and $R_b$ represent a branched alkyl radical having at least 3 carbon atoms or an aryl radical having at least 6 carbon atoms, preferably a tert-butyl, tert-pentyl, tert-hexyl, or phenyl radical, possibly substituted.

**7.** A process according to one of Claims 1 to 6, **characterised in that** the fuchsone is a fuchsone corresponding to the following general formula (IIIa) and/or (IVa) :

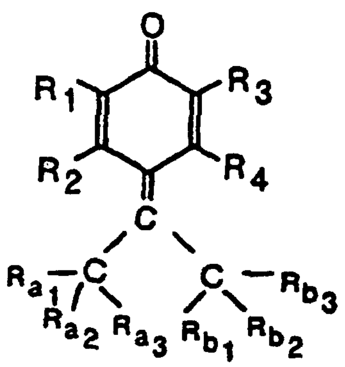

(IIIa)

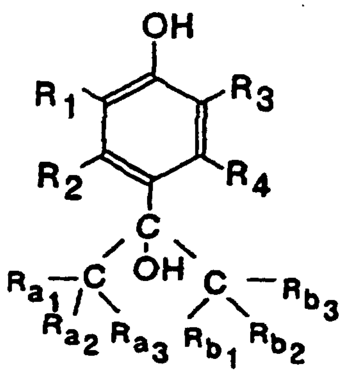

(IVa)

in which formulae (IIIa) and (IVa) :

- $R_{a1}$, $R_{a2}$, $R_{a3}$ and $R_{b1}$, $R_{b2}$, $R_{b3}$ which are identical or different represent branched or straight chain alkyl radicals having from 1 to 10 carbon atoms, optionally substituted cyclohexyl, phenyl or naphthyl radicals,
- $R_{a1}$, $R_{a2}$, $R_{a3}$, on the one hand, and $R_{b1}$, $R_{b2}$, $R_{b3}$, on the other hand, can form together and with the carbon atom which bears them an optionally substituted naphthalene or benzene ring,
- $R_1$, $R_2$, $R_3$ and $R_4$ which are identical or different represent one of the following groups:

  . a hydrogen atom,
  . a branched or straight chain alkyl radical having from 1 to 4 carbon atoms,
  . a branched or straight chain alkoxy radical having from 1 to 4 carbon atoms,
  . a hydroxyl group,
  . a halogen group,
  . a $-CF_3$ group,
  . a cyclohexyl radical,
  . a phenyl radical,

- the two groups $R_1$ and $R_2$ and/or $R_3$ and $R_4$ placed on two neighbouring carbon atoms are able to form together and with the carbon atoms which bear them a benzene ring.

**8.** A process according to one of Claims 1 to 7, **characterised in that** the fuchsone is a fuchsone corresponding to general formula (IIIa) and/or (IVa), in which one of the radicals $R_1$, $R_2$, $R_3$ and $R_4$ represents a hydroxyl group, a methyl radical, a methoxy radical, and the other 3 represent a hydrogen atom.

**9.** A process according to one of Claims 1 to 8, **characterised in that** the fuchsone is a fuchsone corresponding to following formula (IIIb) and/or (IVb):

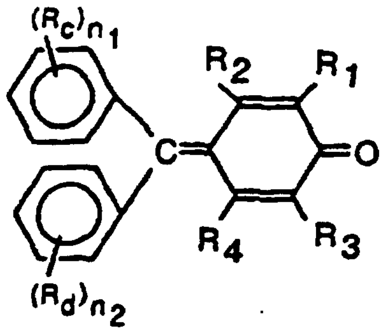

(IIIb)

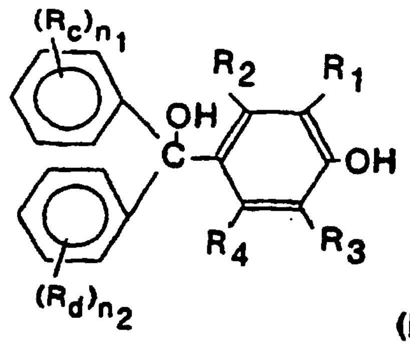

(IVb)

in which formulae (IIIb) and (IVb):

- $R_1$, $R_2$, $R_3$ and $R_4$ which are identical or different represent one of the following groups:
    - a hydrogen atom,
    - a branched or straight chain alkyl radical having from 1 to 4 carbon atoms,
    - a branched or straight chain alkoxy radical having from 1 to 4 carbon atoms,
    - a hydroxyl group,
    - a halogen atom,
    - a group $-CF_3$,
    - a cyclohexyl radical,
    - a phenyl radical,
- two groups $R_1$ and $R_2$ and/or $R_3$ and $R_4$ placed on two neighbouring carbon atoms can form together and with the carbon atoms which bear them a benzene ring.
- $R_c$ and $R_d$ which are identical or different represent a hydrogen atom or a substituent,
- $n_1$, $n_2$ which are identical or different are a number equal to 0, 1, 2 or 3.

**10.** A process according to one of Claims 1 to 9, **characterised in that** the fuchsone is a fuchsone corresponding to general formula (IIIb) and/or (IVb) in which $R_c$ and $R_d$ which are identical or different represent:

- branched or straight chain alkyl radicals having from 1 to 4 carbon atoms,
- a phenyl radical,
- alkoxy radicals $R_{10}$ - O in which $R_{10}$ represents a branched or straight chain alkyl radical having from 1 to 4 carbon atoms or the phenyl radical,
- a halogen atom, preferably chlorine, bromine or fluorine.

**11.** A process according to one of Claims 1 to 10, **characterised in that** the fuchsone is a fuchsone corresponding to general formula (IIIb) and/or (IVb), in which $R_c$ and $R_d$ which are identical or different represent a hydrogen atom or a substituent such as defined in Claim 38, preferably in position 4,4', and $n_1$, $n_2$ which are identical or different are equal to 0 or 1.

**12.** A process according to one of Claims 1 to 11, **characterised in that** the fuchsone is a fuchsone corresponding to general formula (IIIb) and/or (IVb), in which $R_c$ and $R_d$ which are identical or different represent a hydrogen atom; a methyl, ethyl, tert-butyl or phenyl radical; a methoxy or ethoxy radical; a hydroxyl group, preferably in position 3,3' or 4,4'.

**13.** A process according to one of Claims 1 to 12, **characterised in that** the oxidising agent is selected from hydrogen peroxide; peracids, preferably peracetic acid; hydroperoxides, preferably tert-butyl hydroperoxide, cyclohexyl hydroperoxide, cumyl hydroperoxide.

**14.** A process according to Claim 13, **characterised in that** the oxidising agent is an aqueous solution of hydrogen peroxide.

**15.** A process according to one of Claims 1 to 14, **characterised in that** the amount of hydrogen peroxide is preferably equal to the stoichiometric quantity, i.e. 1 mole of $H_2O_2$ for 1 mole of fuchsone of formula (III) and/or (IV).

**16.** A process according to one of Claims 1 to 15, **characterised in that** the fuchsone and the oxidising agent are reacted, in the presence of an acid catalyst which is a strong protonic acid with a pKa in water of less than -0,1, and, preferably, of less than -1.0.

**17.** A process according to one of Claims 1 to 16, **characterised in that** the strong acid is a halogenated or non-halogenated oxyacid, preferably sulphuric acid, pyrosulphuric acid, perchloric acid, halogenosulphonic acids, preferably fluorosulphonic acid, chlorosulphonic acid or trifluoromethanesulphonic acid, methanesulphonic acid, ethanesulphonic acid, ethanedisulphonic acid, benzenesulphonic acid, benzenedisulphonic acids, toluenesulphonic acids, naphthalenesulphonic acids, and naphthalendisulphonic acids; and, still more preferably, perchloric acid, trifluoromethanesulphonic acid or methanesulphonic acid.

**18.** A process according to one of Claims 1 to 17, **characterised in that** the amount of acid, expressed by the ratio of the number of proton equivalents to the number of moles of hydrogen peroxide, varies between about $1.10^{-4}$ and about 1.0, preferably between $1.10^{-3}$ and 0.1.

**19.** A process according to one of Claims 1 to 18, **characterised in that** the reaction is carried out in a reaction solvent which is a phenolic compound of formula (I) and/or an organic solvent.

**20.** A process according to Claim 19, **characterised in that** the organic solvent is selected from :

- alphatic or aromatic nitriles, preferably acetonitrile, propionitrile, butanenitrile, isobutanenitrile, benzonitrile, benzyl cyanide,
- aliphatic, cycloaliphatic or aromatic ether-oxides, and, preferably, diethyl oxide, dipropyl oxide, diisopropyl oxide, dibutyl oxide, methyltertiobutylether, dipentyl oxide, diisopentyl oxide, ethyleneglycol dimethylether (or 1,2-dimethoxyethane), dimethylether of diethyleneglycol (or 1,5-dimethoxy 3-oxapentane), dioxan, tetrahydrofuran,
- chlorinated aliphatic hydrocarbons, preferably dichloromethane, tetrachloromethane, dichloroethane,
- alcohols, preferably methanol, ethanol, isopropanol, tertiobutanol.

**21.** A process according to one of Claims 1 to 20, **characterised in that** the reaction temperature is between 20°C and 150°C, preferably between 30°C and 80°C.

**22.** A process according to one of Claims 1 to 21, **characterised in that** using a solution of hydrogen peroxide, diphenylfuchsone or rosolic acid, the diphenylmethylfuchsone is oxidised with a view to obtaining hydroquinone or methylhydroquinine, respectively.

**23.** A process according to one of Claims 1 to 22, **characterised in that** the fuchsone used is a fuchsone which has been obtained in accordance with the process consisting in reacting a phenolic compound with at least one hydrogen atom in para position and a non-enolisable ketonic compound in the presence of an effective quantity of a protonic acid having an acidity-Ho function of at least 5, and possibly in the presence of an effective quantity of an ionisable sulphurous compound.

**24.** A process according to Claim 23, **characterised in that** the phenolic compound which has at least one hydrogen atom in para position is a phenolic compound corresponding to general formula (I):

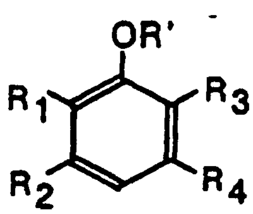

(I)

in which formula (I):

- the para position is free,
- $R_1$, $R_2$, $R_3$ and $R_4$ which are identical or different represent a hydrogen atom or any substituent,
- two groups $R_1$ and $R_2$ and/or $R_3$ and $R_4$ placed on two neighbouring carbon atoms can form together and with the carbon atoms which bear them a ring,
- R' represents a hydrogen atom or a hydrocarbon radical having 1 to 24 carbon atoms, which may be a branched or straight chain, saturated or unsaturated acyclic aliphatic radical; a monocyclic or polycyclic, saturated or unsaturated cycloaliphatic radical; a branched or straight chain, saturated or unsaturated aliphatic radical bearing a cyclic substituent.

**25.** A process according to one of Claims 23 and 24, **characterised in that** the phenolic compound corresponds to general formula (I), in which :

- the radical R' represents one of the following groups:

  · a hydrogen atom
  · a branched or straight chain alkyl radical having from 1 to 6 carbon atoms, preferably from 1 to 4 carbon atoms,
  · a cyclohexyl radical
  · a phenyl radical,
  · a benzyl radical.

- $R_1$, $R_2$, $R_3$ and $R_4$ which are identical or different represent $R_0$, one of the following groups:

  · a hydrogen atom,
  · a branched or straight chain alkyl radical having from 1 to 6 carbon atoms, preferably from 1 to 4 carbon atoms, such as methyl, ethyl, propyl, isopropyl, butyl, sec-butyl, tert-butyl,
  · a branched or straight chain alkenyl radical having from 2 to 6 carbon atoms, preferably from 2 to 4 carbon atoms, such as vinyl, allyl,
  · a branched or straight chain alkoxy radical having from 1 to 6 carbon atoms, preferably from 1 to 4 carbon atoms, such as the methoxy, ethoxy, propoxy, isopropoxy, butoxy radicals,
  · an acyl group having from 2 to 6 carbon atoms,
  · a radical of the formula:

$$-R_5-OH$$

$$-R_5-COOR_6$$

$$-R_5-X$$

$$-R_5-CF_3$$

  in which formulae, $R_5$ represents a valence bond or a saturated or unsaturated, branched or straight chain, divalent hydrocarbon radical having from 1 to 6 carbon atoms, such as methylene, ethylene, propylene, isopropylene, isopropilydene, for example; $R_6$ represents a branched or straight chain alkyl radical having 1 to 6 carbon atoms; X symbolises a halogen atom, preferably a chlorine, bromine or fluorine atom.

- $R_1$, $R_2$, $R_3$ and $R_4$ which are identical or different represent $R_7$, one of the following more complex radicals:

  · a saturated or unsaturated carbocyclic radical having from 4 to 7 carbon atoms, preferably a cyclohexyl radical,
  · a radical of the formula

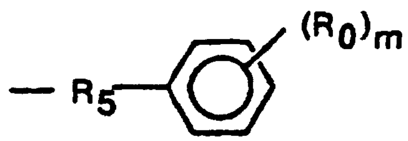

in which $R_5$ represents a valence bond or a saturated or unsaturated, branched or straight chain, divalent hydrocarbon radical having from 1 to 6 carbon atoms, such as methylene, ethylene, propylene, isopropylene, isopropylidene, and $R_0$ having the meaning given hereinabove, and m is an integer of from 0 to 4,

· a radical - $R_5$ - A - $R_8$, in which $R_5$ has the meaning given hereinabove, $R_8$ represents a branched or straight chain alkyl radical having from 1 to 6 carbon atoms or a radical of the formula

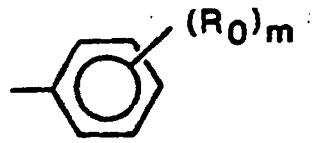

and A symbolises one of the following groups:
-O-, -COO-, -OCOO-, -$SO_2$-,

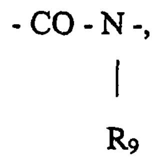

in which formulae, $R_9$ represents a hydrogen atom or an alkyl radical having from 1 to 4 carbon atoms, a cyclohexyl or phenyl radical,

- two groups $R_1$ and $R_2$ and/or $R_3$ and $R_4$ placed on two neighbouring carbon atoms can form together and with the carbon atoms which bear them an unsaturated or aromatic carbocycle having from 4 to 7 carbon atoms, and preferably 6 carbon atoms.

**26.** A process according to one of Claims 23 to 25, **characterised in that** the phenolic compound corresponds to general formula (I), in which:.

- R' represents a hydrogen atom
- $R_1$, $R_2$, $R_3$ and $R_4$ which are identical or different represent one of the following groups:

  · a hydrogen atom,
  · a branched or straight chain alkyl radical having from 1 to 4 carbon atoms,
  · a branched or straight chain alkoxy radical having from 1 to 4 carbon atoms,
  · a hydroxyl group,
  · a halogen atom,
  · a group -$CF_3$,
  · a cyclohexyl radical,
  · a phenyl radical,

- two groups $R_1$ and $R_2$ and/or $R_3$ and $R_4$ placed on two neighbouring carbon atoms can form together and with the carbon atoms which carry them a benzene ring.

**27.** A process according to one of Claims 23 to 26, **characterised in that** the phenolic compound corresponds to

general formula (I) in which R' represents a hydrogen atom, and one of the radicals $R_1$, $R_2$, $R_3$ and $R_4$ represents a hydroxyl group, a methyl radical or a methoxy radical, and the 3 others represent a hydrogen atom.

**28.** A process according to one of Claims 23 to 27, **characterised in that** the phenolic compound is phenol, anisol, orthocresol, metacresol, 2-methoxyphenol, 2-ethylphenol, 3-ethylphenol, 2-propylphenol, 2-sec-butylphenol, 2-tert-butylphenol, 3-tert-butylphenol, 2-methoxyphenol, 3-methoxyphenol, methyl salicylate, 2-chlorophenol, 3-chlorophenol, 2,3-dimethylphenol, 2,5-dimethylphenol, 2,6-dimethylphenol, 3,5-dimethylphenol, 2,3-dichlorophenol, 2,5-dichlorophenol, 2,6-dichlorophenol, 3,5-dichlorophenol, 2,6-ditertbutylphenol, 3,5-ditert-butylphenol, 2,3,5-trimethylphenol, 2,3,6-trimethylphenol, 2,3,5-trichlorophenol, 2,3,6-trichlorophenol; 1-hydroxynaphthalene; 2-phenoxyphenol, 3-phenoxyphenol.

**29.** A process according to one of Claims 23 to 28, **characterised in that** the non-enolisable ketonic compound is a ketonic compound corresponding to general formula (II):

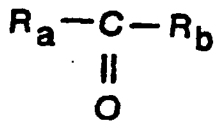

(II)

in which formula (II):

- $R_a$ and $R_b$ which are identical or different are hydrocarbon radicals each having from 3 to 30 carbon atoms; the carbon atoms of each radical $R_a$ and $R_b$ in position $\alpha$ in relation to the carbonyl group being tertiary carbons.

**30.** A process according to one of Claims 23 to 29, **characterised in that** the ketonic compound corresponds to general formula (IIa):

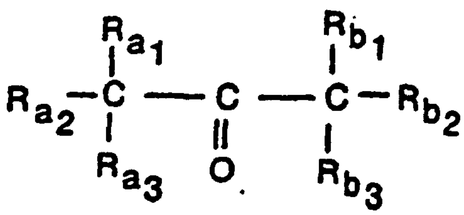

(IIa)

in which formula (IIa):

- $R_{a1}$, $R_{a2}$, $R_{a3}$ and $R_{b1}$, $R_{b2}$, $R_{b3}$ which are identical or different represent branched or straight chain alkyl radicals having from 1 to 10 carbon atoms, optionally substituted cyclohexyl, phenyl or naphthyl radicals,
- $R_{a1}$, $R_{a2}$, $R_{a3}$ and/or $R_{b1}$, $R_{b2}$, $R_{b3}$ can form together and with the carbon atom which bears them an optionally substituted naphthalene or benzene ring.

**31.** A process according to one of Claims 23 to 30, **characterised in that** the ketonic compound corresponds to general formula (IIb):

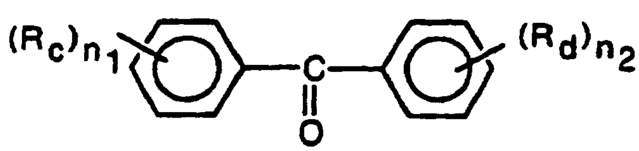

(IIb)

in which formula (IIb):

- $R_c$ and $R_d$ which are identical or different represent a hydrogen atom or a substituent,
- $n_1$, $n_2$ which are identical or different is a number equal to 0, 1, 2 or 3.

**32.** A process according to one of Claims 23 to 31, **characterised in that** the ketonic compound corresponds to general formula (IIb) in which $R_c$ and $R_d$ which are identical or different represent:

- branched or straight chain alkyl radicals having from 1 to 4 carbon atoms,
- a phenyl radical,
- alkoxy radicals $R_{10}$ - O in which $R_{10}$ represents a branched or straight chain alkyl radical having from 1 to 4 carbon atoms or the phenyl radical,
- a hydroxyl group,
- a fluorine atom.

**33.** A process according to one of Claims 23 to 32, **characterised in that** the ketonic compound corresponds to general formula (IIb), in which $R_c$ and $R_d$ which are identical or different represent a hydrogen atom or a substituent, such as defined in Claim 32, preferably in position 4,4' and $n_1$,$n_2$ which are identical or different are equal to 0 or 1.

**34.** A process according to one of Claims 23 to 33, **characterised in that** the ketonic compound corresponds to general formula (IIb) in which $R_c$ and $R_d$ which are identical or different represent a hydrogen atom; a methyl, ethyl, tert-butyl, phenyl radical; a methoxy or ethoxy radical; a hydroxyl group, preferably in position 3,3' or 4,4'.

**35.** A process according to one of Claims 23 to 34, **characterised in that** the ketonic compound of general formula (IIb) is:

benzophenone
2-methyl benzophenone
2,4-dimethyl benzophenone
4,4'-dimethyl benzophenone
2,2'-dimethyl benzophenone
4,4'-dimethoxy benzophenone
4-hydroxy benzophenone
4,4'-dihydroxy benzophenone
4-benzoyl biphenyl

**36.** A process according to one of Claims 23 to 35, **characterised in that** the acid catalyst has an acidity-Ho function of between 5 and 20, preferably of between 10 and 15.

**37.** A process according to Claim 36, **characterised in that** the acid catalyst is a strong protonic acid, preferably hydrofluoric acid, perchloric acid, trifluoromethanesulphonic acid, paratoluenesulphonic acid, chlorosulphonic acid, fluorosulphonic acid, methanesulphonic acid, benzenesulphonic acid, and, still more preferably, hydrofluoric acid, perchloric acid, trifluoromethanesulphonic acid, or methanesulphonic acid.

**38.** A process according to Claim 36, **characterised in that** the acid catalyst is a sulphonic resin, preferably a sulphonated styrene-divinylbenzene copolymer, a sulphonated phenol-formaldehyde copolymer, a tetrafluoroethylene-perfluoro[2-(fluorosulphonylethoxy)-propyl] vinyl ether copolymer, and, preferably, the following resins: TEMEX 50, AMBERLYST 15, AMBERLYST 35, AMBERLYST 36, DOWEX 50 W; DUOLITE ARC 9359: NAFION.

**39.** A process according to Claim 36, **characterised in that** the acid catalyst is selected from: silica-alumina, silica-$Ga_2O_3$, silica-$B_2O_3$; acid clays; bridged clays; natural or synthetic zeolites; boron, aluminium or gallium phosphates; lamellar phosphates of tetravalent metals corresponding to the formula $\alpha$-$M(HPO_4)_2$, $pH_2O$ in which M represents a tetravalent metal selected from titanium, germanium, zirconium or tin and p is a number less than 2: lamellar phosphonates of tetravalent metals, preferably lamellar phosphonates of zirconium; oxides which are made acid by a treatment, preferably the sulphated oxides of titanium, zirconium or iron.

**40.** A process according to Claim 36, **characterised in that** the acid catalyst used is selected from:

- natural clays having a structure referred to as "TOT" or tetrahedral-octahedral-tetrahedral, preferably from the class of smectites, and, still more preferably, montmorillonites,
- commercial clays, already acid or not, or treated with an aqueous solution of an acid.
- bridged clays prepared from smectites, preferably from beidellites, in particular from synthetic beidellites or montmorillonites; bridging of the clay preferably being an aluminium bridging.

**41.** A process according to Claim 36, **characterised in that** the acid catalyst is selected from:

- natural zeolites such as chabazite, clinoptilolite, erionite, mordenite, phillipsite offretite,
- synthetic zeolites, such as zeolite ZSM-5, zeolite Y, ferrierite, zeolite X of faujasite type, zeolite of type L, mordenite, zeolite ZSM-11, mazzite, offretite, preferably a zeolite US-Y or a zeolite ZSM-5.

**42.** A process according to Claim 41, **characterised in that** the zeolite used is an acidified zeolite.

**43.** A process according to one of Claims 23 to 42, **characterised in that** the ionisable sulphurous compound is a sulphurous inorganic compound or a sulphurous organic compound, preferably selected from:

- sulphur halides,
- thiosulphides of ammonium, alkali metal or alkaline earth metal,
- hydrogen sulphide or its precursors,
- mercaptans, preferably alkylmercaptans, phenylmercaptans and phenylalkylmercaptans,
- thioalcohols
- thiophenols
- thioorganic acids, their salts or their esters,
- cation exchange resins modified by reaction with a mercaptoamine alkyl, preferably resins with a polystyrene skeleton bearing sulphonic groups having reacted with a mercaptoamine alkyl, preferably that with a primary amine group, and, still more preferably, that having from 1 to 4 carbon atoms.

**44.** A process according to Claim 43, **characterised in that** the sulphurous compound is selected from:

- sulphur monochloride or sulphur bichloride,
- ammonium, sodium or potassium thiosulphate,
- hydrogen sulphide or calcium or potassium sulphide,
- ethylmercaptan, butylmercaptan, 1-nonylmercaptan, benzylmercaptan,
- 2-mercaptoethanol, 3-mercapto-2-butanol,
- thiophenol, o-thiocresol, m-thiocresol, p-thiocresol, thioxylenol, p-methylthiophenol, o-ethylthiophenol, thiohydroquinone, thionaphthol,
- thioacetic acid, thiopropionic acid, thiolactic acid, thiosalicylic acid, 2-mercaptoethanesulphonic acid, 3-mercaptopropanesulphonic acid, mercaptosuccinic acid, thioglycolic acid, methyl and ethyl thioglycolate,
- resins with a polystyrene skeleton bearing sulphonic groups which have reacted with 2-mercaptoethylamine, 2-mercaptoisopropylamine, 3-mercaptobutylamine.

**45.** A process according to one of Claims 23 to 44, **characterised in that** the molar ratio between the phenolic compound of formula (I) and the ketonic compound of formula (II) is between 1 and 20, preferably between 1 and 10.

**46.** A process according to one of Claims 23, 36 to 38, **characterised in that** the quantity of acid catalyst expressed by the ratio of equivalents of protons to the number of moles of ketonic compound of formula (II) varies between $1.10^{-3}$ and 1.0, preferably between $5.10^{-3}$ and 0.5.

**47.** A process according to Claim 36 or Claim 37, **characterised in that** said ratio is between 1.0 and 20, preferably between 5.0 and 10 when the acid is used as the reaction medium.

**48.** A process according to one of Claims 39 to 42, **characterised in that** the quantity of acid catalyst used represents by weight in relation to the phenolic compound of formula (I) used, from 0.1 to 20%, preferably from 0.5 to 10%.

**49.** A process according to one of Claims 23 to 48, **characterised in that** the quantity of sulphurous compound used, expressed in relation to the ketonic compound of formula (II), is such that the molar ratio between the sulphurous compound and the ketonic compound of formula (II) is between 0.001 and 1.0, preferably between 0.005 and 0.20.

**50.** A process according to one of Claims 23 to 49, **characterised in that** the reaction temperature is between 45°C and 200°C, preferably between 60°C and 150°C.

**51.** A process according to one of Claims 23 to 50, **characterised in that** the diphenylfuchsone is prepared by reacting phenol and benzophenone; the rosolic acid by reacting phenol and 4,4'-dihydroxybenzophenone; the diphenylmethylfuchsone by reacting ortho-cresol and benzophenone.

**52.** A process according to Claim 23, **characterised in that** the p-dihydroxylated aromatic compound is obtained by preparing a fuchsone in accordance with the process described in one of Claims 23 to 51, and then by oxidising it by adding oxidising agent to the reaction medium, without the fuchsone being isolated.

**53.** A process for the preparation of a p-dihydroxylated aromatic compound, by oxidising a phenolic compound, which has a hydrogen atom in para position, with hydrogen peroxide, in the presence of an effective quantity of a strong protonic acid having a pKa in water of less than -0.1, and preferably of less than -1.0, **characterised in that** the reaction is carried out in the presence of an effective quantity of a p-fuchsone.

**54.** A process according to Claim 53, **characterised in that** the phenolic compound is a phenolic compound of formula (I), as described in one of Claims 24 to 28.

**55.** A process according to one of Claims 53 and 54, **characterised in that** the p-fuchsone is a fuchsone corresponding to formula (III) and/or (IV), as described in one of Claims 2 to 12.

**56.** A process according to one of Claims 53 to 55, **characterised in that** the p-fuchsone is obtained in accordance with the process described in one of Claims 23 to 51.

**57.** A process according to one of Claims 53 to 56, **characterised in that** the quantity of fuchsone of formula (III) and/or (IV) is less than 1.0 mole per mole of hydrogen peroxide, preferably between $1.10^{-3}$ and 1.0, and, still more preferably, between 0.05 and 0.5.

**58.** A process according to one of Claims 53 to 57, **characterised in that** the strong acid is an acid, as defined in one of Claims 16 and 17.

**59.** A process according to one of Claims 53 to 58, **characterised in that** the quantity of acid is such that the $H^+ /H_2O_2$ ratio is between about $1.10^{-4}$ and about 1.0, preferably between $1.10^{-3}$ and 0.1.

**60.** A process according to one of Claims 53 to 59, **characterised in that** the $H_2O_2$/phenolic compound of formula (I) ratio is between 0.01 and 0.3, preferably between 0.05 and 0.10.

**61.** A process according to one of Claims 53 to 60, **characterised in that** the reaction is carried out in the presence of a polar, aprotic, organic solvent having a polarity such that its dielectric constant is above or equal to 20, preferably between 25 and 75, and such that its basicity is such that it has a "donor number" of less than 25, preferably less than or equal to 20, and still more preferably between 2 and 17.

**62.** A process according to Claim 61, **characterised in that** the polar organic solvent is selected from : nitrated compounds, preferably nitromethane, nitroethane, 1-nitropropane, 2-nitropropane, or mixtures thereof, nitrobenzene; aliphatic or aromatic nitriles, preferably acetonitrile, propionitrile, butanenitrile, isobutanenitrile, benzonitrile, benzyl cyanide; tetramethylene sulphone and propylene carbonate.

**63.** A process according to one of Claims 61 and 62, **characterised in that** the quantity of solvent used is such that the molar ratio between the number of moles of organic solvent and the number of moles of phenolic compound of formula (I) varies between 0.1 and 2.0, preferably between 0.25 and 1.0.

**64.** A process according to one of Claims 53 to 63, **characterised in that** the reaction is carried out in the presence of a polar, aprotic organic solvent having a polarity such that its dielectric constant is less than about 20, preferably between 2 and 15, and such that its basicity is such that it has a "donor number" greater than or equal to 15 and less than 25, preferably between 15 and 25.

**65.** A process according to Claim 64, **characterised in that** the polar organic solvent is selected from : aromatic,

cycloaliphatic or aliphatic ether-oxides, preferably diethyl oxide, dipropyl oxide, diisopropyl oxide, dibutyl oxide, methyltertiobutylether, dipentyl oxide, diisopentyl oxide, ethyleneglycol dimethylether (or 1,2-dimethoxy ethane), dimethylether of diethyleneglycol (or 1,5-dimethoxy 3-oxapentane), dioxan, tetrahydrofuran; phosphoric neutral esters, preferably trimethyl phosphate, triethyl phosphate, butyl phosphate, triisobutyl phosphate, tripentyl phosphate; ethylene carbonate.

**66.** A process according to one of Claims 64 and 65, **characterised in that** the quantity of solvent used is such that the molar ratio between the number of moles of organic solvent and the number of moles of phenolic compound of formula (I) varies between 0.01 and 0.25, preferably between 0.025 and 0.15.

**67.** A process according to one of Claims 56 to 66, **characterised in that** the operation is carried out at a temperature of between 45°C and 150°C, preferably of between 60°C and 110°C.

**68.** A process according to one of Claims 56 to 67, **characterised in that** the phenol and a solution of hydrogen peroxide are reacted in the presence of an effective quantity of a strong acid and an effective quantity of diphenylfuchsone and/or its carbinol.

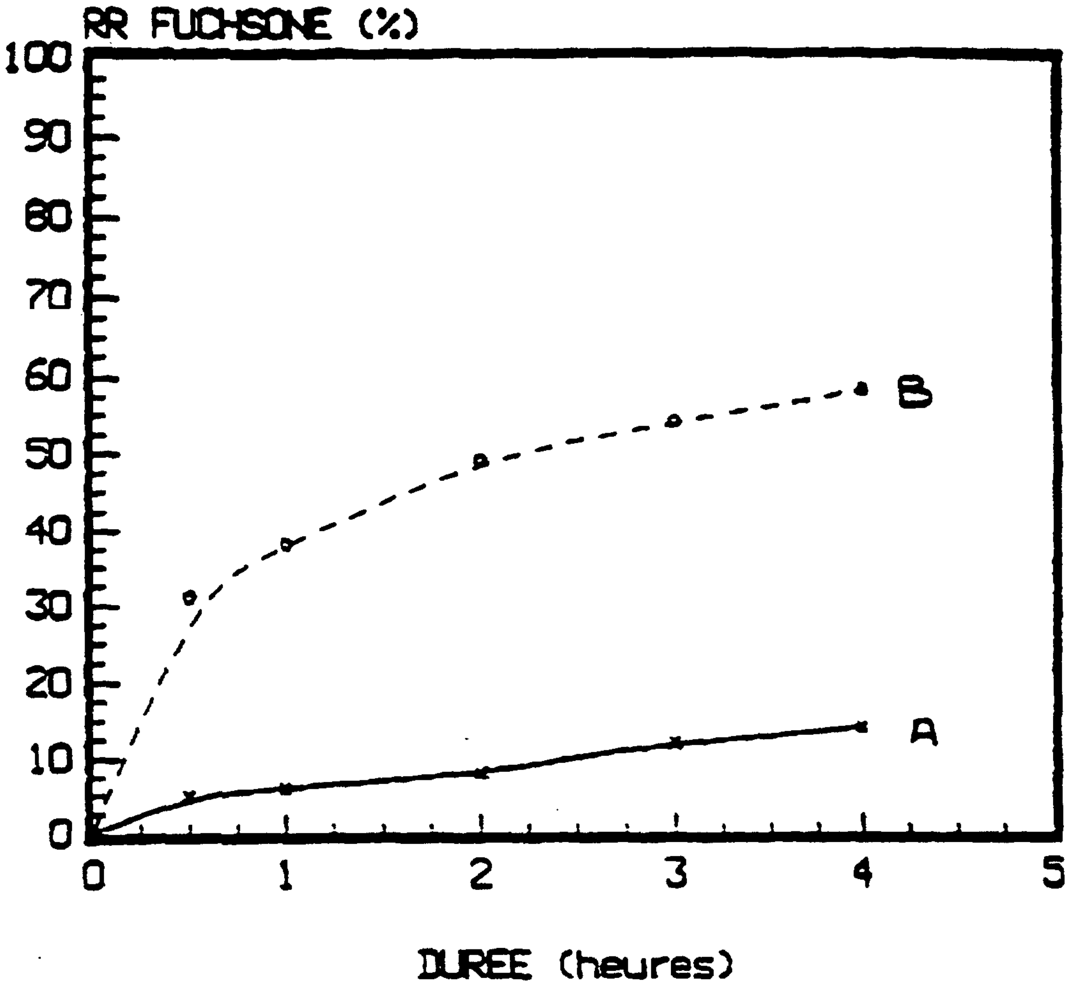
RR FUCHSONE (%)
100
90
80
70
60
50
40
30
20
10
0
B
A
0
1
2
3
4
5
DUREE (heures)

Figure 1